# EUROPEAN PATENT SPECIFICATION

(11) **EP 4 321 536 B1**
(45) Date of publication and mention of the grant of the patent: **01.04.2026**
(21) Application number: 22902772.7
(22) Date of filing: 22.06.2022
(51) Int. Cl.: C07K 16/46, C12N 15/13, G01N 33/68, G01N 33/574

(54) **BISPECIFIC ANTIBODY AND USE THEREOF**
BISPEZIFISCHER ANTIKÖRPER UND VERWENDUNG DAVON
ANTICORPS BISPÉCIFIQUE ET SON UTILISATION

(30) Priority: 07.12.2021 CN 202111485404
(43) Date of publication of application: 14.02.2024
(73) Proprietor: Hefei TG Immunopharma Co., Ltd., Hefei, Anhui 230001 (CN)
(72) Inventor: TIAN, Zhigang, Hefei, Anhui 230001 (CN); CHENG, Ying, Hefei, Anhui 230001 (CN); XIAO, Weihua, Hefei, Anhui 230001 (CN); CAO, Guoshuai, Hefei, Anhui 230001 (CN); SUN, Haoyu, Hefei, Anhui 230001 (CN); SUN, Rui, Hefei, Anhui 230001 (CN)
(74) Representative: Dehns Germany Partnerschaft mbB
(86) International application number: PCT/CN2022/100475
(87) International publication number: WO 2023/103335

(56) References cited:
- WO-A1-2013/169691
- WO-A2-2021/064137
- WO-A2-2021/064137
- CN-A- 110 511 278
- CN-A- 113 286 821
- CN-A- 114 395 044
- CN-A- 114 395 047
- US-B2- 10 087 250
- SUN XIN; ZHAO JINGYUAN; MA LI; SUN XIMING; GE JING; YU YANG; MA JUAN; ZHANG MAN: "B7-H6 as an efficient target for T cell-induced cytotoxicity in haematologic malignant cells", INVESTIGATIONAL NEW DRUGS, SPRINGER US, NEW YORK, vol. 39, no. 1, 7 August 2021 (2021-08-07), New York, pages 24 - 33, XP037353706, ISSN: 0167-6997, DOI: 10.1007/s10637-020-00976-5

## Description

### PRIORITY INFORMATION

This application claims priority to and the benefit of Patent Application No. 202111485404.0, filed on December 07, 2021, in the China National Intellectual Property Office.

### FIELD

The present disclosure belongs to the field of bio-medicine, and particularly, to a bispecific antibody and use thereof, more particularly to a recombinant antibody, an immune cell, a nucleic acid, an expression vector, a recombinant cell, a composition, uses of the above for the manufacture of a medicament, and a kit.

### BACKGROUND

Bispecific antibodies are antibodies that specifically bind to two antigenic sites simultaneously. Bispecific antibodies for tumor therapy can be divided into three classes based on their mechanisms, i.e., bispecific antibodies for redirecting effector cells; bispecific antibodies for immunoregulation; bispecific antibodies for targeting tumor cell receptor and dual binding. Among them, antibodies with the redirecting function predominate, and two currently marketed antibodies for tumor therapy are also based on T cell redirecting. Depending upon the characteristics of the bispecific antibodies, they can also be used well in other therapeutic systems, such as dual immunoregulation or targeting two molecules of the same cell membrane.

B7H6 is a type I transmembrane protein belonging to the B7 family, and it contains two immunoglobulin domains. B7H6 is also a ligand for the NK cell activating receptor NKp30. B7H6 is expressed on a variety of tumor cells, but mRNA of B7H6 is not detected in normal tissues and healthy peripheral blood mononuclear cells of humans. In an inflammatory environment, certain proinflammatory cytokines such as IL-1β and TNF can stimulate the up-regulation of B7H6 expression by CD14⁺ and CD16⁺ mononuclear cells and neutrophils. The tumor expression profile of B7H6 is very wide, including leukemia, lymphoma, colorectal cancer, non-small cell lung cancer, breast cancer, ovarian cancer, gastric cancer, and liver cancer. B7H6 expression is associated with metastasis of some cancers. However, the role of B7H6 in most tumors is currently unclear. Currently, although B7H6 is expressed in a variety of tumors, there are few tumor therapies targeting this molecule. Researchers have developed a bispecific antibody against B7H6, constructed in the form of BiTE, which does not contain the constant region of the native antibody, resulting an excessively short half-life of this molecule *in vivo* and thus limiting its use.

CD3 is an important target in T cell-based redirecting bispecific antibodies. Unlike immuno-checkpoint blocking antibodies, CD3-related bispecific antibodies can mediate T cell activation bypassing TCR and peptide-major histocompatibility complex (pMHC), but the molecular functioning process in the synapse is very similar to the classical TCR-pMHC interaction. The activity of bispecific antibodies is influenced by the affinity of CD3, which has a better pro-killing effect in *in vitro* experiments, but a lethal cytokine release syndrome toxicity *in vivo,* which is very similar to the chimeric antigen receptor (CAR) T cell toxicity. It has also been found that CD3 antibody sequences with very low affinity can also effectively stimulate T cell activation after the construction of bispecific antibodies. When the CD3 antibody with an affinity in the appropriate range (200 ± 78 nM) and the tumor target-associated antibody is of high affinity, the bispecific antibody promotes that T cells are selectively located to the tumor site rather than to the peripheral circulation, avoiding systemic activation. Therefore, the development of highly targeted bispecific antibodies is of great value to the prevention and treatment of tumors. US 10 087 250 B2 discloses a bispecific antibodies comprising a first Fab fragment having at least one antigen binding site specific for a first antigen and a second Fab fragment having at least one antigen binding site specific for a second antigen. WO 2013/169691 A1 provides an isolated antibody or antigen binding fragment thereof, which specifically binds to B7-H6. WO 2021/064137 A2 relates to novel B7H6/CD3 binding proteins.

### SUMMARY

The present disclosure is based on the Applicant's discovery and recognition of the following facts and problems.

In the present disclosure, the Applicant carried out various combinations and screening for CD3 and B7H6 antigen-binding fragments and designed recombinant bispecific antibodies, which can specifically bind to CD3 and B7H6, thereby targeting a B7H6 molecule to the vicinity of tumor cells. The recombinant bispecific antibodies promote CD3⁺ lymphocytes to form immunological synapses with tumor cells by binding to the B7H6 molecules on the tumor cells, thereby activating the lymphocytes to kill the B7H6⁺ tumor cells. The recombinant bispecific antibodies have high CD3- and B7H6-binding activities and high anti-tumor activity.

Thus, in a first aspect of the present disclosure, the present disclosure provides a bispecific recombinant antibody. The recombinant antibody includes CD3 antibody variable region CDR sequences as set forth in SEQ ID NO: 1 to SEQ ID NO: 6, and B7H6 antibody variable region CDR sequences as set forth in SEQ ID NO: 7 to SEQ ID NO: 12. The recombinant antibody according to the present disclosure can effectively bind to CD3 and B7H6, has strong *in vivo* and *in vitro* binding activities, a long *in vivo* half-life, and has a significant anti-tumor effect.
RASQDIRNYLN (SEQ ID NO: 1).
YTSRLES (SEQ ID NO: 2).
QQGNTLPWT (SEQ ID NO: 3).
GYTMN (SEQ ID NO: 4).
LINPYKGVSTYNQKFKD (SEQ ID NO: 5).
SGYYGDSDWYFDV (SEQ ID NO: 6).
KASQSVDYDGDSYMN (SEQ ID NO: 7).
AASTLHS (SEQ ID NO: 8).
QQSKEDPRT (SEQ ID NO: 9).
DYNMD (SEQ ID NO: 10).
DINPNNGGTLYNQKFRG (SEQ ID NO: 11).
SEVFYGNYADY (SEQ ID NO: 12).

In a second aspect of the present disclosure, the present disclosure provides a bispecific recombinant antibody. The recombinant antibody has an amino acid sequence as set forth in SEQ ID NO: 49 and SEQ ID NO: 63. The recombinant antibody according to the embodiments of the present disclosure can effectively bind to CD3 and B7H6, has strong *in vivo* and *in vitro* binding activities, a long *in vivo* half-life, and has a significant anti-tumor effect.

MDIQMTQSPSSLSASVGDRVTITCRASQDIRNYLNWYQQKPGKAPKLLIYY
TSRLESGVPSRFSGSGSGTDYTLTISSLQPEDFATYYCQQGNTLPWTFGQGTKVEIKGG
GGSGGGGSGGGGSEVQLVESGGGLVQPGGSLRLSCAASGYSFTGYTMNWVRQAPGK
GLEWVALINPYKGVSTYNQKFKDRFTISVDKSKNTAYLQMNSLRAEDTAVYYCARSG
YYGDSDWYFDVWGQGTLVTVSSEPKSCDKTHTCPPCPAPEAAGGPSVFLFPPKPKDT
LMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPCRDELTKNQV
SLWCLVKGFYPSDIAVEWESNGQPENNYKTTPPVLDSDGSFFLYSKLTVDKSRWQQG
NVFSCSVMHEALHNHYTQKSLSLSPGK (SEQ ID NO: 49).

DIVLTQTPLSLSVTPGQPASISCKASQSVDYDGDSYMNWYLQKPGQPPQLL
IYAASTLHSGIPDRFSGSGSGTDFTLKISRVEAEDVGVYYCQQSKEDPRTFGQGTKLEI
KGGGGSGGGGSGGGGSEVQLVQSGAEVKKPGASVKVSCKASGYTFTDYNMDWVR
QAPGQGLEWIGDINPNNGGTLYNQKFRGRVTLTVDTSISTAYMELSRLRSDDTAVYYC
ARSEVFYGNYADYWGQGTTVTVSSEPKSCDKTHTCPPCPAPEAAGGPSVFLFPPKPK
DTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVCTLPPSRDELTKNQ
VSLSCAVKGFYPSDIAVEWESNGQPENNYKTTPPVLDSDGSFFLVSKLTVDKSRWQQG
NVFSCSVMHEALHNHYTQKSLSLSPGK (SEQ ID NO: 63).

In a third aspect of the present disclosure, the present disclosure provides a nucleic acid. The nucleic acid encodes the recombinant antibody according to the first aspect. The recombinant antibody encoded by the nucleic acid according to the embodiments of the present disclosure can effectively bind to CD3 and B7H6, has strong *in vivo* and *in vitro* binding activities, a long *in vivo* half-life, and a significant anti-tumor effect.

In a fourth aspect of the present disclosure, the present disclosure provides an expression vector. The expression vector carries the nucleic acid molecule according to the third aspect. The expression vector may include an optional control sequence operably linked to the nucleic acid molecule. The control sequence is one or more control sequences that direct expression of the nucleic acid molecule in a host. The expression vector proposed in the embodiments of the present disclosure can efficiently and massively express the recombinant antibody in suitable host cells, and thus it can be effectively used for specific treatment or prevention of tumors, and particularly tumors expressing B7H6.

In a fifth aspect of the present disclosure, the present disclosure provides a recombinant cell. The recombinant cell carries the nucleic acid molecule according to the third aspect, the expression vector according to the fourth aspect, or the recombinant antibody according to the first aspect. The recombinant cell is obtained by transfecting or transforming the expression vector. According to an embodiment of the present disclosure, the recombinant cell can efficiently and massively express the recombinant antibody under suitable conditions, and the recombinant cell can be effectively used for specific treatment or prevention of a tumor, particularly a tumor expressing B7H6.

In a sixth aspect of the present disclosure, the present disclosure provides a composition. The composition includes the recombinant antibody according to the first or second aspect, the nucleic acid molecule according to the third aspect, the expression vector according to the fourth aspect, or the recombinant cell according to the fifth aspect. As described above, the recombinant antibody according to the embodiments of the present disclosure can effectively bind to CD3 or B7H6 protein molecules, thereby specifically recognizing tumor cells highly expressing B7H6 and promoting selective localization of T cells to the tumor site rather than in the peripheral circulation, avoiding systemic activation. The medicament including the recombinant antibody also has significant effects for treating or preventing the tumors expressing B7H6, with higher safety and fewer side effects.

In a seventh aspect of the present disclosure, the present disclosure provides use of the recombinant antibody according to the first or second aspect, the nucleic acid molecule according to the third aspect, the expression vector according to the fourth aspect, the recombinant cell according to the fifth aspect, or the composition according to the sixth aspect for the manufacture of a medicament. According to an embodiment of the present disclosure, the medicament is used for the treatment or prevention of tumors. As described above, the recombinant antibody according to the embodiments of the present disclosure can effectively bind to CD3 and B7H6 protein, thereby specifically recognizing tumor cells highly expressing the B7H6. Thus, the medicament prepared with the recombinant antibody and the corresponding series of substances also has significant effects for treating or preventing tumors expressing B7H6, with the medicament has higher safety and fewer side effects.

In an eighth aspect of the present disclosure, the present disclosure provides a medicament. According to an embodiment of the present disclosure, the medicament includes the recombinant antibody according to the first or second aspect, the nucleic acid molecule according to the third aspect, the expression vector according to the fourth aspect, or the recombinant cell according to the fifth aspect or the composition of the sixth aspect. The medicament is used for treating or preventing cancer. As described above, the recombinant antibody according to the embodiments of the present disclosure can effectively bind to CD3 and B7H6 protein, thereby specifically recognizing tumor cells highly expressing the B7H6. Thus, the medicament prepared using the recombinant antibody and the corresponding series of substances also has significant effects for treating or preventing tumors expressing B7H6, with higher safety and fewer side effects.

In a ninth aspect of the present disclosure, the present disclosure provides a kit. According to an embodiment of the present disclosure, the kit includes the recombinant antibody according to the first or second aspect. The antibody according to the embodiments of the present disclosure can effectively bind to CD3 and B7H6 protein molecules, and thus it can specifically recognize tumor cells specifically high expressing the B7H6. Therefore, the recombinant antibody can be used for the preparation of a kit for diagnosing or detecting at least one of rectal cancer, non-small cell lung cancer, breast cancer, and liver cancer. The kit can be used for scientific studies such as qualitative or quantitative detection of CD3 and/or B7H6 protein molecules in a biological sample.

Additional aspects and advantages of the present disclosure will be provided in part in the following description and will be in part obvious from the following description, or may be learned by practice of the present disclosure.

### BRIEF DESCRIPTION OF DRAWINGS

The foregoing and/or additional aspects and advantages of the present disclosure will become apparent and readily appreciated from the following description of the embodiments, taken in conjunction with the accompanying drawings of which:
FIG. 1 is a schematic structural diagram of a recombinant bispecific antibody according to an embodiment of the present disclosure;
FIG. 2A is a binding analysis result graph of a CD3xB7H6 recombinant bispecific antibody binding to human B7H6 protein according to an embodiment of the present disclosure;
FIG. 2B is a binding analysis result graph of a CD3xB7H6 recombinant bispecific antibody binding to human CD3 protein according to an embodiment of the present disclosure;
FIG. 2C is an ELISA detection result graph of a CD3xB7H6 recombinant bispecific antibody with B7H6 and human CD3 double antigen according to an embodiment of the present disclosure;
FIG. 3A is a graph showing the results of an *in vitro* cytotoxicity test analysis of a CD3xB7H6 recombinant bispecific antibody and T cells on HCT-15 cells according to an embodiment of the present disclosure;
FIG. 3B is a graph showing the results of an *in vitro* cytotoxicity test analysis of a CD3xB7H6 recombinant bispecific antibody and T cells on LoVo cells according to an embodiment of the present disclosure;
FIG. 3C is a graph showing the results of an *in vitro* cytotoxicity test of a CD3xB7H6 recombinant bispecific antibody and T cells on Hep G2 cells according to an embodiment of the present disclosure;
FIG. 3D is a graph showing the results of an *in vitro* cytotoxicity test of a CD3xB7H6 recombinant bispecific antibody and T cells on SK-BR-3 cells according to an embodiment of the present disclosure;
FIG. 4A is a chemiluminescence value analysis graph according to an embodiment of the present disclosure, gradient concentrations of a CD3xB7H6 recombinant bispecific antibody molecule being added into a mixture obtained by mixing T cells with Ho8910 cells overexpressing luciferase, and chemiluminescence values being detected 24 hours after the addition of a substrate for reaction;
FIG. 4B is a chemiluminescence value analysis graph according to an embodiment of the present disclosure, gradient concentrations of a CD3xB7H6 recombinant bispecific antibody molecule being added into a mixture obtained by mixing T cells with K562 cells overexpressing luciferase, and chemiluminescence values being detected 24 hours after the addition of a substrate for reaction;
FIG. 5A is a graph showing the effect of CD3xB7H6 recombinant bispecific antibody molecules on T cell proliferation in the presence of HCT-15 cells according to an embodiment of the present disclosure;
FIG. 5B is a statistical analysis graph of the effect of CD3xB7H6 recombinant bispecific antibody molecules on T cell proliferation in the presence of HCT-15 cells according to an embodiment of the present disclosure;
FIG. 6A is a graph showing the effect of CD3xB7H6 recombinant bispecific antibody molecules on CD4⁺ and CD8⁺ T cell activation in the presence of HCT-15 cells according to an embodiment of the present disclosure;
FIG. 6B is a graph showing the effect of CD3xB7H6 recombinant bispecific antibody molecules on CD4⁺ and CD8⁺ T cell degranulation level in the presence of HCT-15 cells according to an embodiment of the present disclosure;
FIG. 7A is a graph showing the effect of a CD3xB7H6 recombinant bispecific antibody molecules on IFN-y secreted by T cells in the presence of HCT-15 cells according to an embodiment of the present disclosure;
FIG. 7B is a graph showing the effect of CD3xB7H6 recombinant bispecific antibody molecules on IL-2 secreted by T cells in the presence of HCT-15 cells according to an embodiment of the present disclosure;
FIG. 7C is a graph showing the effect of CD3xB7H6 recombinant bispecific antibody molecules on IL-10 secreted by T cells in the presence of HCT-15 cells according to an embodiment of the present disclosure;
FIG. 7D is a graph showing the effect of CD3xB7H6 recombinant bispecific antibody molecules on IL-17A secreted by T cells in the presence of HCT-15 cells according to an embodiment of the present disclosure; and
FIG. 8 is a graph showing the *in vivo* anti-tumor activity of CD3xB7H6 recombinant bispecific antibody molecules on HCT-15 cells according to an embodiment of the present disclosure.

### DETAILED DESCRIPTION

Embodiments of the present disclosure are described in detail below, and examples of the embodiments are illustrated in the accompanying drawings. The embodiments described below with reference to the accompanying drawings are illustrative and intended to explain the present disclosure, rather than limiting the present disclosure.

Furthermore, the terms such as "first" and "second" are used for descriptive purposes only and are not to be construed as indicating or implying relative importance or implicitly indicating the number of technical features indicated. Thus, a feature defined as "first" or "second" may explicitly or implicitly include at least one such feature. In the description of the present disclosure, the meaning of "plurality" is at least two, e.g., two, three, etc. unless specifically and specifically limited otherwise.

### Recombinant antibody

In a first aspect of the present disclosure, the present disclosure provides a recombinant antibody. According to some specific embodiments of the present disclosure, the recombinant antibody includes a first antigen-binding region for binding to CD3 and a second antigen-binding region for specifically binding to B7H6. The recombinant antibody according to the embodiments of the present disclosure can effectively bind to CD3 on T cells and B7H6 on tumor cells, have a strong *in vivo* and *in vitro* binding activities and a long *in vivo* half-life and the recombinant antibody can form cell synapses by simultaneously binding to T cells and tumor cells, thereby having a significant anti-tumor effect.

According to some specific embodiments of the present disclosure, the above-mentioned recombinant antibody may further include at least one of the following additional technical features.

According to some specific embodiments of the present disclosure, the recombinant antibody includes a CDR sequence selected from at least one of the following sequences, or an amino acid sequence having at least 95% identity thereto: CD3 antibody variable region CDR sequences: SEQ ID NO: 1 to SEQ ID NO: 6; and B7H6 antibody variable region CDR sequences: SEQ ID NO: 7 to SEQ ID NO: 12. The recombinant antibody according to the embodiments of the present disclosure can effectively bind to CD3 and B7H6, has strong *in vivo* and *in vitro* binding activities, a long *in vivo half-life,* and a significant anti-tumor effect.
RASQDIRNYLN (SEQ ID NO: 1).
YTSRLES (SEQ ID NO: 2).
QQGNTLPWT (SEQ ID NO: 3).
GYTMN (SEQ ID NO: 4).
LINPYKGVSTYNQKFKD (SEQ ID NO: 5).
SGYYGDSDWYFDV (SEQ ID NO: 6).
KASQSVDYDGDSYMN (SEQ ID NO: 7).
AASTLHS (SEQ ID NO: 8).
QQSKEDPRT (SEQ ID NO: 9).
DYNMD (SEQ ID NO: 10).
DINPNNGGTLYNQKFRG (SEQ ID NO: 11).
SEVFYGNYADY (SEQ ID NO: 12).

According to some specific embodiments of the present disclosure, the recombinant antibody includes CD3 antibody variable regions. The CD3 antibody variable regions include a light chain variable region as set forth in SEQ ID NO: 13 and a heavy chain variable region as set forth in SEQ ID NO: 14; or an amino acid sequence having at least 70%, at least 75%, at least 80%, at least 85%, at least 90%, at least 95%, or at least 99% identity to an amino acid sequence as set forth in SEQ ID NO: 13, and an amino acid sequence having at least 70%, at least 75%, at least 80%, at least 85%, at least 90%, at least 95%, or at least 99% identity to an amino acid sequence as set forth in SEQ ID NO: 14.

MDIQMTQSPSSLSASVGDRVTITCRASQDIRNYLNWYQQKPGKAPKLLIYY
TSRLESGVPSRFSGSGSGTDYTLTISSLQPEDFATYYCQQGNTLPWTFGQGTKVEIK
(SEQ ID NO: 13).




 (SEQ ID NO: 14).

According to some specific embodiments of the present disclosure, the antibody includes B7H6 antibody variable regions. The B7H6 antibody variable regions include: a light chain variable region as set forth in SEQ ID NO: 20 and a heavy chain variable region of the amino acid sequence as set forth in SEQ ID NO: 23; or an amino acid sequence having at least 70%, at least 75%, at least 80%, at least 85%, at least 90%, at least 95%, or at least 99% identity to an amino acid sequence as set forth in SEQ ID NO: 20, and an amino acid sequence having at least 70%, at least 75%, at least 80%, at least 85%, at least 90%, at least 95%, or at least 99% identity to an amino acid sequence as set forth in SEQ ID NO: 23.

DIVLTQTPLSLSVTPGQPASISCKASQSVDYDGDSYMNWYLQKPGQPPQLL
IYAASTLHSGIPDRFSGSGSGTDFTLKISRVEAEDVGVYYCQQSKEDPRTFGQGTKLEI
K (SEQ ID NO: 20).

EVQLVQSGAEVKKPGASVKVSCKASGYTFTDYNMDWVRQAPGQGLEWI
GDINPNNGGTLYNQKFRGRVTLTVDTSISTAYMELSRLRSDDTAVYYCARSEVFYGNY
ADYWGQGTTVTVSS (SEQ ID NO: 23).

According to some specific embodiments of the present disclosure, the B7H6 antibody variable regions include: a light chain variable region as set forth in SEQ ID NO: 16 and a heavy chain variable region of the amino acid sequence as set forth in SEQ ID NO: 22; or an amino acid sequence having at least 70%, at least 75%, at least 80%, at least 85%, at least 90%, at least 95%, or at least 99% identity to an amino acid sequence as set forth in SEQ ID NO: 16, and an amino acid sequence having at least 70%, at least 75%, at least 80%, at least 85%, at least 90%, at least 95%, or at least 99% identity to an amino acid sequence as set forth in SEQ ID NO: 22.

According to some specific embodiments of the present disclosure, the B7H6 antibody variable regions include: a light chain variable region as set forth in SEQ ID NO: 16 and a heavy chain variable region of the amino acid sequence as set forth in SEQ ID NO: 23; or an amino acid sequence having at least 70%, at least 75%, at least 80%, at least 85%, at least 90%, at least 95%, or at least 99% identity to an amino acid sequence as set forth in SEQ ID NO: 16, and an amino acid sequence having at least 70%, at least 75%, at least 80%, at least 85%, at least 90%, at least 95%, or at least 99% identity to an amino acid sequence as set forth in SEQ ID NO: 23.

According to some specific embodiments of the present disclosure, the B7H6 antibody variable regions include: a light chain variable region as set forth in SEQ ID NO: 16 and a heavy chain variable region of the amino acid sequence as set forth in SEQ ID NO: 24; or an amino acid sequence having at least 70%, at least 75%, at least 80%, at least 85%, at least 90%, at least 95%, or at least 99% identity to an amino acid sequence as set forth in SEQ ID NO: 16, and an amino acid sequence having at least 70%, at least 75%, at least 80%, at least 85%, at least 90%, at least 95%, or at least 99% identity to an amino acid sequence as set forth in SEQ ID NO: 24.

According to some specific embodiments of the present disclosure, the B7H6 antibody variable regions include: a light chain variable region as set forth in SEQ ID NO: 16 and a heavy chain variable region of the amino acid sequence as set forth in SEQ ID NO: 25; or an amino acid sequence having at least 70%, at least 75%, at least 80%, at least 85%, at least 90%, at least 95%, or at least 99% identity to an amino acid sequence as set forth in SEQ ID NO: 16, and an amino acid sequence having at least 70%, at least 75%, at least 80%, at least 85%, at least 90%, at least 95%, or at least 99% identity to an amino acid sequence as set forth in SEQ ID NO: 25.

According to some specific embodiments of the present disclosure, the B7H6 antibody variable regions include: a light chain variable region as set forth in SEQ ID NO: 17 and a heavy chain variable region of the amino acid sequence as set forth in SEQ ID NO: 22; or an amino acid sequence having at least 70%, at least 75%, at least 80%, at least 85%, at least 90%, at least 95%, or at least 99% identity to an amino acid sequence as set forth in SEQ ID NO: 17, and an amino acid sequence having at least 70%, at least 75%, at least 80%, at least 85%, at least 90%, at least 95%, or at least 99% identity to an amino acid sequence as set forth in SEQ ID NO: 22.

According to some specific embodiments of the present disclosure, the B7H6 antibody variable regions include: a light chain variable region as set forth in SEQ ID NO: 17 and a heavy chain variable region of the amino acid sequence as set forth in SEQ ID NO: 23; or an amino acid sequence having at least 70%, at least 75%, at least 80%, at least 85%, at least 90%, at least 95%, or at least 99% identity to an amino acid sequence as set forth in SEQ ID NO: 17, and an amino acid sequence having at least 70%, at least 75%, at least 80%, at least 85%, at least 90%, at least 95%, or at least 99% identity to an amino acid sequence as set forth in SEQ ID NO: 23.

According to some specific embodiments of the present disclosure, the B7H6 antibody variable regions include: a light chain variable region as set forth in SEQ ID NO: 17 and a heavy chain variable region of the amino acid sequence as set forth in SEQ ID NO: 24; or an amino acid sequence having at least 70%, at least 75%, at least 80%, at least 85%, at least 90%, at least 95%, or at least 99% identity to an amino acid sequence as set forth in SEQ ID NO: 17, and an amino acid sequence having at least 70%, at least 75%, at least 80%, at least 85%, at least 90%, at least 95%, or at least 99% identity to an amino acid sequence as set forth in SEQ ID NO: 24.

According to some specific embodiments of the present disclosure, the B7H6 antibody variable regions include: a light chain variable region as set forth in SEQ ID NO: 17 and a heavy chain variable region of the amino acid sequence as set forth in SEQ ID NO: 25; or an amino acid sequence having at least 70%, at least 75%, at least 80%, at least 85%, at least 90%, at least 95%, or at least 99% identity to an amino acid sequence as set forth in SEQ ID NO: 17, and an amino acid sequence having at least 70%, at least 75%, at least 80%, at least 85%, at least 90%, at least 95%, or at least 99% identity to an amino acid sequence as set forth in SEQ ID NO: 25.

According to some specific embodiments of the present disclosure, the B7H6 antibody variable regions include: a light chain variable region as set forth in SEQ ID NO: 19 and a heavy chain variable region of the amino acid sequence as set forth in SEQ ID NO: 22; or an amino acid sequence having at least 70%, at least 75%, at least 80%, at least 85%, at least 90%, at least 95%, or at least 99% identity to an amino acid sequence as set forth in SEQ ID NO: 19, and an amino acid sequence having at least 70%, at least 75%, at least 80%, at least 85%, at least 90%, at least 95%, or at least 99% identity to an amino acid sequence as set forth in SEQ ID NO: 22.

According to some specific embodiments of the present disclosure, the B7H6 antibody variable regions include: a light chain variable region as set forth in SEQ ID NO: 19 and a heavy chain variable region of the amino acid sequence as set forth in SEQ ID NO: 23; or an amino acid sequence having at least 70%, at least 75%, at least 80%, at least 85%, at least 90%, at least 95%, or at least 99% identity to an amino acid sequence as set forth in SEQ ID NO: 19, and an amino acid sequence having at least 70%, at least 75%, at least 80%, at least 85%, at least 90%, at least 95%, or at least 99% identity to an amino acid sequence as set forth in SEQ ID NO: 23.

According to some specific embodiments of the present disclosure, the B7H6 antibody variable regions include: a light chain variable region as set forth in SEQ ID NO: 19 and a heavy chain variable region of the amino acid sequence as set forth in SEQ ID NO: 24; or an amino acid sequence having at least 70%, at least 75%, at least 80%, at least 85%, at least 90%, at least 95%, or at least 99% identity to an amino acid sequence as set forth in SEQ ID NO: 19, and an amino acid sequence having at least 70%, at least 75%, at least 80%, at least 85%, at least 90%, at least 95%, or at least 99% identity to an amino acid sequence as set forth in SEQ ID NO: 24.

According to some specific embodiments of the present disclosure, the B7H6 antibody variable regions include: a light chain variable region as set forth in SEQ ID NO: 19 and a heavy chain variable region of the amino acid sequence as set forth in SEQ ID NO: 25; or an amino acid sequence having at least 70%, at least 75%, at least 80%, at least 85%, at least 90%, at least 95%, or at least 99% identity to an amino acid sequence as set forth in SEQ ID NO: 19, and an amino acid sequence having at least 70%, at least 75%, at least 80%, at least 85%, at least 90%, at least 95%, or at least 99% identity to an amino acid sequence as set forth in SEQ ID NO: 25.

According to some specific embodiments of the present disclosure, the B7H6 antibody variable regions include: a light chain variable region as set forth in SEQ ID NO: 20 and a heavy chain variable region of the amino acid sequence as set forth in SEQ ID NO: 22; or an amino acid sequence having at least 70%, at least 75%, at least 80%, at least 85%, at least 90%, at least 95%, or at least 99% identity to an amino acid sequence as set forth in SEQ ID NO: 20, and an amino acid sequence having at least 70%, at least 75%, at least 80%, at least 85%, at least 90%, at least 95%, or at least 99% identity to an amino acid sequence as set forth in SEQ ID NO: 22.

According to some specific embodiments of the present disclosure, the B7H6 antibody variable regions include: a light chain variable region as set forth in SEQ ID NO: 20 and a heavy chain variable region of the amino acid sequence as set forth in SEQ ID NO: 24; or an amino acid sequence having at least 70%, at least 75%, at least 80%, at least 85%, at least 90%, at least 95%, or at least 99% identity to an amino acid sequence as set forth in SEQ ID NO: 20, and an amino acid sequence having at least 70%, at least 75%, at least 80%, at least 85%, at least 90%, at least 95%, or at least 99% identity to an amino acid sequence as set forth in SEQ ID NO: 24.

According to some specific embodiments of the present disclosure, the B7H6 antibody variable regions include: a light chain variable region as set forth in SEQ ID NO: 20 and a heavy chain variable region of the amino acid sequence as set forth in SEQ ID NO: 25; or an amino acid sequence having at least 70%, at least 75%, at least 80%, at least 85%, at least 90%, at least 95%, or at least 99% identity to an amino acid sequence as set forth in SEQ ID NO: 20, and an amino acid sequence having at least 70%, at least 75%, at least 80%, at least 85%, at least 90%, at least 95%, or at least 99% identity to an amino acid sequence as set forth in SEQ ID NO: 25.

According to some specific embodiments of the present disclosure, the B7H6 antibody variable regions include: a light chain variable region as set forth in SEQ ID NO: 15 and a heavy chain variable region of the amino acid sequence as set forth in SEQ ID NO: 26; or an amino acid sequence having at least 70%, at least 75%, at least 80%, at least 85%, at least 90%, at least 95%, or at least 99% identity to an amino acid sequence as set forth in SEQ ID NO: 15, and an amino acid sequence having at least 70%, at least 75%, at least 80%, at least 85%, at least 90%, at least 95%, or at least 99% identity to an amino acid sequence as set forth in SEQ ID NO: 26.

According to some specific embodiments of the present disclosure, the B7H6 antibody variable regions include: a light chain variable region as set forth in SEQ ID NO: 18 and a heavy chain variable region of the amino acid sequence as set forth in SEQ ID NO: 21; or an amino acid sequence having at least 70%, at least 75%, at least 80%, at least 85%, at least 90%, at least 95%, or at least 99% identity to an amino acid sequence as set forth in SEQ ID NO: 18, and an amino acid sequence having at least 70%, at least 75%, at least 80%, at least 85%, at least 90%, at least 95%, or at least 99% identity to an amino acid sequence as set forth in SEQ ID NO: 21.

DIVLTQSPVSLAVPLGQRATISCKASQSVDYDGDSYMNWYQQKPGQPPKL
LIYAASTLHSGIPVRFSGSGSGTDFTLNIHPVEEEDAASYYCQQSKEDPRTFGGGTKLEI
K (SEQ ID NO: 15).

DIVLTQSPDSLAVSLGERATINCKASQSVDYDGDSYMNWYQQKPGQPPKL
LIYAASTLHSGVPDRFSGSGSGTDFTLTISSLQAEDVAVYYCQQSKEDPRTFGQGTKLEI
K (SEQ ID NO: 16).

DIVLTQSPDSLAVSLGERATINCKASQSVDYDGDSYMNWYQQKPGQPPKL
LIYAASTLHSGIPDRFSGSGSGTDFTLTISSLQAEDVAVYYCQQSKEDPRTFGQGTKLEI
K (SEQ ID NO: 17).

DIVLTQSPVSLAVPLGQRATISCKASQSVDYDADSYMNWYQQKPGQPPKL
LIYAASTLHSGIPVRFSGSGSGTDFTLNIHPVEEEDAASYYCQQSKEDPRTFGGGTKLEI
K (SEQ ID NO: 18).

DIVLTQTPLSLSVTPGQPASISCKASQSVDYDGDSYMNWYLQKPGQPPQLL
IYAASTLHSGVPDRFSGSGSGTDFTLKISRVEAEDVGVYYCQQSKEDPRTFGQGTKLEI
K (SEQ ID NO: 19).

EVLLQQSGPEVVKPGASVKITCKASGYTFTDYNMDWVKQSHGKSLEWIG
DINPNNGGTLYNQKFRGRVTLIVDKSSSTAYMELRSLTSDDTAVYYCARSEVFYGNYA
DYWGQGTTLTVSS (SEQ ID NO: 21).

EVQLVQSGAEVKKPGASVKVSCKASGYTFTDYNMDWVRQAPGQGLEWM
GDINPNNGGTLYNQKFRGRVTMTVDTSISTAYMELSRLRSDDTAVYYCARSEVFYGN
YADYWGQGTTVTVSS (SEQ ID NO: 22).

EVQLVQSGAEVKKPGASVKVSCKASGYTFTDYNMDWVRQAPGQGLEWI
GDINPNNGGTLYNQKFRGRVTLTVDKSISTAYMELSRLRSDDTAVYYCARSEVFYGNY
ADYWGQGTTVTVSS (SEQ ID NO: 24).

EVQLVQSGAEVKKPGASVKVSCKASGYTFTDYNMDWVRQAPGKGLEWI
GDINPNNGGTLYNQKFRGRVTLTVDKSISTAYMELSRLRSDDTAVYYCARSEVFYGNY
ADYWGQGTTVTVSS (SEQ ID NO: 25).

EVLLQQSGPEVVKPGASVKITCKASGYTFTDYNMDWVKQSHGKSLEWIG
DINPNNAGTLYNQKFRGRVTLIVDKSSSTAYMELRSLTSDDTAVYYCARSEVFYGNYA

DYWGQGTTLTVSS (SEQ ID NO: 26).

According to some specific embodiments of the present disclosure, the recombinant antibody further includes a linker peptide.

According to some specific embodiments of the present disclosure, the linker peptide has an amino acid sequence as set forth in SEQ ID NO: 46.
GGGGSGGGGSGGGGS (SEQ ID NO: 46).

According to some specific embodiments of the present disclosure, an N-end of the linker peptide is linked to a C-end of an CD3 antibody light chain variable region, and a C-end of the linker peptide is linked to an N-end of an CD3 antibody heavy chain variable region.

According to some specific embodiments of the present disclosure, an N-end of the linker peptide is linked to a C-end of a B7H6 light chain variable region, and a C-end of the linker peptide is linked to an N-end of the B7H6 heavy chain variable region.

According to some specific embodiments of the present disclosure, the recombinant antibody further includes a first Fc region and a second Fc region; at least a portion of the first Fc region and the second Fc region is derived from at least one of a murine antibody, a human antibody, a primate antibody, or a mutant thereof.

According to some specific embodiments of the present disclosure, at least a portion of the first Fc region and the second Fc region is derived from human IgG or a mutant thereof.

According to some specific embodiments of the present disclosure, at least a portion of the first Fc region and the second Fc region is derived from human IgG1 or a mutant thereof.

According to some specific embodiments of the present disclosure, the first Fc region has at least one of S384C mutation and T396W mutation as compared to a Fc region of wild-type IgG1.

According to some specific embodiments of the present disclosure, the second Fc region has at least one of Y380C mutation, T397S mutation, L399A mutation, and Y408V mutation as compared to the Fc region of wild-type IgG1.

According to some specific embodiments of the present disclosure, the first Fc region has an amino acid sequence as set forth in SEQ ID NO: 47, and the second Fc region has an amino acid sequence as set forth in SEQ ID NO: 48.

EPKSCDKTHTCPPCPAPEAAGGPSVFLFPPKPKDTLMISRTPEVTCVVVDVS

HEDPEVKFNWYVDGVEVHNAKTKPREEQYNSTYRVVSVLTVLHQDWLNGKEYKCK
VSNKALPAPIEKTISKAKGQPREPQVYTLPPCRDELTKNQVSLWCLVKGFYPSDIAVE
WESNGQPENNYKTTPPVLDSDGSFFLYSKLTVDKSRWQQGNVFSCSVMHEALHNHY
TQKSLSLSPGK (SEQ ID NO: 47).

EPKSCDKTHTCPPCPAPEAAGGPSVFLFPPKPKDTLMISRTPEVTCVVVDVS
HEDPEVKFNWYVDGVEVHNAKTKPREEQYNSTYRVVSVLTVLHQDWLNGKEYKCK
VSNKALPAPIEKTISKAKGQPREPQVCTLPPSRDELTKNQVSLSCAVKGFYPSDIAVEW
ESNGQPENNYKTTPPVLDSDGSFFLVSKLTVDKSRWQQGNVFSCSVMHEALHNHYT
QKSLSLSPGK (SEQ ID NO: 48).

In a second aspect of the present disclosure, the present disclosure provides a recombinant antibody. According to an embodiment of the present disclosure, the recombinant antibody has an amino acid sequence as set forth in SEQ ID NO: 49 and SEQ ID NO: 63. The recombinant antibody according to the embodiments of the present disclosure can effectively bind to CD3 and B7H6, has strong *in vivo* and *in vitro* binding activities, a long *in vivo* half-life, and a significant anti-tumor effect.

MDIQMTQSPSSLSASVGDRVTITCRASQDIRNYLNWYQQKPGKAPKLLIYY
TSRLESGVPSRFSGSGSGTDYTLTISSLQPEDFATYYCQQGNTLPWTFGQGTKVEIKGG
GGSGGGGSGGGGSEVQLVESGGGLVQPGGSLRLSCAASGYSFTGYTMNWVRQAPGK
GLEWVALINPYKGVSTYNQKFKDRFTISVDKSKNTAYLQMNSLRAEDTAVYYCARSG
YYGDSDWYFDVWGQGTLVTVSSEPKSCDKTHTCPPCPAPEAAGGPSVFLFPPKPKDT
LMISRTPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPCRDELTKNQV
SLWCLVKGFYPSDIAVEWESNGQPENNYKTTPPVLDSDGSFFLYSKLTVDKSRWQQG
NVFSCSVMHEALHNHYTQKSLSLSPGK (SEQ ID NO: 49).

DIVLTQTPLSLSVTPGQPASISCKASQSVDYDGDSYMNWYLQKPGQPPQLL
IYAASTLHSGIPDRFSGSGSGTDFTLKISRVEAEDVGVYYCQQSKEDPRTFGQGTKLEI
KGGGGSGGGGSGGGGSEVQLVQSGAEVKKPGASVKVSCKASGYTFTDYNMDWVR
QAPGQGLEWIGDINPNNGGTLYNQKFRGRVTLTVDTSISTAYMELSRLRSDDTAVYYC
ARSEVFYGNYADYWGQGTTVTVSSEPKSCDKTHTCPPCPAPEAAGGPSVFLFPPKPK
DTLMISRTPEVTCVVVDVSHEDPEVKFNWYVDGVEVHNAKTKPREEQYNSTYRVVS

VLTVLHQDWLNGKEYKCKVSNKALPAPIEKTISKAKGQPREPQVCTLPPSRDELTKNQ
VSLSCAVKGFYPSDIAVEWESNGQPENNYKTTPPVLDSDGSFFLVSKLTVDKSRWQQG
NVFSCSVMHEALHNHYTQKSLSLSPGK (SEQ ID NO: 63).

### A preventive or therapeutic composition

In a third aspect of the present disclosure, the present disclosure provides a nucleic acid encoding the recombinant antibody as described above. The recombinant antibody encoded by the nucleic acid according to some specific embodiments of the present disclosure can effectively bind to CD3 and B7H6, has strong *in vivo* and *in vitro* binding activities, and a long *in vivo* half-life, and a significant anti-tumor effect.

According to some specific embodiments of the present disclosure, the nucleic acid has a nucleotide sequence as set forth in SEQ ID NO: 69 and at least one of nucleotide sequences as set forth in SEQ ID NO: 70 to SEQ ID NO: 87.

Nucleotide sequence encoding CD3 antibody

CAGGCCGTGGTGACCCAGGAGCCCTCCCTGACCGTGTCCCCCGGCGGC
ACCGTGACCCTGACCTGCAGGTCCTCCACCGGCGCCGTGACCACCTCCAACTACG
CCAACTGGGTGCAGCAGAAGCCCGGCCAGGCCCCCAGGGGCCTGATCGGCGGCA
CCAACAAGAGGGCCCCCTGGACCCCCGCCAGGTTCTCCGGCTCCCTGCTGGGCGG
CAAGGCCGCCCTGACCATCACCGGCGCCCAGGCCGAGGACGAGGCCGACTACTAC
TGCGCCCTGTGGTACTCCAACCTGTGGGTGTTCGGCGGCGGCACCAAGCTGACCG
TGCTGGGCGGAGGCGGTGGATCAGGCGGAGGTGGCTCAGGTGGAGGCGGTTCTG
AGGTGCAGCTGGTGGAGTCCGGCGGCGGCCTGGTGCAGCCCGGCGGCTCCCTGA
GGCTGTCCTGCGCCGCCTCCGGCTTCACCTTCAACACCTACGCCATGAACTGGGTG
AGGCAGGCCCCCGGCAAGGGCCTGGAGTGGGTGGCCAGGATCAGGTCCAAGTAC
AACAACTACGCCACCTACTACGCCGACTCCGTGAAGGACAGGTTCACCATCTCCA
GGGACGACTCCAAGAACTCCCTGTACCTGCAGATGAACTCCCTGAAGACCGAGGA
CACCGCCGTGTACTACTGCGTGAGGCACGGCAACTTCGGCAACTCCTACGTGTCCT
GGTTCGCCTACTGGGGCCAGGGCACCCTGGTGACCGTGTCCTCCGAGCCCAAGTC
CTGCGACAAGACCCACACCTGCCCCCCCTGCCCCGCCCCCGAGGCCGCCGGCGGC
CCCTCCGTGTTCCTGTTCCCCCCCAAGCCCAAGGACACCCTGATGATCTCCAGGAC
CCCCGAGGTGACCTGCGTGGTGGTGGACGTGTCCCACGAGGACCCCGAGGTGAA

GTTCAACTGGTACGTGGACGGCGTGGAGGTGCACAACGCCAAGACCAAGCCCAG
GGAGGAGCAGTACAACTCCACCTACAGGGTGGTGTCCGTGCTGACCGTGCTGCAC
CAGGACTGGCTGAACGGCAAGGAGTACAAGTGCAAGGTGTCCAACAAGGCCCTG
CCCGCCCCCATCGAGAAGACCATCTCCAAGGCCAAGGGCCAGCCCAGGGAGCCCC
AGGTGTACACCCTGCCCCCCTGCAGGGACGAGCTGACCAAGAACCAGGTGTCCCT
GTGGTGCCTGGTGAAGGGCTTCTACCCCTCCGACATCGCCGTGGAGTGGGAGTCC
AACGGCCAGCCCGAGAACAACTACAAGACCACCCCCCCCGTGCTGGACTCCGAC
GGCTCCTTCTTCCTGTACTCCAAGCTGACCGTGGACAAGTCCAGGTGGCAGCAGG
GCAACGTGTTCTCCTGCTCCGTGATGCACGAGGCCCTGCACAACCACTACACCCA
GAAGTCCCTGTCCCTGTCCCCCGGCAAGTAA (SEQ ID NO: 69).

Nucleotide sequence encoding B7H6-1 antibody

GACATCGTGCTGACCCAGTCCCCCGACTCCCTGGCCGTGTCCCTGGGCG
AGAGGGCCACCATCAACTGCAAGGCCTCCCAGTCCGTGGACTACGACGGCGACTC
CTACATGAACTGGTACCAGCAGAAGCCCGGCCAGCCCCCCAAGCTGCTGATCTAC
GCCGCCTCCACCCTGCACTCCGGCGTGCCCGACAGGTTCTCCGGCTCCGGCTCCG
GCACCGACTTCACCCTGACCATCTCCTCCCTGCAGGCCGAGGACGTGGCCGTGTA
CTACTGCCAGCAGTCCAAGGAGGACCCCAGGACCTTCGGCCAGGGCACCAAGCT
GGAGATCAAGGGAGGCGGTGGATCAGGCGGAGGTGGCTCAGGTGGAGGCGGTTC
TGAGGTGCAGCTGGTGCAGTCCGGCGCCGAGGTGAAGAAGCCCGGCGCCTCCGT
GAAGGTGTCCTGCAAGGCCTCCGGCTACACCTTCACCGACTACAACATGGACTGG
GTGAGGCAGGCCCCCGGCCAGGGCCTGGAGTGGATGGGCGACATCAACCCCAAC
AACGGCGGCACCCTGTACAACCAGAAGTTCAGGGGCAGGGTGACCATGACCGTG
GACACCTCCATCTCCACCGCCTACATGGAGCTGTCCAGGCTGAGGTCCGACGACA
CCGCCGTGTACTACTGCGCCAGGTCCGAGGTGTTCTACGGCAACTACGCCGACTAC
TGGGGCCAGGGCACCACCGTGACCGTGTCCTCCGAGCCCAAGTCCTGCGACAAG
ACCCACACCTGCCCCCCCTGCCCCGCCCCCGAGGCCGCCGGCGGCCCCTCCGTGT
TCCTGTTCCCCCCCAAGCCCAAGGACACCCTGATGATCTCCAGGACCCCCGAGGT
GACCTGCGTGGTGGTGGACGTGTCCCACGAGGACCCCGAGGTGAAGTTCAACTG
GTACGTGGACGGCGTGGAGGTGCACAACGCCAAGACCAAGCCCAGGGAGGAGCA
GTACAACTCCACCTACAGGGTGGTGTCCGTGCTGACCGTGCTGCACCAGGACTGG

CTGAACGGCAAGGAGTACAAGTGCAAGGTGTCCAACAAGGCCCTGCCCGCCCCC
ATCGAGAAGACCATCTCCAAGGCCAAGGGCCAGCCCAGGGAGCCCCAGGTGTGC
ACCCTGCCCCCCTCCAGGGACGAGCTGACCAAGAACCAGGTGTCCCTGTCCTGCG
CCGTGAAGGGCTTCTACCCCTCCGACATCGCCGTGGAGTGGGAGTCCAACGGCCA
GCCCGAGAACAACTACAAGACCACCCCCCCCGTGCTGGACTCCGACGGCTCCTTC
TTCCTGGTGTCCAAGCTGACCGTGGACAAGTCCAGGTGGCAGCAGGGCAACGTGT
TCTCCTGCTCCGTGATGCACGAGGCCCTGCACAACCACTACACCCAGAAGTCCCT
GTCCCTGTCCCCCGGCAAGTAA (SEQ ID NO: 70).

Nucleotide sequence encoding B7H6-2 antibody

GACATCGTGCTGACCCAGTCCCCCGACTCCCTGGCCGTGTCCCTGGGCG
AGAGGGCCACCATCAACTGCAAGGCCTCCCAGTCCGTGGACTACGACGGCGACTC
CTACATGAACTGGTACCAGCAGAAGCCCGGCCAGCCCCCCAAGCTGCTGATCTAC
GCCGCCTCCACCCTGCACTCCGGCGTGCCCGACAGGTTCTCCGGCTCCGGCTCCG
GCACCGACTTCACCCTGACCATCTCCTCCCTGCAGGCCGAGGACGTGGCCGTGTA
CTACTGCCAGCAGTCCAAGGAGGACCCCAGGACCTTCGGCCAGGGCACCAAGCT
GGAGATCAAGGGAGGCGGTGGATCAGGCGGAGGTGGCTCAGGTGGAGGCGGTTC
TGAGGTGCAGCTGGTGCAGTCCGGCGCCGAGGTGAAGAAGCCCGGCGCCTCCGT
GAAGGTGTCCTGCAAGGCCTCCGGCTACACCTTCACCGACTACAACATGGACTGG
GTGAGGCAGGCCCCCGGCCAGGGCCTGGAGTGGATCGGCGACATCAACCCCAAC
AACGGCGGCACCCTGTACAACCAGAAGTTCAGGGGCAGGGTGACCCTGACCGTG
GACACCTCCATCTCCACCGCCTACATGGAGCTGTCCAGGCTGAGGTCCGACGACA
CCGCCGTGTACTACTGCGCCAGGTCCGAGGTGTTCTACGGCAACTACGCCGACTAC
TGGGGCCAGGGCACCACCGTGACCGTGTCCTCCGAGCCCAAGTCCTGCGACAAG
ACCCACACCTGCCCCCCCTGCCCCGCCCCCGAGGCCGCCGGCGGCCCCTCCGTGT
TCCTGTTCCCCCCCAAGCCCAAGGACACCCTGATGATCTCCAGGACCCCCGAGGT
GACCTGCGTGGTGGTGGACGTGTCCCACGAGGACCCCGAGGTGAAGTTCAACTG
GTACGTGGACGGCGTGGAGGTGCACAACGCCAAGACCAAGCCCAGGGAGGAGCA
GTACAACTCCACCTACAGGGTGGTGTCCGTGCTGACCGTGCTGCACCAGGACTGG
CTGAACGGCAAGGAGTACAAGTGCAAGGTGTCCAACAAGGCCCTGCCCGCCCCC
ATCGAGAAGACCATCTCCAAGGCCAAGGGCCAGCCCAGGGAGCCCCAGGTGTGC

ACCCTGCCCCCCTCCAGGGACGAGCTGACCAAGAACCAGGTGTCCCTGTCCTGCG
CCGTGAAGGGCTTCTACCCCTCCGACATCGCCGTGGAGTGGGAGTCCAACGGCCA
GCCCGAGAACAACTACAAGACCACCCCCCCCGTGCTGGACTCCGACGGCTCCTTC
TTCCTGGTGTCCAAGCTGACCGTGGACAAGTCCAGGTGGCAGCAGGGCAACGTGT
TCTCCTGCTCCGTGATGCACGAGGCCCTGCACAACCACTACACCCAGAAGTCCCT
GTCCCTGTCCCCCGGCAAGTAA (SEQ ID NO: 71).

Nucleotide sequence encoding B7H6-3 antibody

GACATCGTGCTGACCCAGTCCCCCGACTCCCTGGCCGTGTCCCTGGGCG
AGAGGGCCACCATCAACTGCAAGGCCTCCCAGTCCGTGGACTACGACGGCGACTC
CTACATGAACTGGTACCAGCAGAAGCCCGGCCAGCCCCCCAAGCTGCTGATCTAC
GCCGCCTCCACCCTGCACTCCGGCGTGCCCGACAGGTTCTCCGGCTCCGGCTCCG
GCACCGACTTCACCCTGACCATCTCCTCCCTGCAGGCCGAGGACGTGGCCGTGTA
CTACTGCCAGCAGTCCAAGGAGGACCCCAGGACCTTCGGCCAGGGCACCAAGCT
GGAGATCAAGGGAGGCGGTGGATCAGGCGGAGGTGGCTCAGGTGGAGGCGGTTC
TGAGGTGCAGCTGGTGCAGTCCGGCGCCGAGGTGAAGAAGCCCGGCGCCTCCGT
GAAGGTGTCCTGCAAGGCCTCCGGCTACACCTTCACCGACTACAACATGGACTGG
GTGAGGCAGGCCCCCGGCCAGGGCCTGGAGTGGATCGGCGACATCAACCCCAAC
AACGGCGGCACCCTGTACAACCAGAAGTTCAGGGGCAGGGTGACCCTGACCGTG
GACAAGTCCATCTCCACCGCCTACATGGAGCTGTCCAGGCTGAGGTCCGACGACA
CCGCCGTGTACTACTGCGCCAGGTCCGAGGTGTTCTACGGCAACTACGCCGACTAC
TGGGGCCAGGGCACCACCGTGACCGTGTCCTCCGAGCCCAAGTCCTGCGACAAG
ACCCACACCTGCCCCCCCTGCCCCGCCCCCGAGGCCGCCGGCGGCCCCTCCGTGT
TCCTGTTCCCCCCCAAGCCCAAGGACACCCTGATGATCTCCAGGACCCCCGAGGT
GACCTGCGTGGTGGTGGACGTGTCCCACGAGGACCCCGAGGTGAAGTTCAACTG
GTACGTGGACGGCGTGGAGGTGCACAACGCCAAGACCAAGCCCAGGGAGGAGCA
GTACAACTCCACCTACAGGGTGGTGTCCGTGCTGACCGTGCTGCACCAGGACTGG
CTGAACGGCAAGGAGTACAAGTGCAAGGTGTCCAACAAGGCCCTGCCCGCCCCC
ATCGAGAAGACCATCTCCAAGGCCAAGGGCCAGCCCAGGGAGCCCCAGGTGTGC
ACCCTGCCCCCCTCCAGGGACGAGCTGACCAAGAACCAGGTGTCCCTGTCCTGCG
CCGTGAAGGGCTTCTACCCCTCCGACATCGCCGTGGAGTGGGAGTCCAACGGCCA

GCCCGAGAACAACTACAAGACCACCCCCCCCGTGCTGGACTCCGACGGCTCCTTC
TTCCTGGTGTCCAAGCTGACCGTGGACAAGTCCAGGTGGCAGCAGGGCAACGTGT
TCTCCTGCTCCGTGATGCACGAGGCCCTGCACAACCACTACACCCAGAAGTCCCT
GTCCCTGTCCCCCGGCAAGTAA (SEQ ID NO: 72).

Nucleotide sequence encoding B7H6-4 antibody

GACATCGTGCTGACCCAGTCCCCCGACTCCCTGGCCGTGTCCCTGGGCG
AGAGGGCCACCATCAACTGCAAGGCCTCCCAGTCCGTGGACTACGACGGCGACTC
CTACATGAACTGGTACCAGCAGAAGCCCGGCCAGCCCCCCAAGCTGCTGATCTAC
GCCGCCTCCACCCTGCACTCCGGCGTGCCCGACAGGTTCTCCGGCTCCGGCTCCG
GCACCGACTTCACCCTGACCATCTCCTCCCTGCAGGCCGAGGACGTGGCCGTGTA
CTACTGCCAGCAGTCCAAGGAGGACCCCAGGACCTTCGGCCAGGGCACCAAGCT
GGAGATCAAGGGAGGCGGTGGATCAGGCGGAGGTGGCTCAGGTGGAGGCGGTTC
TGAGGTGCAGCTGGTGCAGTCCGGCGCCGAGGTGAAGAAGCCCGGCGCCTCCGT
GAAGGTGTCCTGCAAGGCCTCCGGCTACACCTTCACCGACTACAACATGGACTGG
GTGAGGCAGGCCCCCGGCAAGGGCCTGGAGTGGATCGGCGACATCAACCCCAAC
AACGGCGGCACCCTGTACAACCAGAAGTTCAGGGGCAGGGTGACCCTGACCGTG
GACAAGTCCATCTCCACCGCCTACATGGAGCTGTCCAGGCTGAGGTCCGACGACA
CCGCCGTGTACTACTGCGCCAGGTCCGAGGTGTTCTACGGCAACTACGCCGACTAC
TGGGGCCAGGGCACCACCGTGACCGTGTCCTCCGAGCCCAAGTCCTGCGACAAG
ACCCACACCTGCCCCCCCTGCCCCGCCCCCGAGGCCGCCGGCGGCCCCTCCGTGT
TCCTGTTCCCCCCCAAGCCCAAGGACACCCTGATGATCTCCAGGACCCCCGAGGT
GACCTGCGTGGTGGTGGACGTGTCCCACGAGGACCCCGAGGTGAAGTTCAACTG
GTACGTGGACGGCGTGGAGGTGCACAACGCCAAGACCAAGCCCAGGGAGGAGCA
GTACAACTCCACCTACAGGGTGGTGTCCGTGCTGACCGTGCTGCACCAGGACTGG
CTGAACGGCAAGGAGTACAAGTGCAAGGTGTCCAACAAGGCCCTGCCCGCCCCC
ATCGAGAAGACCATCTCCAAGGCCAAGGGCCAGCCCAGGGAGCCCCAGGTGTGC
ACCCTGCCCCCCTCCAGGGACGAGCTGACCAAGAACCAGGTGTCCCTGTCCTGCG
CCGTGAAGGGCTTCTACCCCTCCGACATCGCCGTGGAGTGGGAGTCCAACGGCCA
GCCCGAGAACAACTACAAGACCACCCCCCCCGTGCTGGACTCCGACGGCTCCTTC
TTCCTGGTGTCCAAGCTGACCGTGGACAAGTCCAGGTGGCAGCAGGGCAACGTGT

TCTCCTGCTCCGTGATGCACGAGGCCCTGCACAACCACTACACCCAGAAGTCCCT
GTCCCTGTCCCCCGGCAAGTAA (SEQ ID NO: 73).

Nucleotide sequence encoding B7H6-5 antibody

GACATCGTGCTGACCCAGTCCCCCGACTCCCTGGCCGTGTCCCTGGGCG
AGAGGGCCACCATCAACTGCAAGGCCTCCCAGTCCGTGGACTACGACGGCGACTC
CTACATGAACTGGTACCAGCAGAAGCCCGGCCAGCCCCCCAAGCTGCTGATCTAC
GCCGCCTCCACCCTGCACTCCGGCATCCCCGACAGGTTCTCCGGCTCCGGCTCCGG
CACCGACTTCACCCTGACCATCTCCTCCCTGCAGGCCGAGGACGTGGCCGTGTACT
ACTGCCAGCAGTCCAAGGAGGACCCCAGGACCTTCGGCCAGGGCACCAAGCTGG
AGATCAAGGGAGGCGGTGGATCAGGCGGAGGTGGCTCAGGTGGAGGCGGTTCTG
AGGTGCAGCTGGTGCAGTCCGGCGCCGAGGTGAAGAAGCCCGGCGCCTCCGTGA
AGGTGTCCTGCAAGGCCTCCGGCTACACCTTCACCGACTACAACATGGACTGGGT
GAGGCAGGCCCCCGGCCAGGGCCTGGAGTGGATGGGCGACATCAACCCCAACAA
CGGCGGCACCCTGTACAACCAGAAGTTCAGGGGCAGGGTGACCATGACCGTGGA
CACCTCCATCTCCACCGCCTACATGGAGCTGTCCAGGCTGAGGTCCGACGACACC
GCCGTGTACTACTGCGCCAGGTCCGAGGTGTTCTACGGCAACTACGCCGACTACTG
GGGCCAGGGCACCACCGTGACCGTGTCCTCCGAGCCCAAGTCCTGCGACAAGAC
CCACACCTGCCCCCCCTGCCCCGCCCCCGAGGCCGCCGGCGGCCCCTCCGTGTTC
CTGTTCCCCCCCAAGCCCAAGGACACCCTGATGATCTCCAGGACCCCCGAGGTGA
CCTGCGTGGTGGTGGACGTGTCCCACGAGGACCCCGAGGTGAAGTTCAACTGGTA
CGTGGACGGCGTGGAGGTGCACAACGCCAAGACCAAGCCCAGGGAGGAGCAGTA
CAACTCCACCTACAGGGTGGTGTCCGTGCTGACCGTGCTGCACCAGGACTGGCTG
AACGGCAAGGAGTACAAGTGCAAGGTGTCCAACAAGGCCCTGCCCGCCCCCATC
GAGAAGACCATCTCCAAGGCCAAGGGCCAGCCCAGGGAGCCCCAGGTGTGCACC
CTGCCCCCCTCCAGGGACGAGCTGACCAAGAACCAGGTGTCCCTGTCCTGCGCCG
TGAAGGGCTTCTACCCCTCCGACATCGCCGTGGAGTGGGAGTCCAACGGCCAGCC
CGAGAACAACTACAAGACCACCCCCCCCGTGCTGGACTCCGACGGCTCCTTCTTC
CTGGTGTCCAAGCTGACCGTGGACAAGTCCAGGTGGCAGCAGGGCAACGTGTTCT
CCTGCTCCGTGATGCACGAGGCCCTGCACAACCACTACACCCAGAAGTCCCTGTC
CCTGTCCCCCGGCAAGTAA (SEQ ID NO: 74).

Nucleotide sequence encoding B7H6-6 antibody

GACATCGTGCTGACCCAGTCCCCCGACTCCCTGGCCGTGTCCCTGGGCG
AGAGGGCCACCATCAACTGCAAGGCCTCCCAGTCCGTGGACTACGACGGCGACTC
CTACATGAACTGGTACCAGCAGAAGCCCGGCCAGCCCCCCAAGCTGCTGATCTAC
GCCGCCTCCACCCTGCACTCCGGCATCCCCGACAGGTTCTCCGGCTCCGGCTCCGG
CACCGACTTCACCCTGACCATCTCCTCCCTGCAGGCCGAGGACGTGGCCGTGTACT
ACTGCCAGCAGTCCAAGGAGGACCCCAGGACCTTCGGCCAGGGCACCAAGCTGG
AGATCAAGGGAGGCGGTGGATCAGGCGGAGGTGGCTCAGGTGGAGGCGGTTCTG
AGGTGCAGCTGGTGCAGTCCGGCGCCGAGGTGAAGAAGCCCGGCGCCTCCGTGA
AGGTGTCCTGCAAGGCCTCCGGCTACACCTTCACCGACTACAACATGGACTGGGT
GAGGCAGGCCCCCGGCCAGGGCCTGGAGTGGATCGGCGACATCAACCCCAACAA
CGGCGGCACCCTGTACAACCAGAAGTTCAGGGGCAGGGTGACCCTGACCGTGGA
CACCTCCATCTCCACCGCCTACATGGAGCTGTCCAGGCTGAGGTCCGACGACACC
GCCGTGTACTACTGCGCCAGGTCCGAGGTGTTCTACGGCAACTACGCCGACTACTG
GGGCCAGGGCACCACCGTGACCGTGTCCTCCGAGCCCAAGTCCTGCGACAAGAC
CCACACCTGCCCCCCCTGCCCCGCCCCCGAGGCCGCCGGCGGCCCCTCCGTGTTC
CTGTTCCCCCCCAAGCCCAAGGACACCCTGATGATCTCCAGGACCCCCGAGGTGA
CCTGCGTGGTGGTGGACGTGTCCCACGAGGACCCCGAGGTGAAGTTCAACTGGTA
CGTGGACGGCGTGGAGGTGCACAACGCCAAGACCAAGCCCAGGGAGGAGCAGTA
CAACTCCACCTACAGGGTGGTGTCCGTGCTGACCGTGCTGCACCAGGACTGGCTG
AACGGCAAGGAGTACAAGTGCAAGGTGTCCAACAAGGCCCTGCCCGCCCCCATC
GAGAAGACCATCTCCAAGGCCAAGGGCCAGCCCAGGGAGCCCCAGGTGTGCACC
CTGCCCCCCTCCAGGGACGAGCTGACCAAGAACCAGGTGTCCCTGTCCTGCGCCG
TGAAGGGCTTCTACCCCTCCGACATCGCCGTGGAGTGGGAGTCCAACGGCCAGCC
CGAGAACAACTACAAGACCACCCCCCCCGTGCTGGACTCCGACGGCTCCTTCTTC
CTGGTGTCCAAGCTGACCGTGGACAAGTCCAGGTGGCAGCAGGGCAACGTGTTCT
CCTGCTCCGTGATGCACGAGGCCCTGCACAACCACTACACCCAGAAGTCCCTGTC
CCTGTCCCCCGGCAAGTAA (SEQ ID NO: 75).

Nucleotide sequence encoding B7H6-7 antibody

GACATCGTGCTGACCCAGTCCCCCGACTCCCTGGCCGTGTCCCTGGGCG

AGAGGGCCACCATCAACTGCAAGGCCTCCCAGTCCGTGGACTACGACGGCGACTC
CTACATGAACTGGTACCAGCAGAAGCCCGGCCAGCCCCCCAAGCTGCTGATCTAC
GCCGCCTCCACCCTGCACTCCGGCATCCCCGACAGGTTCTCCGGCTCCGGCTCCGG
CACCGACTTCACCCTGACCATCTCCTCCCTGCAGGCCGAGGACGTGGCCGTGTACT
ACTGCCAGCAGTCCAAGGAGGACCCCAGGACCTTCGGCCAGGGCACCAAGCTGG
AGATCAAGGGAGGCGGTGGATCAGGCGGAGGTGGCTCAGGTGGAGGCGGTTCTG
AGGTGCAGCTGGTGCAGTCCGGCGCCGAGGTGAAGAAGCCCGGCGCCTCCGTGA
AGGTGTCCTGCAAGGCCTCCGGCTACACCTTCACCGACTACAACATGGACTGGGT
GAGGCAGGCCCCCGGCCAGGGCCTGGAGTGGATCGGCGACATCAACCCCAACAA
CGGCGGCACCCTGTACAACCAGAAGTTCAGGGGCAGGGTGACCCTGACCGTGGA
CAAGTCCATCTCCACCGCCTACATGGAGCTGTCCAGGCTGAGGTCCGACGACACC
GCCGTGTACTACTGCGCCAGGTCCGAGGTGTTCTACGGCAACTACGCCGACTACTG
GGGCCAGGGCACCACCGTGACCGTGTCCTCCGAGCCCAAGTCCTGCGACAAGAC
CCACACCTGCCCCCCCTGCCCCGCCCCCGAGGCCGCCGGCGGCCCCTCCGTGTTC
CTGTTCCCCCCCAAGCCCAAGGACACCCTGATGATCTCCAGGACCCCCGAGGTGA
CCTGCGTGGTGGTGGACGTGTCCCACGAGGACCCCGAGGTGAAGTTCAACTGGTA
CGTGGACGGCGTGGAGGTGCACAACGCCAAGACCAAGCCCAGGGAGGAGCAGTA
CAACTCCACCTACAGGGTGGTGTCCGTGCTGACCGTGCTGCACCAGGACTGGCTG
AACGGCAAGGAGTACAAGTGCAAGGTGTCCAACAAGGCCCTGCCCGCCCCCATC
GAGAAGACCATCTCCAAGGCCAAGGGCCAGCCCAGGGAGCCCCAGGTGTGCACC
CTGCCCCCCTCCAGGGACGAGCTGACCAAGAACCAGGTGTCCCTGTCCTGCGCCG
TGAAGGGCTTCTACCCCTCCGACATCGCCGTGGAGTGGGAGTCCAACGGCCAGCC
CGAGAACAACTACAAGACCACCCCCCCCGTGCTGGACTCCGACGGCTCCTTCTTC
CTGGTGTCCAAGCTGACCGTGGACAAGTCCAGGTGGCAGCAGGGCAACGTGTTCT
CCTGCTCCGTGATGCACGAGGCCCTGCACAACCACTACACCCAGAAGTCCCTGTC
CCTGTCCCCCGGCAAGTAA (SEQ ID NO: 76).

Nucleotide sequence encoding B7H6-8 antibody

GACATCGTGCTGACCCAGTCCCCCGACTCCCTGGCCGTGTCCCTGGGCG
AGAGGGCCACCATCAACTGCAAGGCCTCCCAGTCCGTGGACTACGACGGCGACTC
CTACATGAACTGGTACCAGCAGAAGCCCGGCCAGCCCCCCAAGCTGCTGATCTAC

GCCGCCTCCACCCTGCACTCCGGCATCCCCGACAGGTTCTCCGGCTCCGGCTCCGG
CACCGACTTCACCCTGACCATCTCCTCCCTGCAGGCCGAGGACGTGGCCGTGTACT
ACTGCCAGCAGTCCAAGGAGGACCCCAGGACCTTCGGCCAGGGCACCAAGCTGG
AGATCAAGGGAGGCGGTGGATCAGGCGGAGGTGGCTCAGGTGGAGGCGGTTCTG
AGGTGCAGCTGGTGCAGTCCGGCGCCGAGGTGAAGAAGCCCGGCGCCTCCGTGA
AGGTGTCCTGCAAGGCCTCCGGCTACACCTTCACCGACTACAACATGGACTGGGT
GAGGCAGGCCCCCGGCAAGGGCCTGGAGTGGATCGGCGACATCAACCCCAACAA
CGGCGGCACCCTGTACAACCAGAAGTTCAGGGGCAGGGTGACCCTGACCGTGGA
CAAGTCCATCTCCACCGCCTACATGGAGCTGTCCAGGCTGAGGTCCGACGACACC
GCCGTGTACTACTGCGCCAGGTCCGAGGTGTTCTACGGCAACTACGCCGACTACTG
GGGCCAGGGCACCACCGTGACCGTGTCCTCCGAGCCCAAGTCCTGCGACAAGAC
CCACACCTGCCCCCCCTGCCCCGCCCCCGAGGCCGCCGGCGGCCCCTCCGTGTTC
CTGTTCCCCCCCAAGCCCAAGGACACCCTGATGATCTCCAGGACCCCCGAGGTGA
CCTGCGTGGTGGTGGACGTGTCCCACGAGGACCCCGAGGTGAAGTTCAACTGGTA
CGTGGACGGCGTGGAGGTGCACAACGCCAAGACCAAGCCCAGGGAGGAGCAGTA
CAACTCCACCTACAGGGTGGTGTCCGTGCTGACCGTGCTGCACCAGGACTGGCTG
AACGGCAAGGAGTACAAGTGCAAGGTGTCCAACAAGGCCCTGCCCGCCCCCATC
GAGAAGACCATCTCCAAGGCCAAGGGCCAGCCCAGGGAGCCCCAGGTGTGCACC
CTGCCCCCCTCCAGGGACGAGCTGACCAAGAACCAGGTGTCCCTGTCCTGCGCCG
TGAAGGGCTTCTACCCCTCCGACATCGCCGTGGAGTGGGAGTCCAACGGCCAGCC
CGAGAACAACTACAAGACCACCCCCCCCGTGCTGGACTCCGACGGCTCCTTCTTC
CTGGTGTCCAAGCTGACCGTGGACAAGTCCAGGTGGCAGCAGGGCAACGTGTTCT
CCTGCTCCGTGATGCACGAGGCCCTGCACAACCACTACACCCAGAAGTCCCTGTC
CCTGTCCCCCGGCAAGTAA (SEQ ID NO: 77).

Nucleotide sequence encoding B7H6-9 antibody

GACATCGTGCTGACCCAGACCCCCCTGTCCCTGTCCGTGACCCCCGGCC
AGCCCGCCTCCATCTCCTGCAAGGCCTCCCAGTCCGTGGACTACGACGGCGACTC
CTACATGAACTGGTACCTGCAGAAGCCCGGCCAGCCCCCCCAGCTGCTGATCTACG
CCGCCTCCACCCTGCACTCCGGCGTGCCCGACAGGTTCTCCGGCTCCGGCTCCGG
CACCGACTTCACCCTGAAGATCTCCAGGGTGGAGGCCGAGGACGTGGGCGTGTAC

TACTGCCAGCAGTCCAAGGAGGACCCCAGGACCTTCGGCCAGGGCACCAAGCTG
GAGATCAAGGGAGGCGGTGGATCAGGCGGAGGTGGCTCAGGTGGAGGCGGTTCT
GAGGTGCAGCTGGTGCAGTCCGGCGCCGAGGTGAAGAAGCCCGGCGCCTCCGTG
AAGGTGTCCTGCAAGGCCTCCGGCTACACCTTCACCGACTACAACATGGACTGGG
TGAGGCAGGCCCCCGGCCAGGGCCTGGAGTGGATGGGCGACATCAACCCCAACA
ACGGCGGCACCCTGTACAACCAGAAGTTCAGGGGCAGGGTGACCATGACCGTGG
ACACCTCCATCTCCACCGCCTACATGGAGCTGTCCAGGCTGAGGTCCGACGACAC
CGCCGTGTACTACTGCGCCAGGTCCGAGGTGTTCTACGGCAACTACGCCGACTACT
GGGGCCAGGGCACCACCGTGACCGTGTCCTCCGAGCCCAAGTCCTGCGACAAGA
CCCACACCTGCCCCCCCTGCCCCGCCCCCGAGGCCGCCGGCGCCCCCTCCGTGTT
CCTGTTCCCCCCCAAGCCCAAGGACACCCTGATGATCTCCAGGACCCCCGAGGTG
ACCTGCGTGGTGGTGGACGTGTCCCACGAGGACCCCGAGGTGAAGTTCAACTGGT
ACGTGGACGGCGTGGAGGTGCACAACGCCAAGACCAAGCCCAGGGAGGAGCAGT
ACAACTCCACCTACAGGGTGGTGTCCGTGCTGACCGTGCTGCACCAGGACTGGCT
GAACGGCAAGGAGTACAAGTGCAAGGTGTCCAACAAGGCCCTGCCCGCCCCCAT
CGAGAAGACCATCTCCAAGGCCAAGGGCCAGCCCAGGGAGCCCCAGGTGTGCAC
CCTGCCCCCCTCCAGGGACGAGCTGACCAAGAACCAGGTGTCCCTGTCCTGCGCC
GTGAAGGGCTTCTACCCCTCCGACATCGCCGTGGAGTGGGAGTCCAACGGCCAGC
CCGAGAACAACTACAAGACCACCCCCCCCGTGCTGGACTCCGACGGCTCCTTCTT
CCTGGTGTCCAAGCTGACCGTGGACAAGTCCAGGTGGCAGCAGGGCAACGTGTTC
TCCTGCTCCGTGATGCACGAGGCCCTGCACAACCACTACACCCAGAAGTCCCTGT
CCCTGTCCCCCGGCAAGTAA (SEQ ID NO: 78).

Nucleotide sequence encoding B7H6-10 antibody

GACATCGTGCTGACCCAGACCCCCCTGTCCCTGTCCGTGACCCCCGGCC
AGCCCGCCTCCATCTCCTGCAAGGCCTCCCAGTCCGTGGACTACGACGGCGACTC
CTACATGAACTGGTACCTGCAGAAGCCCGGCCAGCCCCCCCAGCTGCTGATCTACG
CCGCCTCCACCCTGCACTCCGGCGTGCCCGACAGGTTCTCCGGCTCCGGCTCCGG
CACCGACTTCACCCTGAAGATCTCCAGGGTGGAGGCCGAGGACGTGGGCGTGTAC
TACTGCCAGCAGTCCAAGGAGGACCCCAGGACCTTCGGCCAGGGCACCAAGCTG
GAGATCAAGGGAGGCGGTGGATCAGGCGGAGGTGGCTCAGGTGGAGGCGGTTCT

GAGGTGCAGCTGGTGCAGTCCGGCGCCGAGGTGAAGAAGCCCGGCGCCTCCGTG
AAGGTGTCCTGCAAGGCCTCCGGCTACACCTTCACCGACTACAACATGGACTGGG
TGAGGCAGGCCCCCGGCCAGGGCCTGGAGTGGATCGGCGACATCAACCCCAACA
ACGGCGGCACCCTGTACAACCAGAAGTTCAGGGGCAGGGTGACCCTGACCGTGG
ACACCTCCATCTCCACCGCCTACATGGAGCTGTCCAGGCTGAGGTCCGACGACAC
CGCCGTGTACTACTGCGCCAGGTCCGAGGTGTTCTACGGCAACTACGCCGACTACT
GGGGCCAGGGCACCACCGTGACCGTGTCCTCCGAGCCCAAGTCCTGCGACAAGA
CCCACACCTGCCCCCCCTGCCCCGCCCCCGAGGCCGCCGGCGGCCCCTCCGTGTT
CCTGTTCCCCCCCAAGCCCAAGGACACCCTGATGATCTCCAGGACCCCCGAGGTG
ACCTGCGTGGTGGTGGACGTGTCCCACGAGGACCCCGAGGTGAAGTTCAACTGGT
ACGTGGACGGCGTGGAGGTGCACAACGCCAAGACCAAGCCCAGGGAGGAGCAGT
ACAACTCCACCTACAGGGTGGTGTCCGTGCTGACCGTGCTGCACCAGGACTGGCT
GAACGGCAAGGAGTACAAGTGCAAGGTGTCCAACAAGGCCCTGCCCGCCCCCAT
CGAGAAGACCATCTCCAAGGCCAAGGGCCAGCCCAGGGAGCCCCAGGTGTGCAC
CCTGCCCCCCTCCAGGGACGAGCTGACCAAGAACCAGGTGTCCCTGTCCTGCGCC
GTGAAGGGCTTCTACCCCTCCGACATCGCCGTGGAGTGGGAGTCCAACGGCCAGC
CCGAGAACAACTACAAGACCACCCCCCCCGTGCTGGACTCCGACGGCTCCTTCTT
CCTGGTGTCCAAGCTGACCGTGGACAAGTCCAGGTGGCAGCAGGGCAACGTGTTC
TCCTGCTCCGTGATGCACGAGGCCCTGCACAACCACTACACCCAGAAGTCCCTGT
CCCTGTCCCCCGGCAAGTAA (SEQ ID NO: 79).

Nucleotide sequence encoding B7H6-11 antibody

GACATCGTGCTGACCCAGACCCCCCTGTCCCTGTCCGTGACCCCCGGCC
AGCCCGCCTCCATCTCCTGCAAGGCCTCCCAGTCCGTGGACTACGACGGCGACTC
CTACATGAACTGGTACCTGCAGAAGCCCGGCCAGCCCCCCCAGCTGCTGATCTACG
CCGCCTCCACCCTGCACTCCGGCGTGCCCGACAGGTTCTCCGGCTCCGGCTCCGG
CACCGACTTCACCCTGAAGATCTCCAGGGTGGAGGCCGAGGACGTGGGCGTGTAC
TACTGCCAGCAGTCCAAGGAGGACCCCAGGACCTTCGGCCAGGGCACCAAGCTG
GAGATCAAGGGAGGCGGTGGATCAGGCGGAGGTGGCTCAGGTGGAGGCGGTTCT
GAGGTGCAGCTGGTGCAGTCCGGCGCCGAGGTGAAGAAGCCCGGCGCCTCCGTG
AAGGTGTCCTGCAAGGCCTCCGGCTACACCTTCACCGACTACAACATGGACTGGG

TGAGGCAGGCCCCCGGCCAGGGCCTGGAGTGGATCGGCGACATCAACCCCAACA
ACGGCGGCACCCTGTACAACCAGAAGTTCAGGGGCAGGGTGACCCTGACCGTGG
ACAAGTCCATCTCCACCGCCTACATGGAGCTGTCCAGGCTGAGGTCCGACGACAC
CGCCGTGTACTACTGCGCCAGGTCCGAGGTGTTCTACGGCAACTACGCCGACTACT
GGGGCCAGGGCACCACCGTGACCGTGTCCTCCGAGCCCAAGTCCTGCGACAAGA
CCCACACCTGCCCCCCCTGCCCCGCCCCCGAGGCCGCCGGCGGCCCCTCCGTGTT
CCTGTTCCCCCCCAAGCCCAAGGACACCCTGATGATCTCCAGGACCCCCGAGGTG
ACCTGCGTGGTGGTGGACGTGTCCCACGAGGACCCCGAGGTGAAGTTCAACTGGT
ACGTGGACGGCGTGGAGGTGCACAACGCCAAGACCAAGCCCAGGGAGGAGCAGT
ACAACTCCACCTACAGGGTGGTGTCCGTGCTGACCGTGCTGCACCAGGACTGGCT
GAACGGCAAGGAGTACAAGTGCAAGGTGTCCAACAAGGCCCTGCCCGCCCCCAT
CGAGAAGACCATCTCCAAGGCCAAGGGCCAGCCCAGGGAGCCCCAGGTGTGCAC
CCTGCCCCCCTCCAGGGACGAGCTGACCAAGAACCAGGTGTCCCTGTCCTGCGCC
GTGAAGGGCTTCTACCCCTCCGACATCGCCGTGGAGTGGGAGTCCAACGGCCAGC
CCGAGAACAACTACAAGACCACCCCCCCCGTGCTGGACTCCGACGGCTCCTTCTT
CCTGGTGTCCAAGCTGACCGTGGACAAGTCCAGGTGGCAGCAGGGCAACGTGTTC
TCCTGCTCCGTGATGCACGAGGCCCTGCACAACCACTACACCCAGAAGTCCCTGT
CCCTGTCCCCCGGCAAGTAA (SEQ ID NO: 80).

Nucleotide sequence encoding B7H6-12 antibody

GACATCGTGCTGACCCAGACCCCCCTGTCCCTGTCCGTGACCCCCGGCC
AGCCCGCCTCCATCTCCTGCAAGGCCTCCCAGTCCGTGGACTACGACGGCGACTC
CTACATGAACTGGTACCTGCAGAAGCCCGGCCAGCCCCCCCAGCTGCTGATCTACG
CCGCCTCCACCCTGCACTCCGGCGTGCCCGACAGGTTCTCCGGCTCCGGCTCCGG
CACCGACTTCACCCTGAAGATCTCCAGGGTGGAGGCCGAGGACGTGGGCGTGTAC
TACTGCCAGCAGTCCAAGGAGGACCCCAGGACCTTCGGCCAGGGCACCAAGCTG
GAGATCAAGGGAGGCGGTGGATCAGGCGGAGGTGGCTCAGGTGGAGGCGGTTCT
GAGGTGCAGCTGGTGCAGTCCGGCGCCGAGGTGAAGAAGCCCGGCGCCTCCGTG
AAGGTGTCCTGCAAGGCCTCCGGCTACACCTTCACCGACTACAACATGGACTGGG
TGAGGCAGGCCCCCGGCAAGGGCCTGGAGTGGATCGGCGACATCAACCCCAACA
ACGGCGGCACCCTGTACAACCAGAAGTTCAGGGGCAGGGTGACCCTGACCGTGG

ACAAGTCCATCTCCACCGCCTACATGGAGCTGTCCAGGCTGAGGTCCGACGACAC
CGCCGTGTACTACTGCGCCAGGTCCGAGGTGTTCTACGGCAACTACGCCGACTACT
GGGGCCAGGGCACCACCGTGACCGTGTCCTCCGAGCCCAAGTCCTGCGACAAGA
CCCACACCTGCCCCCCCTGCCCCGCCCCCGAGGCCGCCGGCGGCCCCTCCGTGTT
CCTGTTCCCCCCCAAGCCCAAGGACACCCTGATGATCTCCAGGACCCCCGAGGTG
ACCTGCGTGGTGGTGGACGTGTCCCACGAGGACCCCGAGGTGAAGTTCAACTGGT
ACGTGGACGGCGTGGAGGTGCACAACGCCAAGACCAAGCCCAGGGAGGAGCAGT
ACAACTCCACCTACAGGGTGGTGTCCGTGCTGACCGTGCTGCACCAGGACTGGCT
GAACGGCAAGGAGTACAAGTGCAAGGTGTCCAACAAGGCCCTGCCCGCCCCCAT
CGAGAAGACCATCTCCAAGGCCAAGGGCCAGCCCAGGGAGCCCCAGGTGTGCAC
CCTGCCCCCCTCCAGGGACGAGCTGACCAAGAACCAGGTGTCCCTGTCCTGCGCC
GTGAAGGGCTTCTACCCCTCCGACATCGCCGTGGAGTGGGAGTCCAACGGCCAGC
CCGAGAACAACTACAAGACCACCCCCCCCGTGCTGGACTCCGACGGCTCCTTCTT
CCTGGTGTCCAAGCTGACCGTGGACAAGTCCAGGTGGCAGCAGGGCAACGTGTTC
TCCTGCTCCGTGATGCACGAGGCCCTGCACAACCACTACACCCAGAAGTCCCTGT
CCCTGTCCCCCGGCAAGTAA (SEQ ID NO: 81).

Nucleotide sequence encoding B7H6-13 antibody

GACATCGTGCTGACCCAGACCCCCCTGTCCCTGTCCGTGACCCCCGGCC
AGCCCGCCTCCATCTCCTGCAAGGCCTCCCAGTCCGTGGACTACGACGGCGACTC
CTACATGAACTGGTACCTGCAGAAGCCCGGCCAGCCCCCCCAGCTGCTGATCTACG
CCGCCTCCACCCTGCACTCCGGCATCCCCGACAGGTTCTCCGGCTCCGGCTCCGGC
ACCGACTTCACCCTGAAGATCTCCAGGGTGGAGGCCGAGGACGTGGGCGTGTACT
ACTGCCAGCAGTCCAAGGAGGACCCCAGGACCTTCGGCCAGGGCACCAAGCTGG
AGATCAAGGGAGGCGGTGGATCAGGCGGAGGTGGCTCAGGTGGAGGCGGTTCTG
AGGTGCAGCTGGTGCAGTCCGGCGCCGAGGTGAAGAAGCCCGGCGCCTCCGTGA
AGGTGTCCTGCAAGGCCTCCGGCTACACCTTCACCGACTACAACATGGACTGGGT
GAGGCAGGCCCCCGGCCAGGGCCTGGAGTGGATGGGCGACATCAACCCCAACAA
CGGCGGCACCCTGTACAACCAGAAGTTCAGGGGCAGGGTGACCATGACCGTGGA
CACCTCCATCTCCACCGCCTACATGGAGCTGTCCAGGCTGAGGTCCGACGACACC
GCCGTGTACTACTGCGCCAGGTCCGAGGTGTTCTACGGCAACTACGCCGACTACTG

GGGCCAGGGCACCACCGTGACCGTGTCCTCCGAGCCCAAGTCCTGCGACAAGAC
CCACACCTGCCCCCCCTGCCCCGCCCCCGAGGCCGCCGGCGGCCCCTCCGTGTTC
CTGTTCCCCCCCAAGCCCAAGGACACCCTGATGATCTCCAGGACCCCCGAGGTGA
CCTGCGTGGTGGTGGACGTGTCCCACGAGGACCCCGAGGTGAAGTTCAACTGGTA
CGTGGACGGCGTGGAGGTGCACAACGCCAAGACCAAGCCCAGGGAGGAGCAGTA
CAACTCCACCTACAGGGTGGTGTCCGTGCTGACCGTGCTGCACCAGGACTGGCTG
AACGGCAAGGAGTACAAGTGCAAGGTGTCCAACAAGGCCCTGCCCGCCCCCATC
GAGAAGACCATCTCCAAGGCCAAGGGCCAGCCCAGGGAGCCCCAGGTGTGCACC
CTGCCCCCCTCCAGGGACGAGCTGACCAAGAACCAGGTGTCCCTGTCCTGCGCCG
TGAAGGGCTTCTACCCCTCCGACATCGCCGTGGAGTGGGAGTCCAACGGCCAGCC
CGAGAACAACTACAAGACCACCCCCCCCGTGCTGGACTCCGACGGCTCCTTCTTC
CTGGTGTCCAAGCTGACCGTGGACAAGTCCAGGTGGCAGCAGGGCAACGTGTTCT
CCTGCTCCGTGATGCACGAGGCCCTGCACAACCACTACACCCAGAAGTCCCTGTC
CCTGTCCCCCGGCAAGTAA (SEQ ID NO: 82).

Nucleotide sequence encoding B7H6-14 antibody

GACATCGTGCTGACCCAGACCCCCCTGTCCCTGTCCGTGACCCCCGGCC
AGCCCGCCTCCATCTCCTGCAAGGCCTCCCAGTCCGTGGACTACGACGGCGACTC
CTACATGAACTGGTACCTGCAGAAGCCCGGCCAGCCCCCCCAGCTGCTGATCTACG
CCGCCTCCACCCTGCACTCCGGCATCCCCGACAGGTTCTCCGGCTCCGGCTCCGGC
ACCGACTTCACCCTGAAGATCTCCAGGGTGGAGGCCGAGGACGTGGGCGTGTACT
ACTGCCAGCAGTCCAAGGAGGACCCCAGGACCTTCGGCCAGGGCACCAAGCTGG
AGATCAAGGGAGGCGGTGGATCAGGCGGAGGTGGCTCAGGTGGAGGCGGTTCTG
AGGTGCAGCTGGTGCAGTCCGGCGCCGAGGTGAAGAAGCCCGGCGCCTCCGTGA
AGGTGTCCTGCAAGGCCTCCGGCTACACCTTCACCGACTACAACATGGACTGGGT
GAGGCAGGCCCCCGGCCAGGGCCTGGAGTGGATCGGCGACATCAACCCCAACAA
CGGCGGCACCCTGTACAACCAGAAGTTCAGGGGCAGGGTGACCCTGACCGTGGA
CACCTCCATCTCCACCGCCTACATGGAGCTGTCCAGGCTGAGGTCCGACGACACC
GCCGTGTACTACTGCGCCAGGTCCGAGGTGTTCTACGGCAACTACGCCGACTACTG
GGGCCAGGGCACCACCGTGACCGTGTCCTCCGAGCCCAAGTCCTGCGACAAGAC
CCACACCTGCCCCCCCTGCCCCGCCCCCGAGGCCGCCGGCGGCCCCTCCGTGTTC

CTGTTCCCCCCCAAGCCCAAGGACACCCTGATGATCTCCAGGACCCCCGAGGTGA
CCTGCGTGGTGGTGGACGTGTCCCACGAGGACCCCGAGGTGAAGTTCAACTGGTA
CGTGGACGGCGTGGAGGTGCACAACGCCAAGACCAAGCCCAGGGAGGAGCAGTA
CAACTCCACCTACAGGGTGGTGTCCGTGCTGACCGTGCTGCACCAGGACTGGCTG
AACGGCAAGGAGTACAAGTGCAAGGTGTCCAACAAGGCCCTGCCCGCCCCCATC
GAGAAGACCATCTCCAAGGCCAAGGGCCAGCCCAGGGAGCCCCAGGTGTGCACC
CTGCCCCCCTCCAGGGACGAGCTGACCAAGAACCAGGTGTCCCTGTCCTGCGCCG
TGAAGGGCTTCTACCCCTCCGACATCGCCGTGGAGTGGGAGTCCAACGGCCAGCC
CGAGAACAACTACAAGACCACCCCCCCCGTGCTGGACTCCGACGGCTCCTTCTTC
CTGGTGTCCAAGCTGACCGTGGACAAGTCCAGGTGGCAGCAGGGCAACGTGTTCT
CCTGCTCCGTGATGCACGAGGCCCTGCACAACCACTACACCCAGAAGTCCCTGTC
CCTGTCCCCCGGCAAGTAA (SEQ ID NO: 83).

Nucleotide sequence encoding B7H6-15

GACATCGTGCTGACCCAGACCCCCCTGTCCCTGTCCGTGACCCCCGGCC
AGCCCGCCTCCATCTCCTGCAAGGCCTCCCAGTCCGTGGACTACGACGGCGACTC
CTACATGAACTGGTACCTGCAGAAGCCCGGCCAGCCCCCCCAGCTGCTGATCTACG
CCGCCTCCACCCTGCACTCCGGCATCCCCGACAGGTTCTCCGGCTCCGGCTCCGGC
ACCGACTTCACCCTGAAGATCTCCAGGGTGGAGGCCGAGGACGTGGGCGTGTACT
ACTGCCAGCAGTCCAAGGAGGACCCCAGGACCTTCGGCCAGGGCACCAAGCTGG
AGATCAAGGGAGGCGGTGGATCAGGCGGAGGTGGCTCAGGTGGAGGCGGTTCTG
AGGTGCAGCTGGTGCAGTCCGGCGCCGAGGTGAAGAAGCCCGGCGCCTCCGTGA
AGGTGTCCTGCAAGGCCTCCGGCTACACCTTCACCGACTACAACATGGACTGGGT
GAGGCAGGCCCCCGGCCAGGGCCTGGAGTGGATCGGCGACATCAACCCCAACAA
CGGCGGCACCCTGTACAACCAGAAGTTCAGGGGCAGGGTGACCCTGACCGTGGA
CAAGTCCATCTCCACCGCCTACATGGAGCTGTCCAGGCTGAGGTCCGACGACACC
GCCGTGTACTACTGCGCCAGGTCCGAGGTGTTCTACGGCAACTACGCCGACTACTG
GGGCCAGGGCACCACCGTGACCGTGTCCTCCGAGCCCAAGTCCTGCGACAAGAC
CCACACCTGCCCCCCCTGCCCCGCCCCCGAGGCCGCCGGCGGCCCCTCCGTGTTC
CTGTTCCCCCCCAAGCCCAAGGACACCCTGATGATCTCCAGGACCCCCGAGGTGA
CCTGCGTGGTGGTGGACGTGTCCCACGAGGACCCCGAGGTGAAGTTCAACTGGTA

CGTGGACGGCGTGGAGGTGCACAACGCCAAGACCAAGCCCAGGGAGGAGCAGTA
CAACTCCACCTACAGGGTGGTGTCCGTGCTGACCGTGCTGCACCAGGACTGGCTG
AACGGCAAGGAGTACAAGTGCAAGGTGTCCAACAAGGCCCTGCCCGCCCCCATC
GAGAAGACCATCTCCAAGGCCAAGGGCCAGCCCAGGGAGCCCCAGGTGTGCACC
CTGCCCCCCTCCAGGGACGAGCTGACCAAGAACCAGGTGTCCCTGTCCTGCGCCG
TGAAGGGCTTCTACCCCTCCGACATCGCCGTGGAGTGGGAGTCCAACGGCCAGCC
CGAGAACAACTACAAGACCACCCCCCCCGTGCTGGACTCCGACGGCTCCTTCTTC
CTGGTGTCCAAGCTGACCGTGGACAAGTCCAGGTGGCAGCAGGGCAACGTGTTCT
CCTGCTCCGTGATGCACGAGGCCCTGCACAACCACTACACCCAGAAGTCCCTGTC
CCTGTCCCCCGGCAAGTAA (SEQ ID NO: 84).

Nucleotide sequence encoding B7H6-16 antibody

GACATCGTGCTGACCCAGACCCCCCTGTCCCTGTCCGTGACCCCCGGCC
AGCCCGCCTCCATCTCCTGCAAGGCCTCCCAGTCCGTGGACTACGACGGCGACTC
CTACATGAACTGGTACCTGCAGAAGCCCGGCCAGCCCCCCCAGCTGCTGATCTACG
CCGCCTCCACCCTGCACTCCGGCATCCCCGACAGGTTCTCCGGCTCCGGCTCCGGC
ACCGACTTCACCCTGAAGATCTCCAGGGTGGAGGCCGAGGACGTGGGCGTGTACT
ACTGCCAGCAGTCCAAGGAGGACCCCAGGACCTTCGGCCAGGGCACCAAGCTGG
AGATCAAGGGAGGCGGTGGATCAGGCGGAGGTGGCTCAGGTGGAGGCGGTTCTG
AGGTGCAGCTGGTGCAGTCCGGCGCCGAGGTGAAGAAGCCCGGCGCCTCCGTGA
AGGTGTCCTGCAAGGCCTCCGGCTACACCTTCACCGACTACAACATGGACTGGGT
GAGGCAGGCCCCCGGCAAGGGCCTGGAGTGGATCGGCGACATCAACCCCAACAA
CGGCGGCACCCTGTACAACCAGAAGTTCAGGGGCAGGGTGACCCTGACCGTGGA
CAAGTCCATCTCCACCGCCTACATGGAGCTGTCCAGGCTGAGGTCCGACGACACC
GCCGTGTACTACTGCGCCAGGTCCGAGGTGTTCTACGGCAACTACGCCGACTACTG
GGGCCAGGGCACCACCGTGACCGTGTCCTCCGAGCCCAAGTCCTGCGACAAGAC
CCACACCTGCCCCCCCTGCCCCGCCCCCGAGGCCGCCGGCGGCCCCTCCGTGTTC
CTGTTCCCCCCCAAGCCCAAGGACACCCTGATGATCTCCAGGACCCCCGAGGTGA
CCTGCGTGGTGGTGGACGTGTCCCACGAGGACCCCGAGGTGAAGTTCAACTGGTA
CGTGGACGGCGTGGAGGTGCACAACGCCAAGACCAAGCCCAGGGAGGAGCAGTA
CAACTCCACCTACAGGGTGGTGTCCGTGCTGACCGTGCTGCACCAGGACTGGCTG

AACGGCAAGGAGTACAAGTGCAAGGTGTCCAACAAGGCCCTGCCCGCCCCCATC
GAGAAGACCATCTCCAAGGCCAAGGGCCAGCCCAGGGAGCCCCAGGTGTGCACC
CTGCCCCCCTCCAGGGACGAGCTGACCAAGAACCAGGTGTCCCTGTCCTGCGCCG
TGAAGGGCTTCTACCCCTCCGACATCGCCGTGGAGTGGGAGTCCAACGGCCAGCC
CGAGAACAACTACAAGACCACCCCCCCCGTGCTGGACTCCGACGGCTCCTTCTTC
CTGGTGTCCAAGCTGACCGTGGACAAGTCCAGGTGGCAGCAGGGCAACGTGTTCT
CCTGCTCCGTGATGCACGAGGCCCTGCACAACCACTACACCCAGAAGTCCCTGTC
CCTGTCCCCCGGCAAGTAA (SEQ ID NO: 85).

Nucleotide sequence encoding B7H6-17 antibody

GACATCGTGCTGACCCAGTCCCCCGTGTCCCTGGCCGTGCCCCTGGGCC
AGAGGGCCACCATCTCCTGCAAGGCCTCCCAGTCCGTGGACTACGACGGCGACTC
CTACATGAACTGGTACCAGCAGAAGCCCGGCCAGCCCCCCAAGCTGCTGATCTAC
GCCGCCTCCACCCTGCACTCCGGCATCCCCGTGAGGTTCTCCGGCTCCGGCTCCGG
CACCGACTTCACCCTGAACATCCACCCCGTGGAGGAGGAGGACGCCGCCTCCTAC
TACTGCCAGCAGTCCAAGGAGGACCCCAGGACCTTCGGCGGCGGCACCAAGCTG
GAGATCAAGGGAGGCGGTGGATCAGGCGGAGGTGGCTCAGGTGGAGGCGGTTCT
GAGGTGCTGCTGCAGCAGTCCGGCCCCGAGGTGGTGAAGCCCGGCGCCTCCGTG
AAGATCACCTGCAAGGCCTCCGGCTACACCTTCACCGACTACAACATGGACTGGG
TGAAGCAGTCCCACGGCAAGTCCCTGGAGTGGATCGGCGACATCAACCCCAACAA
CGCCGGCACCCTGTACAACCAGAAGTTCAGGGGCAGGGTGACCCTGATCGTGGAC
AAGTCCTCCTCCACCGCCTACATGGAGCTGAGGTCCCTGACCTCCGACGACACCG
CCGTGTACTACTGCGCCAGGTCCGAGGTGTTCTACGGCAACTACGCCGACTACTGG
GGCCAGGGCACCACCCTGACCGTGTCCTCCGAGCCCAAGTCCTGCGACAAGACCC
ACACCTGCCCCCCCTGCCCCGCCCCCGAGGCCGCCGGCGGCCCCTCCGTGTTCCT
GTTCCCCCCCAAGCCCAAGGACACCCTGATGATCTCCAGGACCCCCGAGGTGACC
TGCGTGGTGGTGGACGTGTCCCACGAGGACCCCGAGGTGAAGTTCAACTGGTACG
TGGACGGCGTGGAGGTGCACAACGCCAAGACCAAGCCCAGGGAGGAGCAGTACA
ACTCCACCTACAGGGTGGTGTCCGTGCTGACCGTGCTGCACCAGGACTGGCTGAA
CGGCAAGGAGTACAAGTGCAAGGTGTCCAACAAGGCCCTGCCCGCCCCCATCGA
GAAGACCATCTCCAAGGCCAAGGGCCAGCCCAGGGAGCCCCAGGTGTGCACCCT

GCCCCCCTCCAGGGACGAGCTGACCAAGAACCAGGTGTCCCTGTCCTGCGCCGTG
AAGGGCTTCTACCCCTCCGACATCGCCGTGGAGTGGGAGTCCAACGGCCAGCCCG
AGAACAACTACAAGACCACCCCCCCCGTGCTGGACTCCGACGGCTCCTTCTTCCT
GGTGTCCAAGCTGACCGTGGACAAGTCCAGGTGGCAGCAGGGCAACGTGTTCTCC
TGCTCCGTGATGCACGAGGCCCTGCACAACCACTACACCCAGAAGTCCCTGTCCC
TGTCCCCCGGCAAGTAA (SEQ ID NO: 86).

Nucleotide sequence encoding B7H6-18 antibody

GACATCGTGCTGACCCAGTCCCCCGTGTCCCTGGCCGTGCCCCTGGGCC
AGAGGGCCACCATCTCCTGCAAGGCCTCCCAGTCCGTGGACTACGACGCCGACTC
CTACATGAACTGGTACCAGCAGAAGCCCGGCCAGCCCCCCAAGCTGCTGATCTAC
GCCGCCTCCACCCTGCACTCCGGCATCCCCGTGAGGTTCTCCGGCTCCGGCTCCGG
CACCGACTTCACCCTGAACATCCACCCCGTGGAGGAGGAGGACGCCGCCTCCTAC
TACTGCCAGCAGTCCAAGGAGGACCCCAGGACCTTCGGCGGCGGCACCAAGCTG
GAGATCAAGGGAGGCGGTGGATCAGGCGGAGGTGGCTCAGGTGGAGGCGGTTCT
GAGGTGCTGCTGCAGCAGTCCGGCCCCGAGGTGGTGAAGCCCGGCGCCTCCGTG
AAGATCACCTGCAAGGCCTCCGGCTACACCTTCACCGACTACAACATGGACTGGG
TGAAGCAGTCCCACGGCAAGTCCCTGGAGTGGATCGGCGACATCAACCCCAACAA
CGGCGGCACCCTGTACAACCAGAAGTTCAGGGGCAGGGTGACCCTGATCGTGGAC
AAGTCCTCCTCCACCGCCTACATGGAGCTGAGGTCCCTGACCTCCGACGACACCG
CCGTGTACTACTGCGCCAGGTCCGAGGTGTTCTACGGCAACTACGCCGACTACTGG
GGCCAGGGCACCACCCTGACCGTGTCCTCCGAGCCCAAGTCCTGCGACAAGACCC
ACACCTGCCCCCCCTGCCCCGCCCCCGAGGCCGCCGGCGGCCCCTCCGTGTTCCT
GTTCCCCCCCAAGCCCAAGGACACCCTGATGATCTCCAGGACCCCCGAGGTGACC
TGCGTGGTGGTGGACGTGTCCCACGAGGACCCCGAGGTGAAGTTCAACTGGTACG
TGGACGGCGTGGAGGTGCACAACGCCAAGACCAAGCCCAGGGAGGAGCAGTACA
ACTCCACCTACAGGGTGGTGTCCGTGCTGACCGTGCTGCACCAGGACTGGCTGAA
CGGCAAGGAGTACAAGTGCAAGGTGTCCAACAAGGCCCTGCCCGCCCCCATCGA
GAAGACCATCTCCAAGGCCAAGGGCCAGCCCAGGGAGCCCCAGGTGTGCACCCT
GCCCCCCTCCAGGGACGAGCTGACCAAGAACCAGGTGTCCCTGTCCTGCGCCGTG
AAGGGCTTCTACCCCTCCGACATCGCCGTGGAGTGGGAGTCCAACGGCCAGCCCG

AGAACAACTACAAGACCACCCCCCCCGTGCTGGACTCCGACGGCTCCTTCTTCCT
GGTGTCCAAGCTGACCGTGGACAAGTCCAGGTGGCAGCAGGGCAACGTGTTCTCC
TGCTCCGTGATGCACGAGGCCCTGCACAACCACTACACCCAGAAGTCCCTGTCCC
TGTCCCCCGGCAAGTAA (SEQ ID NO: 87).

It should be noted that with respect to the nucleic acid referred to in the present specification and claims, those skilled in the art can understand that the nucleic acid includes any one of two complementary strands, or both. For convenience, in this specification and claims, in most cases only one strand is shown, but another strand complementary thereto is disclosed in fact. In addition, the nucleic acid sequences in the present disclosure include the form of DNA or RNA. When one of them is disclosed, the other is also disclosed.

In a fourth aspect of the present disclosure, the present disclosure provides an expression vector carrying the nucleic acid molecule as described above. The expression vector may include an optional control sequence operably linked to the nucleic acid molecule. The control sequence is one or more control sequences that direct expression of the nucleic acid molecule in a host. The expression vector proposed in some specific embodiments of the present disclosure can efficiently express the recombinant antibody in suitable host cells, and thus can be effectively used for specific treatment or prevention of tumors, particularly tumors expressing B7H6.

In a fifth aspect of the present disclosure, the present disclosure provides a recombinant cell carrying the nucleic acid molecule according to the third aspect, the expression vector according to the fourth aspect, or the recombinant antibody according to the first or second aspect. The recombinant cell is obtained by transfecting or transforming the expression vector. According to some specific embodiments of the present disclosure, the recombinant cells can efficiently express the recombinant antibody under suitable conditions, and the recombinant cells can be effectively used for specific treatment or prevention of a tumor, particularly a tumor expressing B7H6.

It should be noted that "suitable conditions" in the specification refers to conditions suitable for the expression of the recombinant antibodies described in the present disclosure. Those skilled in the art will readily appreciate that suitable conditions for the expression of the recombinant antibodies include, but are not limited to, suitable transformation or transfection manners, suitable transformation or transfection conditions, healthy host cell status, suitable host cell density, suitable cell culture environment, and suitable cell culture time. The "suitable conditions" are not particularly limited. Those skilled in the art can optimize the conditions for the expression of the recombinant antibody according to the specific circumstances of the laboratory.

In a sixth aspect of the present disclosure, the present disclosure provides a composition including the recombinant antibody according to the first or second aspect, the nucleic acid molecule according to the third aspect, the expression vector according to the fourth aspect or the recombinant cell according to the fifth aspect. As described above, the recombinant antibody according to the embodiments of the present disclosure can effectively bind to CD3 or B7H6 protein molecules, thereby specifically recognizing tumor cells highly expressing the B7H6. They can promote selective localization of T cells to the tumor site rather than in the peripheral circulation, avoiding systemic activation. The composition, such as a food composition or a pharmaceutical composition, including the recombinant antibody and a series of corresponding substances, also has significant effects for treating or preventing tumors expressing B7H6, with higher safety and fewer side effects.

In an eighth aspect of the present disclosure, the present disclosure provides a medicament including the recombinant antibody according to the first or second aspect, the nucleic acid molecule according to the third aspect, the expression vector according to the fourth aspect, the recombinant cell according to the fifth aspect, or the composition according to the sixth aspect. The medicament is used for treating or preventing cancer. As described above, the recombinant antibody according to the embodiments of the present disclosure can effectively bind to CD3 or B7H6 protein molecules, thereby specifically recognizing tumor cells highly expressing the B7H6. They can also promote selective localization of T cells to the tumor site rather than in the peripheral circulation, avoiding systemic activation. The medicament including the recombinant antibody or a series of corresponding substances also has significant effects for treating or preventing tumors expressing B7H6, with higher safety and fewer side effects.

According to some specific embodiments of the present disclosure, the cancer includes at least one of rectal cancer, non-small cell lung cancer, breast cancer, and liver cancer.

The medicament according to some specific embodiments of the present disclosure includes a pharmaceutically acceptable carrier and an effective amount of the antibody active ingredient.

As used herein, the term "effective amount" or "effective dose" refers to an amount capable of producing function or activity and acceptable for humans and/or animals.

As used herein, the "pharmaceutically acceptable" ingredient is a substance suitable for use in humans and/or mammals without casing undue adverse side effects (such as toxicity, irritation, and allergic response), i.e., a substance having an appropriate benefit/risk ratio. The term "pharmaceutically acceptable carrier" refers to a carrier used in the administration of therapeutic agents, including various excipients and diluents.

The medicament according to the present disclosure contains a safe and effective amount of the active ingredient of the present disclosure and a pharmaceutically acceptable carrier. The carrier includes, but not limited to, saline, buffer, glucose, water, glycerol, ethanol, and combinations thereof. Generally, the pharmaceutical preparation should be adapted to the administration route. A formulation of the medicament according to the present disclosure is injection, oral preparation (tablet, capsule, oral liquid), transdermal agent, or sustained-release agent. For example, the formulation is prepared with the conventional methods using physiological saline or aqueous solutions containing glucose and other adjuvants. The medicament is preferably manufactured under sterile conditions.

The effective amount of active ingredient described herein may vary depending on the administration route and the severity of the disease to be treated. A preferred effective amount can be selected and determined by those skilled in the art based on a variety of factors (e.g., through clinical trials). These factors include, but are not limited to, pharmacokinetic parameters of the active ingredient such as bioavailability, metabolism, half-life, and the like; the severity of the disease to be treated of the patient, the weight of the patient, the immune status of the patient, the administration route, etc. For example, several divided doses may be administered daily, or the dose may be proportionally reduced, based on the exigencies of therapeutic situation.

The pharmaceutically acceptable carrier described herein includes, but not limited to, water, saline, liposomes, lipids, proteins, protein-antibody conjugates, peptides, cellulose, nanogels, or combinations thereof. The selection of carrier should be adapted to the administration route, which is well-known to those skilled in the art.

### Uses

In a seventh aspect of the present disclosure, the present disclosure provides use of the recombinant antibody according to the first or second aspect, the nucleic acid molecule according to the third aspect, the expression vector according to the fourth aspect, the recombinant cell according to the fifth aspect, or the composition according to the sixth aspect in the manufacture of a medicament. The medicament is used for treating or preventing a tumor. As described above, the recombinant antibody provided by some specific embodiments of the present disclosure can effectively bind to CD3 and B7H6 protein, thereby specifically recognizing tumor cells specifically highly expressing the B7H6. Thus, the medicament prepared using the recombinant antibody and a series of corresponding substances also has significant effects for treating or preventing tumors expressing B7H6, with higher safety and fewer side effects.

According to some specific embodiments of the present disclosure, the above-mentioned uses may further include at least one of the following additional technical features.

According to some specific embodiments of the present disclosure, the tumor includes at least one of rectal cancer, non-small cell lung cancer, breast cancer, and liver cancer. Kit

In a ninth aspect of the present disclosure, the present disclosure provides a kit including the recombinant antibody according to the first or second aspect. The recombinant antibody provided by some specific embodiments of the present disclosure can bind to the B7H6 protein. Therefore, the kit including the recombinant antibody can be used for effectively diagnosing or detecting tumors highly expressing the B7H6 protein.

According to some specific embodiments of the present disclosure, the kit is used for diagnosing or detecting at least one of rectal cancer, non-small cell lung cancer, breast cancer, and liver cancer. The antibody provided by some specific embodiments of the present disclosure can effectively bind to CD3 and B7H6 protein molecules, thereby specifically recognizing the tumor cells, which specifically and highly express the B7H6. Therefore, the recombinant antibody can be used in the preparation of a kit for diagnosing or detecting at least one of rectal cancer, non-small cell lung cancer, breast cancer, and liver cancer. The kit can be used for scientific studies such as qualitative or quantitative detection of CD3 and/or B7H6 protein molecules in a biological sample.

### Disease Therapeutic Uses and Methods

In a tenth aspect of the present disclosure, the present disclosure provides use of the above-mentioned recombinant antibody, nucleic acid molecule, expression vector, recombinant cell, composition or medicament in the treatment or prevention of cancer. As described above, the recombinant antibody can specifically recognize the above-mentioned CD3 and/or B7H6 proteins. When the affinity of the CD3 antibody is in an appropriate range and the tumor target-associated antibody has a high affinity, the bispecific antibody can promote the selective localization of T cells to the tumor site rather than in the peripheral circulation, thereby avoiding the systemic activation while effectively treating and preventing cancer. Therefore, cancer can be treated or prevented by the recombinant antibody, composition including the recombinant antibody and medicament according to the embodiments of the present disclosure, as well as a series of substances capable of obtaining the recombinant antibody expressed under the appropriate conditions, for example, the above-mentioned nucleic acid molecule, expression vector, recombinant cell, and the like.

According to some specific embodiments of the present disclosure, the cancer includes at least one of rectal cancer, non-small cell lung cancer, breast cancer, and liver cancer.

In an eleventh aspect of the present disclosure, the present disclosure provides a method for treating or preventing cancer. According to some specific embodiments of the present disclosure, the method includes: administering to the subject at least one of: 1) the recombinant antibody as described above; 2) the nucleic acid molecule as described above; 3) the expression vector as described above; 4) the recombinant cell as described above; or 5) the composition as described above. As described above, the recombinant antibody can specifically recognize the above-mentioned CD3 and/or B7H6 protein. When the affinity of the CD3 antibody is in an appropriate range and the tumor target-associated antibody has a high affinity, the bispecific antibody can promote the selective localization of T cells to the tumor site rather than in the peripheral circulation, thereby avoiding the systemic activation while effectively treating and preventing cancer. Therefore, the method according to embodiments of the present disclosure can effectively treat or prevent cancer and avoid systemic T cell activation.

According to some specific embodiments of the present disclosure, the cancer includes at least one of rectal cancer, non-small cell lung cancer, breast cancer, and liver cancer.

Hereinafter, examples will be described in detail. Where specific techniques or conditions are not specified in the examples, they are performed according to techniques or conditions described in the literature in the related art or according to the product description. The used reagents or instruments without indicating the manufacturer are conventional and commercially available products.

### Example 1: Design and construction of CD3xB7H6 bispecific antibody molecule

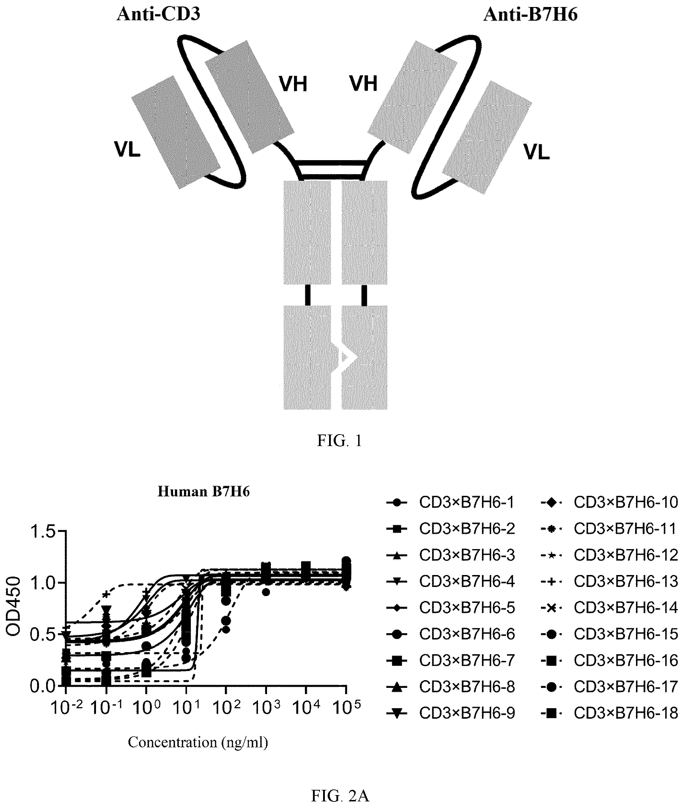

In this example, by performing screening on various configurations, the Applicant identified a bispecific antibody in the scFv-Fc configuration, which was designated CD3xB7H6. The antibody includes two monovalent units, one of which is an anti-CD3 ScFv-Fc form, and the other one of which an anti-B7H6 ScFv-Fc form. The light chain variable region and the heavy chain variable region of the CD3 antibody were connected by one linker peptide, and the same linker peptide was also used to connect the light chain variable region and the heavy chain variable region of the B7H6 antibody. The linker peptide has an amino acid sequence as set forth in SEQ ID NO: 46. The CD3 antibody includes the light chain variable region as set forth in SEQ ID NO: 13, and the heavy chain variable region as set forth in SEQ ID NO: 14. The B7H6 antibody includes the light chain variable region as set forth in SEQ ID NO: 15 to SEQ ID NO: 20, and the heavy chain variable region as set forth in SEQ ID NO: 21 to SEQ ID NO: 26. The Applicant performed screening on different combinations of the variable regions of the B7H6 antibody and the variable regions of the CD3 antibody, and constructed a total of 18 CD3xB7H6 recombinant antibody molecules. In addition, the Applicant performed amino acid mutation on the Fc region of the monovalent unit. The amino acids of the Fc region of the anti-CD3 were changed to cysteine (S384C) at position 384 and tryptophan (T396W) at position 396, and the amino acids of the Fc region of the anti-B7H6 were changed to cysteine (Y380C) at position 380, serine (T397S) at position 397, alanine (L399A) at position 399 and valine (Y408V) at position 408, such that homodimer thereof was unlikely to be formed and heterodimer thereof can be easily formed. The heterodimer is a bispecific antibody CD3xB7H6, and the specific structure is illustrated in FIG. 1. The 18 CD3xB7H6 recombinant antibody molecules have a CD3 single-chain antibody as set forth in SEQ ID NO: 27, a B7H6 single-chain antibody as set forth in SEQ ID NO: 28 to SEQ ID NO: 45, a primary antibody Fc region as set forth in SEQ ID NO: 47, and a secondary antibody Fc region as set forth in SEQ ID NO: 48, which are subjected to the above-mentioned mutations and linked by in a knob-into-hole structure.

Nucleic acid sequences encoding the polypeptide chains of the CD3 and B7H6 (1-18) monovalent units were cloned into the expression vector pTT5 (YouBio, cat. No. VT2202) by means of common molecular biology techniques, respectively. The specific nucleotide sequence is at least one of the nucleotide sequences as set forth in SEQ ID NO: 69, and SEQ ID NO: 70 to SEQ ID NO: 87. In the meantime, in order to express the expression vector and secrete it into the culture medium, a leader peptide of a kappa chain of murine antibody was selected and inserted into the expression vector as secretion signal peptide, which was located at the N-end of the variable region of the antibody. The amino acid sequence of the signal peptide was: METDTLLLWVLLLWVPGSTG (SEQ ID NO: 68), and the nucleotide sequence encoding the signal peptide is shown in SEQ ID NO: 88. 
ATGGAGACCGACACCCTGCTGCTGTGGGTGCTGCTGCTGTGGGTGCCCG
GCTCCACCGGC (SEQ ID NO: 88).
CD3 single-chain antibody 
MDIQMTQSPSSLSASVGDRVTITCRASQDIRNYLNWYQQKPGKAPKLLIYYTSRLESG
VPSRFSGSGSGTDYTLTISSLQPEDFATYYCQQGNTLPWTFGQGTKVEIKGGGGSGGG
GSGGGGSEVQLVESGGGLVQPGGSLRLSCAASGYSFTGYTMNWVRQAPGKGLEWV
ALINPYKGVSTYNQKFKDRFTISVDKSKNTAYLQMNSLRAEDTAVYYCARSGYYGDS
DWYFDVWGQGTLVTVSS (SEQ ID NO: 27).
B7H6-1 single-chain antibody 
DIVLTQSPDSLAVSLGERATINCKASQSVDYDGDSYMNWYQQKPGQPPKLLIYAASTL
HSGVPDRFSGSGSGTDFTLTISSLQAEDVAVYYCQQSKEDPRTFGQGTKLEIKGGGGS
GGGGSGGGGSEVQLVQSGAEVKKPGASVKVSCKASGYTFTDYNMDWVRQAPGQGL
EWMGDINPNNGGTLYNQKFRGRVTMTVDTSISTAYMELSRLRSDDTAVYYCARSEVF
YGNYADYWGQGTTVTVSS (SEQ ID NO: 28).
B7H6-2 single-chain antibody 
DIVLTQSPDSLAVSLGERATINCKASQSVDYDGDSYMNWYQQKPGQPPKLLIYAASTL
HSGVPDRFSGSGSGTDFTLTISSLQAEDVAVYYCQQSKEDPRTFGQGTKLEIKGGGGS
GGGGSGGGGSEVQLVQSGAEVKKPGASVKVSCKASGYTFTDYNMDWVRQAPGQGL
EWIGDINPNNGGTLYNQKFRGRVTLTVDTSISTAYMELSRLRSDDTAVYYCARSEVFY
GNYADYWGQGTTVTVSS (SEQ ID NO: 29).
B7H6-3 single-chain antibody 
DIVLTQSPDSLAVSLGERATINCKASQSVDYDGDSYMNWYQQKPGQPPKLLIYAASTL
HSGVPDRFSGSGSGTDFTLTISSLQAEDVAVYYCQQSKEDPRTFGQGTKLEIKGGGGS
GGGGSGGGGSEVQLVQSGAEVKKPGASVKVSCKASGYTFTDYNMDWVRQAPGQGL
EWIGDINPNNGGTLYNQKFRGRVTLTVDKSISTAYMELSRLRSDDTAVYYCARSEVFY
GNYADYWGQGTTVTVSS (SEQ ID NO: 30).
B7H6-4 single-chain antibody 
DIVLTQSPDSLAVSLGERATINCKASQSVDYDGDSYMNWYQQKPGQPPKLLIYAASTL
HSGVPDRFSGSGSGTDFTLTISSLQAEDVAVYYCQQSKEDPRTFGQGTKLEIKGGGGS
GGGGSGGGGSEVQLVQSGAEVKKPGASVKVSCKASGYTFTDYNMDWVRQAPGKGL
EWIGDINPNNGGTLYNQKFRGRVTLTVDKSISTAYMELSRLRSDDTAVYYCARSEVFY
GNYADYWGQGTTVTVSS (SEQ ID NO: 31).
B7H6-5 single-chain antibody 
DIVLTQSPDSLAVSLGERATINCKASQSVDYDGDSYMNWYQQKPGQPPKLLIYAASTL
HSGIPDRFSGSGSGTDFTLTISSLQAEDVAVYYCQQSKEDPRTFGQGTKLEIKGGGGSG
GGGSGGGGSEVQLVQSGAEVKKPGASVKVSCKASGYTFTDYNMDWVRQAPGQGLE
WMGDINPNNGGTLYNQKFRGRVTMTVDTSISTAYMELSRLRSDDTAVYYCARSEVFY
GNYADYWGQGTTVTVSS (SEQ ID NO: 32).
B7H6-6 single-chain antibody 
DIVLTQSPDSLAVSLGERATINCKASQSVDYDGDSYMNWYQQKPGQPPKLLIYAASTL
HSGIPDRFSGSGSGTDFTLTISSLQAEDVAVYYCQQSKEDPRTFGQGTKLEIKGGGGSG
GGGSGGGGSEVQLVQSGAEVKKPGASVKVSCKASGYTFTDYNMDWVRQAPGQGLE
WIGDINPNNGGTLYNQKFRGRVTLTVDTSISTAYMELSRLRSDDTAVYYCARSEVFYG
NYADYWGQGTTVTVSS (SEQ ID NO: 33).
B7H6-7 single-chain antibody 
DIVLTQSPDSLAVSLGERATINCKASQSVDYDGDSYMNWYQQKPGQPPKLLIYAASTL
HSGIPDRFSGSGSGTDFTLTISSLQAEDVAVYYCQQSKEDPRTFGQGTKLEIKGGGGSG
GGGSGGGGSEVQLVQSGAEVKKPGASVKVSCKASGYTFTDYNMDWVRQAPGQGLE
WIGDINPNNGGTLYNQKFRGRVTLTVDKSISTAYMELSRLRSDDTAVYYCARSEVFYG
NYADYWGQGTTVTVSS (SEQ ID NO: 34).
B7H6-8 single-chain antibody 
DIVLTQSPDSLAVSLGERATINCKASQSVDYDGDSYMNWYQQKPGQPPKLLIYAASTL
HSGIPDRFSGSGSGTDFTLTISSLQAEDVAVYYCQQSKEDPRTFGQGTKLEIKGGGGSG
GGGSGGGGSEVQLVQSGAEVKKPGASVKVSCKASGYTFTDYNMDWVRQAPGKGLE
WIGDINPNNGGTLYNQKFRGRVTLTVDKSISTAYMELSRLRSDDTAVYYCARSEVFYG
NYADYWGQGTTVTVSS (SEQ ID NO: 35).
B7H6-9 single-chain antibody 
DIVLTQTPLSLSVTPGQPASISCKASQSVDYDGDSYMNWYLQKPGQPPQLLIYAASTL
HSGVPDRFSGSGSGTDFTLKISRVEAEDVGVYYCQQSKEDPRTFGQGTKLEIKGGGGS
GGGGSGGGGSEVQLVQSGAEVKKPGASVKVSCKASGYTFTDYNMDWVRQAPGQGL
EWMGDINPNNGGTLYNQKFRGRVTMTVDTSISTAYMELSRLRSDDTAVYYCARSEVF
YGNYADYWGQGTTVTVSS (SEQ ID NO: 36).
B7H6-10 single-chain antibody 
DIVLTQTPLSLSVTPGQPASISCKASQSVDYDGDSYMNWYLQKPGQPPQLLIYAASTL
HSGVPDRFSGSGSGTDFTLKISRVEAEDVGVYYCQQSKEDPRTFGQGTKLEIKGGGGS
GGGGSGGGGSEVQLVQSGAEVKKPGASVKVSCKASGYTFTDYNMDWVRQAPGQGL
EWIGDINPNNGGTLYNQKFRGRVTLTVDTSISTAYMELSRLRSDDTAVYYCARSEVFY
GNYADYWGQGTTVTVSS (SEQ ID NO: 37).
B7H6-11 single-chain antibody 
DIVLTQTPLSLSVTPGQPASISCKASQSVDYDGDSYMNWYLQKPGQPPQLLIYAASTL
HSGVPDRFSGSGSGTDFTLKISRVEAEDVGVYYCQQSKEDPRTFGQGTKLEIKGGGGS
GGGGSGGGGSEVQLVQSGAEVKKPGASVKVSCKASGYTFTDYNMDWVRQAPGQGL
EWIGDINPNNGGTLYNQKFRGRVTLTVDKSISTAYMELSRLRSDDTAVYYCARSEVFY
GNYADYWGQGTTVTVSS (SEQ ID NO: 38).
B7H6-12 single-chain antibody 
DIVLTQTPLSLSVTPGQPASISCKASQSVDYDGDSYMNWYLQKPGQPPQLLIYAASTL
HSGVPDRFSGSGSGTDFTLKISRVEAEDVGVYYCQQSKEDPRTFGQGTKLEIKGGGGS
GGGGSGGGGSEVQLVQSGAEVKKPGASVKVSCKASGYTFTDYNMDWVRQAPGKGL
EWIGDINPNNGGTLYNQKFRGRVTLTVDKSISTAYMELSRLRSDDTAVYYCARSEVFY
GNYADYWGQGTTVTVSS (SEQ ID NO: 39).
B7H-13 single-chain antibody 
DIVLTQTPLSLSVTPGQPASISCKASQSVDYDGDSYMNWYLQKPGQPPQLLIYAASTL
HSGIPDRFSGSGSGTDFTLKISRVEAEDVGVYYCQQSKEDPRTFGQGTKLEIKGGGGS
GGGGSGGGGSEVQLVQSGAEVKKPGASVKVSCKASGYTFTDYNMDWVRQAPGQGL
EWMGDINPNNGGTLYNQKFRGRVTMTVDTSISTAYMELSRLRSDDTAVYYCARSEVF
YGNYADYWGQGTTVTVSS (SEQ ID NO: 40).
B7H6-14 single-chain antibody 
DIVLTQTPLSLSVTPGQPASISCKASQSVDYDGDSYMNWYLQKPGQPPQLLIYAASTL
HSGIPDRFSGSGSGTDFTLKISRVEAEDVGVYYCQQSKEDPRTFGQGTKLEIKGGGGS
GGGGSGGGGSEVQLVQSGAEVKKPGASVKVSCKASGYTFTDYNMDWVRQAPGQGL
EWIGDINPNNGGTLYNQKFRGRVTLTVDTSISTAYMELSRLRSDDTAVYYCARSEVFY
GNYADYWGQGTTVTVSS (SEQ ID NO: 41).
B7H6-15 single-chain antibody 
DIVLTQTPLSLSVTPGQPASISCKASQSVDYDGDSYMNWYLQKPGQPPQLLIYAASTL
HSGIPDRFSGSGSGTDFTLKISRVEAEDVGVYYCQQSKEDPRTFGQGTKLEIKGGGGS
GGGGSGGGGSEVQLVQSGAEVKKPGASVKVSCKASGYTFTDYNMDWVRQAPGQGL
EWIGDINPNNGGTLYNQKFRGRVTLTVDKSISTAYMELSRLRSDDTAVYYCARSEVFY
GNYADYWGQGTTVTVSS (SEQ ID NO: 42).
B7H6-16 single-chain antibody 
DIVLTQTPLSLSVTPGQPASISCKASQSVDYDGDSYMNWYLQKPGQPPQLLIYAASTL
HSGIPDRFSGSGSGTDFTLKISRVEAEDVGVYYCQQSKEDPRTFGQGTKLEIKGGGGS
GGGGSGGGGSEVQLVQSGAEVKKPGASVKVSCKASGYTFTDYNMDWVRQAPGKGL
EWIGDINPNNGGTLYNQKFRGRVTLTVDKSISTAYMELSRLRSDDTAVYYCARSEVFY
GNYADYWGQGTTVTVSS (SEQ ID NO: 43).
B7H6-17 single-chain antibody 
DIVLTQSPVSLAVPLGQRATISCKASQSVDYDGDSYMNWYQQKPGQPPKLLIYAASTL
HSGIPVRFSGSGSGTDFTLNIHPVEEEDAASYYCQQSKEDPRTFGGGTKLEIKGGGGS
GGGGSGGGGSEVLLQQSGPEVVKPGASVKITCKASGYTFTDYNMDWVKQSHGKSLE
WIGDINPNNAGTLYNQKFRGRVTLIVDKSSSTAYMELRSLTSDDTAVYYCARSEVFYG
NYADYWGQGTTLTVSS (SEQ ID NO: 44).
B7H6-18 single-chain antibody 
DIVLTQSPVSLAVPLGQRATISCKASQSVDYDADSYMNWYQQKPGQPPKLLIYAASTL
HSGIPVRFSGSGSGTDFTLNIHPVEEEDAASYYCQQSKEDPRTFGGGTKLEIKGGGGS
GGGGSGGGGSEVLLQQSGPEVVKPGASVKITCKASGYTFTDYNMDWVKQSHGKSLE
WIGDINPNNGGTLYNQKFRGRVTLIVDKSSSTAYMELRSLTSDDTAVYYCARSEVFYG
NYADYWGQGTTLTVSS (SEQ ID NO: 45).
Amino acid sequence of first Fc region 
EPKSCDKTHTCPPCPAPEAAGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKAL
PAPIEKTISKAKGQPREPQVYTLPPCRDELTKNQVSLWCLVKGFYPSDIAVEWESNGQP
ENNYKTTPPVLDSDGSFFLYSKLTVDKSRWQQGNVFSCSVMHEALHNHYTQKSLSLS
PGK (SEQ ID NO: 47).
Amino acid sequence of second Fc region

EPKSCDKTHTCPPCPAPEAAGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKAL
PAPIEKTISKAKGQPREPQVCTLPPSRDELTKNQVSLSCAVKGFYPSDIAVEWESNGQP
ENNYKTTPPVLDSDGSFFLVSKLTVDKSRWQQGNVFSCSVMHEALHNHYTQKSLSLS
PGK (SEQ ID NO: 48).

### Example 2: Expression and purification of CD3xB7H6 bispecific antibodies

### 2.1 Expression of CD3xB7H6 bispecific antibody molecules

Bispecific molecules binding to CD3 and B7H6 were prepared by transient transfection of ExpiCHO-S cells (Gibco, cat. No. A29127) with pTT5 vectors carrying the CD3xB7H6 chain encoding genes. The eighteen CD3xB7H6 chain encoding genes had anti-CD3 ScFv-Fc as set forth in SEQ ID NO: 49, and anti-B7H6 ScFv-Fc as set forth in SEQ ID NO: 50 to SEQ ID NO: 67, respectively. Specific experimental procedures are described below. One day before the transfection, a cell density of the ExpiCHO-S cells was adjusted to (3-4)x10⁶/mL and incubated overnight under the conditions of 37°C and 8% CO₂, with shaking at 120 rpm. On the day of the transfection, when the cells were grown to a cell density of 7x10⁶ to 1x 10⁷/mL and had a viability greater than 95%, the transfection began. The cells were diluted to a cell density of 6x10⁶/mL with freshly pre-warmed ExpiCHO medium (Gibco, cat. No. A2910002). Plasmids containing CD3 polypeptide chains and B7H6 polypeptide chains were transfected into the ExpiCHO-S cells at a mass ratio of 1:1 using ExpiFectamine CHO transfection reagent (Gibco, cat. No. A29129) and incubated under the conditions of 37°C and 8% CO₂,with shaking at 120 rpm. 18 to 22 hours after the transfection, ExpiFectamine CHO Enhancer and ExpiCHO Feed were mixed and immediately added into the transfected cells, mixed well, and the mixture was incubated at 32°C, 5% CO₂, with shaking at 120 rpm. Five days after the transfection, the cells were supplemented with 8 mL of ExpiCHO Feed, mixed well, and incubated. The changes in cell number and cell viability were observed every day. After the cell viability has decreased to less than 80% or the cells had been cultured for 10-14 days, the cells were harvested through centrifugation, and the supernatant was purified or frozen at -80°C for later use.

### 2.2 Purification of CD3xB7H6 bispecific antibody molecules

2.1 The obtained supernatant was filtered with a 0.22 µm filter membrane, and the Fc domain-containing antibodies were captured from the expression supernatant using a Mabselect prism A affinity chromatography column (GE, cat. No. 17549854). After the chromatography column was equilibrated with phosphate buffer (pH 7.2), the supernatant was passed through the affinity chromatography column, eluted with elution buffer (100 mM citric acid, pH 2.7), and finally concentrated and replaced with PBS buffer. The purified antibodies had a purity above 95%, as identified by means of SDS-PAGE.
CD3 ScFv-Fc amino acid sequence 
MDIQMTQSPSSLSASVGDRVTITCRASQDIRNYLNWYQQKPGKAPKLLIYYTSRLESG
VPSRFSGSGSGTDYTLTISSLQPEDFATYYCQQGNTLPWTFGQGTKVEIKGGGGSGGG
GSGGGGSEVQLVESGGGLVQPGGSLRLSCAASGYSFTGYTMNWVRQAPGKGLEWV
ALINPYKGVSTYNQKFKDRFTISVDKSKNTAYLQMNSLRAEDTAVYYCARSGYYGDS
DWYFDVWGQGTLVTVSSEPKSCDKTHTCPPCPAPEAAGGPSVFLFPPKPKDTLMISRT
 
PEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPCRDELTKNQVSLWCL
VKGFYPSDIAVEWESNGQPENNYKTTPPVLDSDGSFFLYSKLTVDKSRWQQGNVFSC
SVMHEALHNHYTQKSLSLSPGK (SEQ ID NO: 49).
B7H6-1 ScFv-Fc amino acid sequence 
DIVLTQSPDSLAVSLGERATINCKASQSVDYDGDSYMNWYQQKPGQPPKLLIYAASTL
HSGVPDRFSGSGSGTDFTLTISSLQAEDVAVYYCQQSKEDPRTFGQGTKLEIKGGGGS
GGGGSGGGGSEVQLVQSGAEVKKPGASVKVSCKASGYTFTDYNMDWVRQAPGQGL
EWMGDINPNNGGTLYNQKFRGRVTMTVDTSISTAYMELSRLRSDDTAVYYCARSEVF
YGNYADYWGQGTTVTVSSEPKSCDKTHTCPPCPAPEAAGGPSVFLFPPKPKDTLMISR
TPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVCTLPPSRDELTKNQVSLSCAV
KGFYPSDIAVEWESNGQPENNYKTTPPVLDSDGSFFLVSKLTVDKSRWQQGNVFSCS
VMHEALHNHYTQKSLSLSPGK (SEQ ID NO: 50).
B7H6-2 ScFv-Fc amino acid sequence 
DIVLTQSPDSLAVSLGERATINCKASQSVDYDGDSYMNWYQQKPGQPPKLLIYAASTL
HSGVPDRFSGSGSGTDFTLTISSLQAEDVAVYYCQQSKEDPRTFGQGTKLEIKGGGGS
GGGGSGGGGSEVQLVQSGAEVKKPGASVKVSCKASGYTFTDYNMDWVRQAPGQGL
EWIGDINPNNGGTLYNQKFRGRVTLTVDTSISTAYMELSRLRSDDTAVYYCARSEVFY
GNYADYWGQGTTVTVSSEPKSCDKTHTCPPCPAPEAAGGPSVFLFPPKPKDTLMISRT
PEVTCVVVDVSHEDPEVKFNWYVDGVEVHNAKTKPREEQYNSTYRVVSVLTVLHQ
DWLNGKEYKCKVSNKALPAPIEKTISKAKGQPREPQVCTLPPSRDELTKNQVSLSCAV
KGFYPSDIAVEWESNGQPENNYKTTPPVLDSDGSFFLVSKLTVDKSRWQQGNVFSCS
VMHEALHNHYTQKSLSLSPGK (SEQ ID NO: 51).
B7H6-3 ScFv-Fc amino acid sequence 
DIVLTQSPDSLAVSLGERATINCKASQSVDYDGDSYMNWYQQKPGQPPKLLIYAASTL
HSGVPDRFSGSGSGTDFTLTISSLQAEDVAVYYCQQSKEDPRTFGQGTKLEIKGGGGS
GGGGSGGGGSEVQLVQSGAEVKKPGASVKVSCKASGYTFTDYNMDWVRQAPGQGL
EWIGDINPNNGGTLYNQKFRGRVTLTVDKSISTAYMELSRLRSDDTAVYYCARSEVFY
GNYADYWGQGTTVTVSSEPKSCDKTHTCPPCPAPEAAGGPSVFLFPPKPKDTLMISRT
 
PEVTCVVVDVSHEDPEVKFNWYVDGVEVHNAKTKPREEQYNSTYRVVSVLTVLHQ
DWLNGKEYKCKVSNKALPAPIEKTISKAKGQPREPQVCTLPPSRDELTKNQVSLSCAV
KGFYPSDIAVEWESNGQPENNYKTTPPVLDSDGSFFLVSKLTVDKSRWQQGNVFSCS
VMHEALHNHYTQKSLSLSPGK (SEQ ID NO: 52).
B7H6-4 ScFv-Fc amino acid sequence 
DIVLTQSPDSLAVSLGERATINCKASQSVDYDGDSYMNWYQQKPGQPPKLLIYAASTL
HSGVPDRFSGSGSGTDFTLTISSLQAEDVAVYYCQQSKEDPRTFGQGTKLEIKGGGGS
GGGGSGGGGSEVQLVQSGAEVKKPGASVKVSCKASGYTFTDYNMDWVRQAPGKGL
EWIGDINPNNGGTLYNQKFRGRVTLTVDKSISTAYMELSRLRSDDTAVYYCARSEVFY
GNYADYWGQGTTVTVSSEPKSCDKTHTCPPCPAPEAAGGPSVFLFPPKPKDTLMISRT
PEVTCVVVDVSHEDPEVKFNWYVDGVEVHNAKTKPREEQYNSTYRVVSVLTVLHQ
DWLNGKEYKCKVSNKALPAPIEKTISKAKGQPREPQVCTLPPSRDELTKNQVSLSCAV
KGFYPSDIAVEWESNGQPENNYKTTPPVLDSDGSFFLVSKLTVDKSRWQQGNVFSCS
VMHEALHNHYTQKSLSLSPGK (SEQ ID NO: 53).
B7H6-5 ScFv-Fc amino acid sequence 
DIVLTQSPDSLAVSLGERATINCKASQSVDYDGDSYMNWYQQKPGQPPKLLIYAASTL
HSGIPDRFSGSGSGTDFTLTISSLQAEDVAVYYCQQSKEDPRTFGQGTKLEIKGGGGSG
GGGSGGGGSEVQLVQSGAEVKKPGASVKVSCKASGYTFTDYNMDWVRQAPGQGLE
WMGDINPNNGGTLYNQKFRGRVTMTVDTSISTAYMELSRLRSDDTAVYYCARSEVFY
GNYADYWGQGTTVTVSSEPKSCDKTHTCPPCPAPEAAGGPSVFLFPPKPKDTLMISRT
PEVTCVVVDVSHEDPEVKFNWYVDGVEVHNAKTKPREEQYNSTYRVVSVLTVLHQ
DWLNGKEYKCKVSNKALPAPIEKTISKAKGQPREPQVCTLPPSRDELTKNQVSLSCAV
KGFYPSDIAVEWESNGQPENNYKTTPPVLDSDGSFFLVSKLTVDKSRWQQGNVFSCS
VMHEALHNHYTQKSLSLSPGK (SEQ ID NO: 54).
B7H6-6 ScFv-Fc amino acid sequence 
DIVLTQSPDSLAVSLGERATINCKASQSVDYDGDSYMNWYQQKPGQPPKLLIYAASTL
HSGIPDRFSGSGSGTDFTLTISSLQAEDVAVYYCQQSKEDPRTFGQGTKLEIKGGGGSG
GGGSGGGGSEVQLVQSGAEVKKPGASVKVSCKASGYTFTDYNMDWVRQAPGQGLE
WIGDINPNNGGTLYNQKFRGRVTLTVDTSISTAYMELSRLRSDDTAVYYCARSEVFYG
NYADYWGQGTTVTVSSEPKSCDKTHTCPPCPAPEAAGGPSVFLFPPKPKDTLMISRTP
 
EVTCVVVDVSHEDPEVKFNWYVDGVEVHNAKTKPREEQYNSTYRVVSVLTVLHQD
WLNGKEYKCKVSNKALPAPIEKTISKAKGQPREPQVCTLPPSRDELTKNQVSLSCAVK
GFYPSDIAVEWESNGQPENNYKTTPPVLDSDGSFFLVSKLTVDKSRWQQGNVFSCSV
MHEALHNHYTQKSLSLSPGK (SEQ ID NO: 55).
B7H6-7 ScFv-Fc amino acid sequence 
DIVLTQSPDSLAVSLGERATINCKASQSVDYDGDSYMNWYQQKPGQPPKLLIYAASTL
HSGIPDRFSGSGSGTDFTLTISSLQAEDVAVYYCQQSKEDPRTFGQGTKLEIKGGGGSG
GGGSGGGGSEVQLVQSGAEVKKPGASVKVSCKASGYTFTDYNMDWVRQAPGQGLE
WIGDINPNNGGTLYNQKFRGRVTLTVDKSISTAYMELSRLRSDDTAVYYCARSEVFYG
NYADYWGQGTTVTVSSEPKSCDKTHTCPPCPAPEAAGGPSVFLFPPKPKDTLMISRTP
EVTCVVVDVSHEDPEVKFNWYVDGVEVHNAKTKPREEQYNSTYRVVSVLTVLHQD
WLNGKEYKCKVSNKALPAPIEKTISKAKGQPREPQVCTLPPSRDELTKNQVSLSCAVK
GFYPSDIAVEWESNGQPENNYKTTPPVLDSDGSFFLVSKLTVDKSRWQQGNVFSCSV
MHEALHNHYTQKSLSLSPGK (SEQ ID NO: 56).
B7H6-8 ScFv-Fc amino acid sequence 
DIVLTQSPDSLAVSLGERATINCKASQSVDYDGDSYMNWYQQKPGQPPKLLIYAASTL
HSGIPDRFSGSGSGTDFTLTISSLQAEDVAVYYCQQSKEDPRTFGQGTKLEIKGGGGSG
GGGSGGGGSEVQLVQSGAEVKKPGASVKVSCKASGYTFTDYNMDWVRQAPGKGLE
WIGDINPNNGGTLYNQKFRGRVTLTVDKSISTAYMELSRLRSDDTAVYYCARSEVFYG
NYADYWGQGTTVTVSSEPKSCDKTHTCPPCPAPEAAGGPSVFLFPPKPKDTLMISRTP
EVTCVVVDVSHEDPEVKFNWYVDGVEVHNAKTKPREEQYNSTYRVVSVLTVLHQD
WLNGKEYKCKVSNKALPAPIEKTISKAKGQPREPQVCTLPPSRDELTKNQVSLSCAVK
GFYPSDIAVEWESNGQPENNYKTTPPVLDSDGSFFLVSKLTVDKSRWQQGNVFSCSV
MHEALHNHYTQKSLSLSPGK (SEQ ID NO: 57).
B7H6-9 ScFv-Fc amino acid sequence 
DIVLTQTPLSLSVTPGQPASISCKASQSVDYDGDSYMNWYLQKPGQPPQLLIYAASTL
HSGVPDRFSGSGSGTDFTLKISRVEAEDVGVYYCQQSKEDPRTFGQGTKLEIKGGGGS
GGGGSGGGGSEVQLVQSGAEVKKPGASVKVSCKASGYTFTDYNMDWVRQAPGQGL
EWMGDINPNNGGTLYNQKFRGRVTMTVDTSISTAYMELSRLRSDDTAVYYCARSEVF
YGNYADYWGQGTTVTVSSEPKSCDKTHTCPPCPAPEAAGGPSVFLFPPKPKDTLMISR
 
TPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVCTLPPSRDELTKNQVSLSCAV
KGFYPSDIAVEWESNGQPENNYKTTPPVLDSDGSFFLVSKLTVDKSRWQQGNVFSCS
VMHEALHNHYTQKSLSLSPGK (SEQ ID NO: 58).
B7H6-10 ScFv-Fc amino acid sequence 
DIVLTQTPLSLSVTPGQPASISCKASQSVDYDGDSYMNWYLQKPGQPPQLLIYAASTL
HSGVPDRFSGSGSGTDFTLKISRVEAEDVGVYYCQQSKEDPRTFGQGTKLEIKGGGGS
GGGGSGGGGSEVQLVQSGAEVKKPGASVKVSCKASGYTFTDYNMDWVRQAPGQGL
EWIGDINPNNGGTLYNQKFRGRVTLTVDTSISTAYMELSRLRSDDTAVYYCARSEVFY
GNYADYWGQGTTVTVSSEPKSCDKTHTCPPCPAPEAAGGPSVFLFPPKPKDTLMISRT
PEVTCVVVDVSHEDPEVKFNWYVDGVEVHNAKTKPREEQYNSTYRVVSVLTVLHQ
DWLNGKEYKCKVSNKALPAPIEKTISKAKGQPREPQVCTLPPSRDELTKNQVSLSCAV
KGFYPSDIAVEWESNGQPENNYKTTPPVLDSDGSFFLVSKLTVDKSRWQQGNVFSCS
VMHEALHNHYTQKSLSLSPGK (SEQ ID NO: 59).
B7H6-11 ScFv-Fc amino acid sequence 
DIVLTQTPLSLSVTPGQPASISCKASQSVDYDGDSYMNWYLQKPGQPPQLLIYAASTL
HSGVPDRFSGSGSGTDFTLKISRVEAEDVGVYYCQQSKEDPRTFGQGTKLEIKGGGGS
GGGGSGGGGSEVQLVQSGAEVKKPGASVKVSCKASGYTFTDYNMDWVRQAPGQGL
EWIGDINPNNGGTLYNQKFRGRVTLTVDKSISTAYMELSRLRSDDTAVYYCARSEVFY
GNYADYWGQGTTVTVSSEPKSCDKTHTCPPCPAPEAAGGPSVFLFPPKPKDTLMISRT
PEVTCVVVDVSHEDPEVKFNWYVDGVEVHNAKTKPREEQYNSTYRVVSVLTVLHQ
DWLNGKEYKCKVSNKALPAPIEKTISKAKGQPREPQVCTLPPSRDELTKNQVSLSCAV
KGFYPSDIAVEWESNGQPENNYKTTPPVLDSDGSFFLVSKLTVDKSRWQQGNVFSCS
VMHEALHNHYTQKSLSLSPGK (SEQ ID NO: 60).
B7H6-12 ScFv-Fc amino acid sequence 
DIVLTQTPLSLSVTPGQPASISCKASQSVDYDGDSYMNWYLQKPGQPPQLLIYAASTL
HSGVPDRFSGSGSGTDFTLKISRVEAEDVGVYYCQQSKEDPRTFGQGTKLEIKGGGGS
GGGGSGGGGSEVQLVQSGAEVKKPGASVKVSCKASGYTFTDYNMDWVRQAPGKGL
EWIGDINPNNGGTLYNQKFRGRVTLTVDKSISTAYMELSRLRSDDTAVYYCARSEVFY
GNYADYWGQGTTVTVSSEPKSCDKTHTCPPCPAPEAAGGPSVFLFPPKPKDTLMISRT
 
PEVTCVVVDVSHEDPEVKFNWYVDGVEVHNAKTKPREEQYNSTYRVVSVLTVLHQ
DWLNGKEYKCKVSNKALPAPIEKTISKAKGQPREPQVCTLPPSRDELTKNQVSLSCAV
KGFYPSDIAVEWESNGQPENNYKTTPPVLDSDGSFFLVSKLTVDKSRWQQGNVFSCS
VMHEALHNHYTQKSLSLSPGK (SEQ ID NO: 61).
B7H6-13 ScFv-Fc amino acid sequence 
DIVLTQTPLSLSVTPGQPASISCKASQSVDYDGDSYMNWYLQKPGQPPQLLIYAASTL
HSGIPDRFSGSGSGTDFTLKISRVEAEDVGVYYCQQSKEDPRTFGQGTKLEIKGGGGS
GGGGSGGGGSEVQLVQSGAEVKKPGASVKVSCKASGYTFTDYNMDWVRQAPGQGL
EWMGDINPNNGGTLYNQKFRGRVTMTVDTSISTAYMELSRLRSDDTAVYYCARSEVF
YGNYADYWGQGTTVTVSSEPKSCDKTHTCPPCPAPEAAGGPSVFLFPPKPKDTLMISR
TPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVCTLPPSRDELTKNQVSLSCAV
KGFYPSDIAVEWESNGQPENNYKTTPPVLDSDGSFFLVSKLTVDKSRWQQGNVFSCS
VMHEALHNHYTQKSLSLSPGK (SEQ ID NO: 62).
B7H6-14 ScFv-Fc amino acid sequence 
DIVLTQTPLSLSVTPGQPASISCKASQSVDYDGDSYMNWYLQKPGQPPQLLIYAASTL
HSGIPDRFSGSGSGTDFTLKISRVEAEDVGVYYCQQSKEDPRTFGQGTKLEIKGGGGS
GGGGSGGGGSEVQLVQSGAEVKKPGASVKVSCKASGYTFTDYNMDWVRQAPGQGL
EWIGDINPNNGGTLYNQKFRGRVTLTVDTSISTAYMELSRLRSDDTAVYYCARSEVFY
GNYADYWGQGTTVTVSSEPKSCDKTHTCPPCPAPEAAGGPSVFLFPPKPKDTLMISRT
PEVTCVVVDVSHEDPEVKFNWYVDGVEVHNAKTKPREEQYNSTYRVVSVLTVLHQ
DWLNGKEYKCKVSNKALPAPIEKTISKAKGQPREPQVCTLPPSRDELTKNQVSLSCAV
KGFYPSDIAVEWESNGQPENNYKTTPPVLDSDGSFFLVSKLTVDKSRWQQGNVFSCS
VMHEALHNHYTQKSLSLSPGK (SEQ ID NO: 63).
B7H6-15 ScFv-Fc amino acid sequence 
DIVLTQTPLSLSVTPGQPASISCKASQSVDYDGDSYMNWYLQKPGQPPQLLIYAASTL
HSGIPDRFSGSGSGTDFTLKISRVEAEDVGVYYCQQSKEDPRTFGQGTKLEIKGGGGS
GGGGSGGGGSEVQLVQSGAEVKKPGASVKVSCKASGYTFTDYNMDWVRQAPGQGL
EWIGDINPNNGGTLYNQKFRGRVTLTVDKSISTAYMELSRLRSDDTAVYYCARSEVFY
GNYADYWGQGTTVTVSSEPKSCDKTHTCPPCPAPEAAGGPSVFLFPPKPKDTLMISRT
 
PEVTCVVVDVSHEDPEVKFNWYVDGVEVHNAKTKPREEQYNSTYRVVSVLTVLHQ
DWLNGKEYKCKVSNKALPAPIEKTISKAKGQPREPQVCTLPPSRDELTKNQVSLSCAV
KGFYPSDIAVEWESNGQPENNYKTTPPVLDSDGSFFLVSKLTVDKSRWQQGNVFSCS
VMHEALHNHYTQKSLSLSPGK (SEQ ID NO: 64).
B7H6-16 ScFv-Fc amino acid sequence 
DIVLTQTPLSLSVTPGQPASISCKASQSVDYDGDSYMNWYLQKPGQPPQLLIYAASTL
HSGIPDRFSGSGSGTDFTLKISRVEAEDVGVYYCQQSKEDPRTFGQGTKLEIKGGGGS
GGGGSGGGGSEVQLVQSGAEVKKPGASVKVSCKASGYTFTDYNMDWVRQAPGKGL
EWIGDINPNNGGTLYNQKFRGRVTLTVDKSISTAYMELSRLRSDDTAVYYCARSEVFY
GNYADYWGQGTTVTVSSEPKSCDKTHTCPPCPAPEAAGGPSVFLFPPKPKDTLMISRT
PEVTCVVVDVSHEDPEVKFNWYVDGVEVHNAKTKPREEQYNSTYRVVSVLTVLHQ
DWLNGKEYKCKVSNKALPAPIEKTISKAKGQPREPQVCTLPPSRDELTKNQVSLSCAV
KGFYPSDIAVEWESNGQPENNYKTTPPVLDSDGSFFLVSKLTVDKSRWQQGNVFSCS
VMHEALHNHYTQKSLSLSPGK (SEQ ID NO: 65).
B7H6-17 ScFv-Fc amino acid sequence 
DIVLTQSPVSLAVPLGQRATISCKASQSVDYDGDSYMNWYQQKPGQPPKLLIYAASTL
HSGIPVRFSGSGSGTDFTLNIHPVEEEDAASYYCQQSKEDPRTFGGGTKLEIKGGGGS
GGGGSGGGGSEVLLQQSGPEVVKPGASVKITCKASGYTFTDYNMDWVKQSHGKSLE
WIGDINPNNAGTLYNQKFRGRVTLIVDKSSSTAYMELRSLTSDDTAVYYCARSEVFYG
NYADYWGQGTTLTVSSEPKSCDKTHTCPPCPAPEAAGGPSVFLFPPKPKDTLMISRTPE
VTCVVVDVSHEDPEVKFNWYVDGVEVHNAKTKPREEQYNSTYRVVSVLTVLHQDW
LNGKEYKCKVSNKALPAPIEKTISKAKGQPREPQVCTLPPSRDELTKNQVSLSCAVKG
FYPSDIAVEWESNGQPENNYKTTPPVLDSDGSFFLVSKLTVDKSRWQQGNVFSCSVM
HEALHNHYTQKSLSLSPGK (SEQ ID NO: 66).
B7H6-18 ScFv-Fc amino acid sequence

DIVLTQSPVSLAVPLGQRATISCKASQSVDYDADSYMNWYQQKPGQPPKLLIYAASTL
HSGIPVRFSGSGSGTDFTLNIHPVEEEDAASYYCQQSKEDPRTFGGGTKLEIKGGGGS
GGGGSGGGGSEVLLQQSGPEVVKPGASVKITCKASGYTFTDYNMDWVKQSHGKSLE
WIGDINPNNGGTLYNQKFRGRVTLIVDKSSSTAYMELRSLTSDDTAVYYCARSEVFYG
NYADYWGQGTTLTVSSEPKSCDKTHTCPPCPAPEAAGGPSVFLFPPKPKDTLMISRTPE

VTCVVVDVSHEDPEVKFNWYVDGVEVHNAKTKPREEQYNSTYRVVSVLTVLHQDW
LNGKEYKCKVSNKALPAPIEKTISKAKGQPREPQVCTLPPSRDELTKNQVSLSCAVKG
FYPSDIAVEWESNGQPENNYKTTPPVLDSDGSFFLVSKLTVDKSRWQQGNVFSCSVM
HEALHNHYTQKSLSLSPGK (SEQ ID NO: 67).

### Example 3: Detection of binding activity of CD3xB7H6 bispecific antibodies to antigen (ELISA)

In this example, the binding activity of CD3xB7H6 bispecific antibodies to a single antigen (the B7H6 antigen or the CD3 antigen) and the binding activity of the CD3xB7H6 bispecific antibodies simultaneously to the double antigens (the B7H6 antigen and the CD3 antigen) were detected, respectively. The specific experimental procedures are described below.

### 3.1 Determination of binding activity of CD3xB7H6 bispecific antibody to single antigen

Human B7H6 (ACRO biosystems, cat. No. B76-H52H8) and human CD3 antigen (ACRO biosystems, cat. No. CDD-H52W1) were diluted to 2 µg/mL with coating buffer (35 mM NaHCO₃, 15 mM Na₂CO₃, pH 9.6), respectively. 100 µL of the diluted solution was added to each well of an ELISA plate at 4°C overnight, and then washes with PBST (0.05% Tween 20-PBS, pH 7.2) 3 times. 300 µL of blocking buffer (1% BSA, 0.05% Tween20-PBS, pH 7.2) was added to the plate and allowed to stand at room temperature for 2 h, followed by washing with PBST three times. The corresponding bispecific antibody was added to each well and incubated at room temperature for 1 h, followed by washing with PBST three times. 100 µL of HRP-rabbit anti human IgG secondary antibody (Boster, cat. No. BA1070) diluted in blocking buffer was added to each well and incubated for 1 h at room temperature, followed by washing with PBST three times. TMB was added into each well. The plate was kept away from light at room temperature for 2-5 min, the reaction was stopped by adding 2 M sulfuric acid in each well, and finally OD450 values were read with microplate reader.

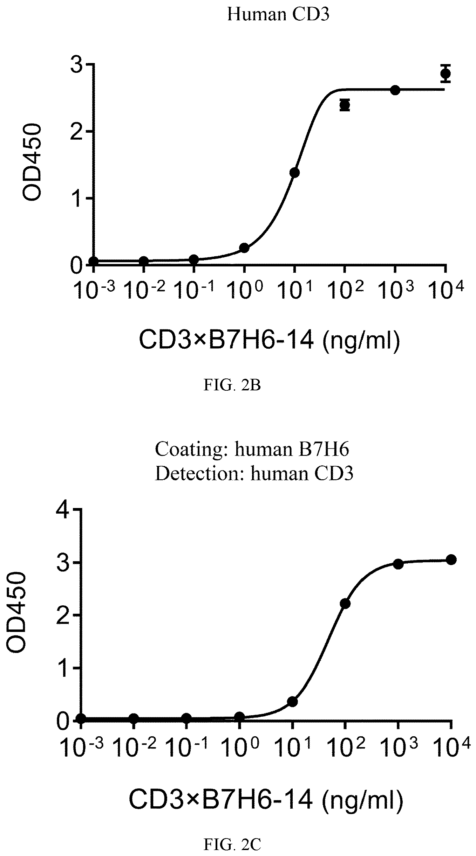

Specific experimental results are shown in FIG. 2A. FIG. 2A shows the binding of CD3xB7H6 bispecific antibody molecules to B7H6 antigen, the CD3xB7H6 bispecific antibody molecules being composed of different B7H6 sequences and CD3 sequences. It can be seen from FIG. 2 that these bispecific antibody molecules can all bind to B7H6 antigen. Among them, CD3xB7H6-14 has the strongest binding activity. Therefore, subsequent experiments mainly focus on CD3xB7H6-14. FIG. 2B shows that CD3xB7H6-14 bispecific antibody molecules can bind to CD3 antigen.

### 3.2 Determination of binding activity of CD3xB7H6 bispecific antibody and double antigen

Human B7H6 (ACRO biosystems, cat. No. B76-H82Wb) was diluted to 2 µg/mL with coating buffer (35 mM NaHCO₃, 15 mM Na₂CO₃, pH 9.6), and 100 µL of the diluted solution was added to each well of an ELISA plate at 4°C overnight. The plate was washed with PBST (0.05% Tween 20-PBS, pH 7.2) three times. 300 µL of blocking buffer (1% BSA, 0.05% Tween20-PBS, pH 7.2) was added to the plate and allowed to stand at room temperature for 2 h, followed by washing with PBST three times. The corresponding bispecific antibody was added to each well and incubated at room temperature for 1 h, followed by washing with PBST three times. 100 µL of human CD3 antigen (ACRO biosystems, cat. No. CDD-H52W1) diluted in blocking buffer was added to each well and incubated for 1 h at room temperature, followed by washing with PBST three times. 100 µL of HRP-anti-His tagged secondary antibody (Genscript, cat. No. A00612) diluted in blocking buffer was added to each well and incubated for 1 h at room temperature, followed by washing with PBST three times. TMB was added into each well. The plate was kept away from light at room temperature for 2-5 min. The reaction was stopped by adding 2 M sulfuric acid in each well, and finally, OD450 values were read with a microplate reader. The results are shown in FIG. 2C and show that as the concentration of the CD3xB7H6-14 bispecific antibody molecule increases, the OD450 reading increases, indicating that the bispecific antibody molecule can bind to both human CD3 and B7H6 antigens.

### Example 4: In vitro killing detection of bispecific antibody molecules

### 4.1 Isolation of human peripheral blood mononuclear cells (PBMCs)

Anticoagulated human peripheral blood was added into an equal volume of sterile PBS, and 25 mL of diluted peripheral blood was gently added onto 15 mL of lymph separation fluid (GE healthcare, cat. No. 17144003) in a 50 mL sterile centrifuge tube. The interface was kept clear without shaking during the whole process, followed by centrifuging at 21°C for 30 min at 400 g, with the centrifuge increasing at speed 1 and decreasing at speed 0. After the centrifugation, the tube was divided into three layers, with the lymphocyte layer in the middle layer. The white membrane layer in the middle layer was sucked and added into a new 50 mL centrifuge tube, added with PBS to 50 mL after completion of suction, and then centrifugation was performed at 500 g for 10 min. The supernatant was discarded, 1 mL PBS was added to gently blow, PBS was added to 40 mL, and centrifugation was performed for 10 min at 250 g.

Supernatants were discarded after centrifugation, the remaining was resuspended with 1 mL of MACS buffer (0.5% BSA, 2.5 mM EDTA-PBS), and counted by dilution.

### 4.2 Isolation of purified T-cells

Human T cells were isolated from peripheral blood using magnetic bead sorting (Miltenyi, cat. No. 130096535). After PBMCs were collected, the supernatant was discarded, 40 µL of MACS buffer was added for every 10⁷ cells to resuspend the cells, then 10 µL of T Cell Biotin-Antibody Cocktail was added, mixed well, and incubated at 4°C for 10 min. 30 µL of MACS buffer and 20 µL of T Cell MicroBead Cocktail were added per 10⁷ cells, mixed well, and incubated for 15 min at 4°C. The LS separation column was placed in the magnetic field, the separation column was wetted with 3 mL MACS buffer, the cell suspension was added to the separation column, after the centrifuge tube was rinsed with 1 mL MACS buffer, the separation column was added, and the effluent was collected. The column was washed with 3 mL MACS buffer and the effluent was collected. After mixing well, the cells were counted. 300 g of the cell suspension was centrifuged at 4°C for 10 min and resuspended in a culture medium.

### 4.3 In vitro killing detection of adherent tumor cells

Adherently cultured tumor cells were digested and counted, and the cell density thereof was adjusted to 2 x 10⁵/mL. RTCA instrument (Agilent) was activated to select instrument type DP and experiment mode, and to fill in cell information and medicament information. After entering the schedule set-up experiment step, 50 µL of fresh medium (89% RPMI 1640 medium+ 10% fetal bovine serum+ 1% Penicillin Streptomycin) was added to the plate, and the plate was put into the instrument. The instrument was closed, and the first step initiated by clicking. The plate was taken out after the first step was done, added with 100 µL of cell suspension, and left to stand at room temperature for 15-30 min to prevent edge effect. Then, the plate was put into the instrument, and Start button was clicked. After the cells grew to the log phase, the procedure was stopped, 50 µL of purified T cells (1 x 10⁶/mL) and the bispecific antibodies at gradient concentrations were added, followed by clicking the Start button and performing the analysis after some time.

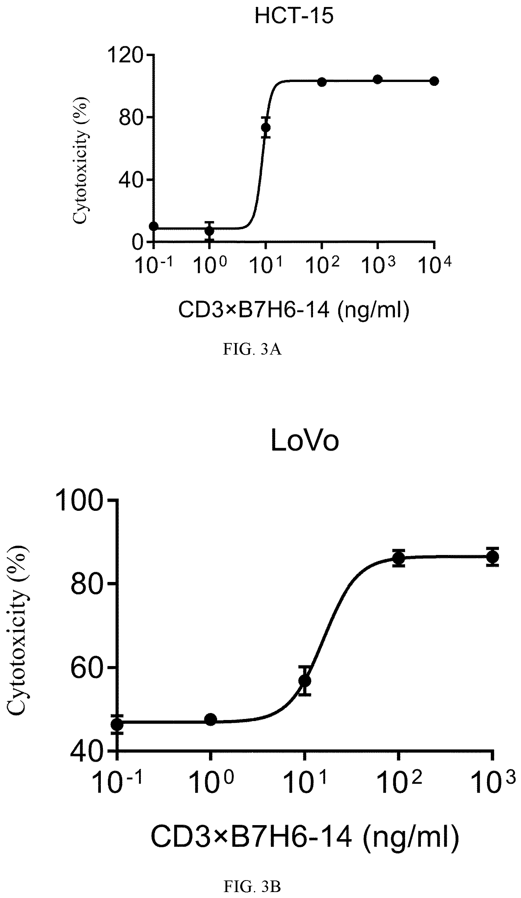

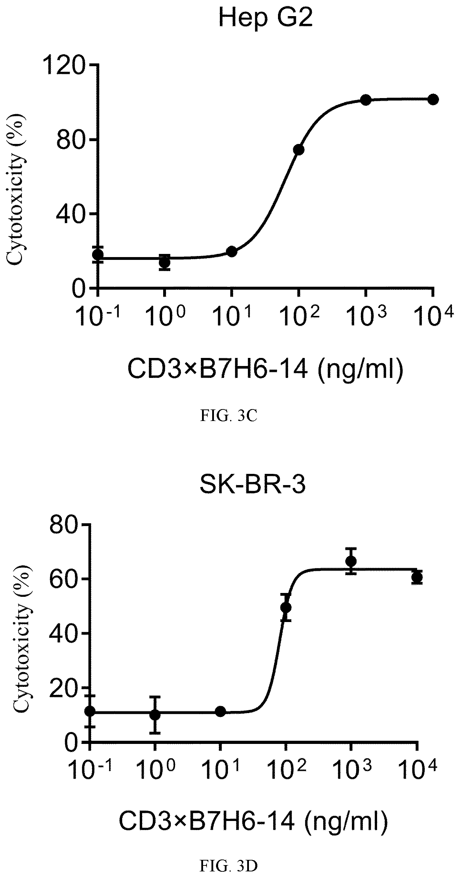

Specific experimental results are shown in the figures, in which the killing efficiency of T cells on HCT-15 cells (FIG. 3A), LoVo cells (FIG. 3B), Hep G2 cells (FIG. 3C), and SK-BR-3 cells (FIG. 3D) increased an increase in CD3xB7H6-14 bispecific antibody concentration, indicating that CD3xB7H6-14 can promote T cells to kill B7H6+ tumor cells.

### Example 5: Detection of in vitro killing of suspension cells by the luciferase reaction

After the luciferase-overexpressing tumor cells were collected, the cell density thereof was adjusted to 2 x 10⁵/mL. T cells were isolated and purified, collected, purified, and adjusted to 5 x 10⁵/mL, as described in Examples 4.1 and 4.2. 50 µL of the tumor cells and 50 µL of purified T cells were plated in a 96-well flat bottom plate and added with the bispecific antibodies at gradient concentrations and mixed well. After incubation at 37°C, 5% CO₂ for 4 h, 100 µL (15 mg/mL) of fluorescein potassium salt solution (Goldbio, cat. No. LUCK-1G) was added to each well, mixed well quickly, and chemiluminescence values were detected with a microplate reader.

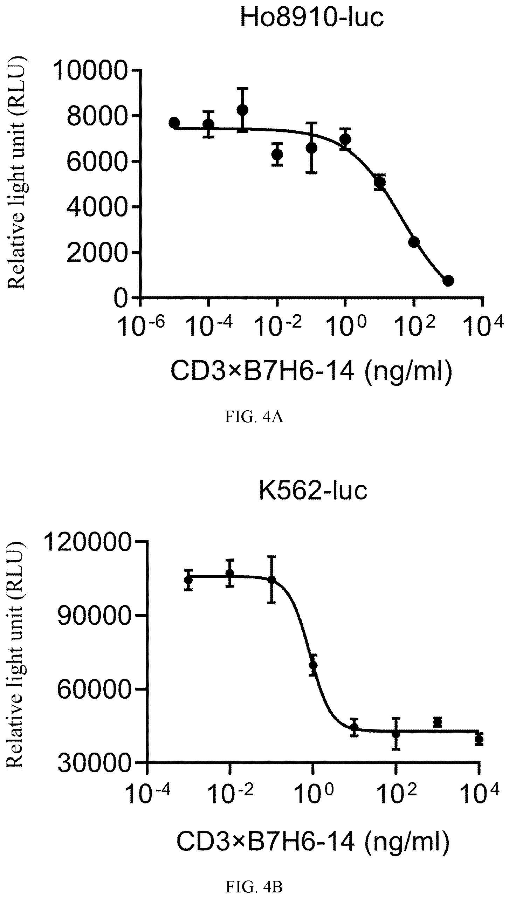

Specific experimental results are shown in the figure, the chemiluminescence values of Ho8910 and K562 were gradually decreased as the concentration of CD3xB7H6-14 bispecific antibody increased, indicating that fewer tumor cells survived, i.e., CD3xB7H6-14 can significantly promote T cell killing of Ho8910 cells (FIG. 4A) and K562 cells (FIG. 4B).

### Example 6: Detection of T cell proliferation in the presence of B7H6 positive tumor cells

To determine T cell proliferation, this example was set up as a 2 * 2 two-factor experiment, including the following groups: T cells + PBS group, T cells + CD3xB7H6-14 group, HCT-15 + T cells + PBS group, HCT-15 + T cells+ CD3xB7H6-14 group. T cells were purified and isolated as described in Examples 4.1 and 4.2 and labeled with 5 µM cell Trace CFSE (Invitrogen, cat. No. C34554). The labeled T cells were then mixed with human colon cancer cells (HCT-15 cells) at an effect target ratio of 2: 1, to which PBS or a bispecific antibody was added. After 3 days, the cells were harvested and T cell proliferation was detected by flow cytometer.

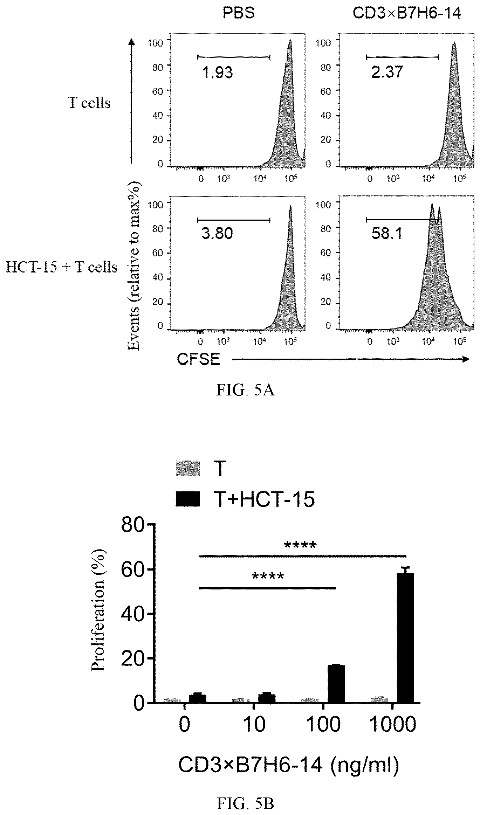

Specific experimental results are shown in the figures. The CD3xB7H6-14 bispecific antibodies significantly promoted T cell proliferation in the presence of HCT-15 (FIG. 5A), and the effect on T cell proliferation was more significant as the concentration of the bispecific antibody CD3xB7H6-14 was increased (FIG. 5B).

### Example 7: Detection of T cell activation and degranulation levels in the presence of B7H6 positive tumor cells

T cells are isolated and purified as described in Examples 4.1 and 4.2. Two groups are set up in the experiment. The control group was a T cell group, and in the experimental group, T cells and HCT-15 target cells were added into a 96-well plate at an effect target ratio of 1: 1, and then CD3xB7H6 bispecific antibodies at gradient concentrations were added, and mixed well. After incubation at 37°C for 24 h, the cells were collected by centrifugation, resuspended with PBS, blocked with mice serum at room temperature for 15 min, and then added with antibody for flow cytometry for detection of surface molecules. After incubation at 4°C for 30 min in the dark, the cells were washed with PBS, and then fixed transmembrane solution (Invitrogen, cat. No. 00512343) was added for fixing at room temperature for more than half an hour. After washing with transmembrane buffer (Invitrogen, cat. No. 00833356) and blocking with mice serum at room temperature for 15 min, the corresponding intracellular molecular labeled antibody was added, incubated at room temperature for 30 min in the dark, washed twice with PBS and detected by flow cytometer.

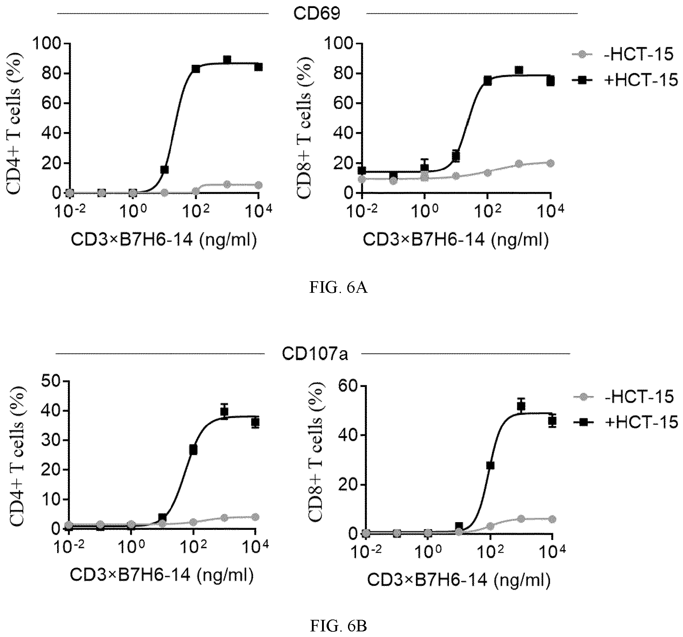

Specific experimental results are shown in FIG. 6A and FIG. 6B. In the presence of B7H6 + cells (+HCT-15), as the concentration of CD3xB7H6-14 bispecific antibody increased, the proportion of CD69 molecules expressed on the surface of CD4 + T cells and CD8 + T cells (FIG. 6A) and CD107a expressed in the cells (FIG. 6B) gradually increased, while in the absence of B7H6+ tumor cells (-HCT-15), the activation and degranulation of T cells after bispecific antibody incubation was limited. This indicates that the bispecific antibody can specifically target T cells and B7H6+ tumor cells and promote T cell activation and degranulation.

### Example 8: Detection of cytokines secreted by T cells in the presence of B7H6 positive tumor cells

T cells are isolated and purified as described in Examples 4.1 and 4.2. The control group only contained T cells, and the experimental group contained T cells and HCT-15 cells. 5 x 10⁵ T cells, 5 x 10⁵ T cells and 5 x 10⁵ HCT-15 cells were respectively added into a 24-well plate in a total volume of 500 µL, and then CD3xB7H6 bispecific antibodies at gradient concentrations (0, 10⁰, 10¹, 10², 10³) were added, and mixed well. After incubation at 37°C for 24 h, the supernatant was collected by centrifugation, and the concentration of each cytokine in the supernatant was determined using the CBA human Th1/Th2/Th17 cytokine detection kit (BD, cat. No. 560484) and finally determined by flow cytometer.

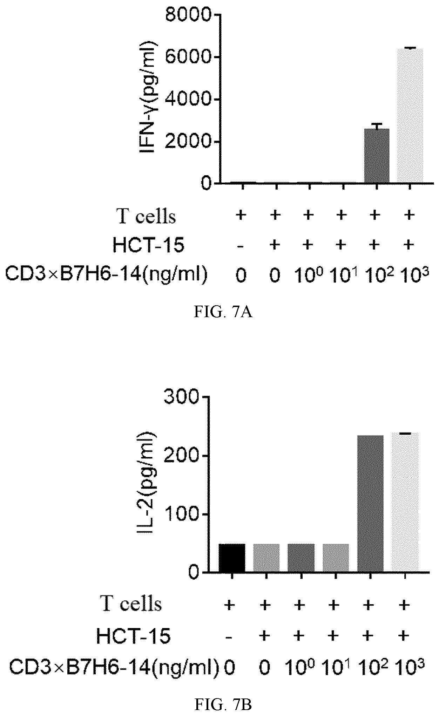

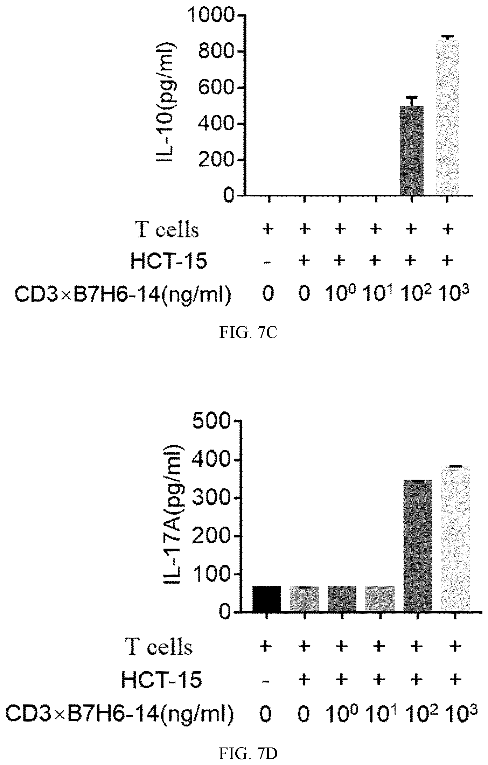

Specific experimental results are shown in FIG. 7A to FIG. D, in which the concentrations of IFN-y, IL-2, IL-10 and IL-17A cytokines in the cell co-incubation supernatant were shown, respectively. In the presence of HCT-15 tumor cells, the levels of these four cytokines secreted by T cells increased gradually with the increase of CD3xB7H6-14 antibody concentration, indicating that the bispecific antibody can promote T cell activation and cytokine secretion.

### Example 9: Study on the efficacy of xenotransplantation in vivo

To detect the *in vivo* anti-tumor activity of CD3xB7H6 bispecific antibody molecule on HCT-15 tumor cells, this example set up three groups for the experiment, including buffer group, human immunoglobulin (IGg) group and CD3xB7H6-14 bispecific antibody molecule group. T cells were isolated and purified as described in Examples 4.1 and 4.2 and amplified for 7 days using the T cell amplification kit (Gibco, cat. No. 11131D). HCT-15 colorectal cancer cells (1 x 10⁶) were mixed with amplified T cells (2 x 10⁶) and subcutaneously injected (s. c.) into male NOD-Prkdcscid Il2rgem1/Smoc mice, and CD3xB7H6 bispecific antibody (1mg/kg in PBS), IgG control antibody (sigma, cat. No. I4506) or buffer (control solvent) was intravenously injected into the mice three times every two days after inoculation. Tumor length and width were measured by external calipers and tumor volume was calculated using standard formulas.

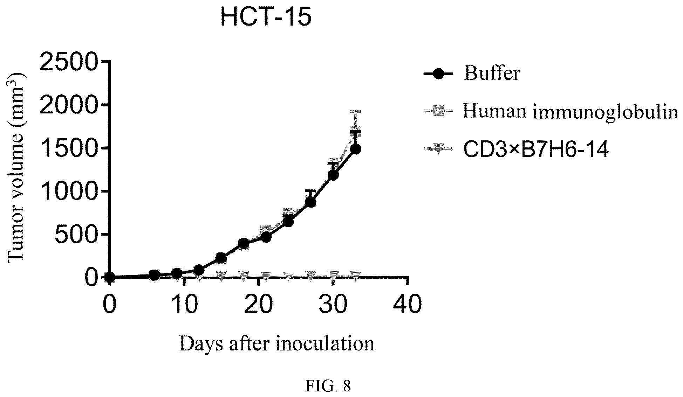

FIG. 8 shows the *in vivo* antitumor activity of CD3xB7H6-14 bispecific antibody molecules on HCT-15 cells. Treatment with CD3xB7H6-14 bispecific antibody significantly promoted the regression of HCT-15 tumor, but the therapeutic effect could not be achieved by using control IgG or control solvent, indicating that CD3xB7H6 bispecific antibody had significant anti-tumor activity *in vivo.*

In the description of the present disclosure, references to descriptions of the terms "one embodiment", "some embodiments", "examples", "specific examples", or "some examples", etc. mean that a particular feature, structure, material, or characteristic described in connection with the embodiment or example is included in at least one embodiment or example of the present disclosure. In this specification, schematic representations of the above terms are not necessarily directed to the same embodiment or example. Furthermore, the particular features, structures, materials, or characteristics described may be combined in any suitable manner in any one or more embodiments or examples. Furthermore, those skilled in the art can combine the various embodiments or examples as well as features in the various embodiments or examples described in this specification, without departing from the scope of the present disclosure.

While the embodiments of the present disclosure have been illustrated and described above, it will be understood that the above-described embodiments are illustrative and not restrictive and that changes, modifications, substitutions, and alterations may be made by those skilled in the art without departing from the scope of the present disclosure.

## Claims

1. A bispecific recombinant antibody, comprising:
CD3 antibody variable region CDR sequences as set forth in SEQ ID NO: 1 to SEQ ID NO: 6; and
B7H6 antibody variable region CDR sequences as set forth in SEQ ID NO: 7 to SEQ ID NO: 12.

2. The bispecific recombinant antibody according to claim 1, comprising CD3 antibody variable regions, wherein the CD3 antibody variable regions comprise:
a light chain variable region as set forth in SEQ ID NO: 13 and a heavy chain variable region as set forth in SEQ ID NO: 14.

3. The bispecific recombinant antibody according to claim 1, comprising B7H6 antibody variable regions, wherein:
the B7H6 antibody variable regions comprise: a light chain variable region as set forth in SEQ ID NO: 20 and a heavy chain variable region of the amino acid sequence as set forth in SEQ ID NO: 23; or
the B7H6 antibody variable regions comprise: a light chain variable region as set forth in SEQ ID NO: 16 and a heavy chain variable region of the amino acid sequence as set forth in SEQ ID NO: 22; or
the B7H6 antibody variable regions comprise: a light chain variable region as set forth in SEQ ID NO: 16 and a heavy chain variable region of the amino acid sequence as set forth in SEQ ID NO: 23; or
the B7H6 antibody variable regions comprise: a light chain variable region as set forth in SEQ ID NO: 16 and a heavy chain variable region of the amino acid sequence as set forth in SEQ ID NO: 24; or
the B7H6 antibody variable regions comprise: a light chain variable region as set forth in SEQ ID NO: 16 and a heavy chain variable region of the amino acid sequence as set forth in SEQ ID NO: 25; or
the B7H6 antibody variable regions comprise: a light chain variable region as set forth in SEQ ID NO: 17 and a heavy chain variable region of the amino acid sequence as set forth in SEQ ID NO: 22; or
the B7H6 antibody variable regions comprise: a light chain variable region as set forth in SEQ ID NO: 17 and a heavy chain variable region of the amino acid sequence as set forth in SEQ ID NO: 23; or
the B7H6 antibody variable regions comprise: a light chain variable region as set forth in SEQ ID NO: 17 and a heavy chain variable region of the amino acid sequence as set forth in SEQ ID NO: 24; or
the B7H6 antibody variable regions comprise: a light chain variable region as set forth in SEQ ID NO: 17 and a heavy chain variable region of the amino acid sequence as set forth in SEQ ID NO: 25; or
the B7H6 antibody variable regions comprise: a light chain variable region as set forth in SEQ ID NO: 19 and a heavy chain variable region of the amino acid sequence as set forth in SEQ ID NO: 22; or
the B7H6 antibody variable regions comprise: a light chain variable region as set forth in SEQ ID NO: 19 and a heavy chain variable region of the amino acid sequence as set forth in SEQ ID NO: 23; or
the B7H6 antibody variable regions comprise: a light chain variable region as set forth in SEQ ID NO: 19 and a heavy chain variable region of the amino acid sequence as set forth in SEQ ID NO: 24; or
the B7H6 antibody variable regions comprise: a light chain variable region as set forth in SEQ ID NO: 19 and a heavy chain variable region of the amino acid sequence as set forth in SEQ ID NO: 25; or
the B7H6 antibody variable regions comprise: a light chain variable region as set forth in SEQ ID NO: 20 and a heavy chain variable region of the amino acid sequence as set forth in SEQ ID NO: 22; or
the B7H6 antibody variable regions comprise: a light chain variable region as set forth in SEQ ID NO: 20 and a heavy chain variable region of the amino acid sequence as set forth in SEQ ID NO: 24; or
the B7H6 antibody variable regions comprise: a light chain variable region as set forth in SEQ ID NO: 20 and a heavy chain variable region of the amino acid sequence as set forth in SEQ ID NO: 25; or
the B7H6 antibody variable regions comprise: a light chain variable region as set forth in SEQ ID NO: 15 and a heavy chain variable region of the amino acid sequence as set forth in SEQ ID NO: 26; or
the B7H6 antibody variable regions comprise: a light chain variable region as set forth in SEQ ID NO: 18 and a heavy chain variable region of the amino acid sequence as set forth in SEQ ID NO: 21.

4. The bispecific recombinant antibody according to claim 2, further comprising a linker peptide, optionally, the linker peptide having an amino acid sequence as set forth in SEQ ID NO: 46, wherein:
an N-end of the linker peptide is linked to a C-end of an CD3 antibody light chain variable region; and
a C-end of the linker peptide is linked to an N-end of an CD3 antibody heavy chain variable region.

5. The bispecific recombinant antibody according to claim 3, further comprising a linker peptide, optionally, the linker peptide having an amino acid sequence as set forth in SEQ ID NO: 46, wherein:
an N-end of the linker peptide is linked to a C-end of a B7H6 light chain variable region; and
a C-end of the linker peptide is linked to an N-end of the B7H6 heavy chain variable region.

6. The bispecific recombinant antibody according to claim 1, further comprising a first Fc region and a second Fc region, wherein at least a portion of the first Fc region and the second Fc region is derived from at least one of a murine antibody, a human antibody, a primate antibody, or a mutant thereof.

7. The bispecific recombinant antibody according to claim 6, wherein at least a portion of the first Fc region and the second Fc region is derived from human IgG or a mutant thereof;
optionally, at least a portion of the first Fc region and the second Fc region is derived from human IgG1 or a mutant thereof.

8. The bispecific recombinant antibody according to claim 6, wherein the first Fc region has at least one of S384C mutation and T396W mutation as compared to a Fc region of wild-type IgG1.

9. The bispecific recombinant antibody according to claim 8, wherein the second Fc region has at least one of Y380C mutation, T397S mutation, L399A mutation, and Y408V mutation as compared to the Fc region of wild-type IgG1.

10. The bispecific recombinant antibody according to any one of claims 6 to 9, wherein:
the first Fc region has an amino acid sequence as set forth in SEQ ID NO: 47; and
the second Fc region has an amino acid sequence as set forth in SEQ ID NO: 48.

11. A bispecific recombinant antibody, comprising amino acid sequences as set forth in SEQ ID NO: 49 and SEQ ID NO: 63.

12. A nucleic acid, encoding the bispecific recombinant antibody according to any one of claims 1 to 11.

13. The nucleic acid according to claim 12, having a nucleotide sequence as set forth in SEQ ID NO: 69 and at least one of nucleotide sequences as set forth in SEQ ID NO: 70 to SEQ ID NO: 87.

14. An expression vector, carrying the nucleic acid according to claim 12 or 13.

15. A recombinant cell, carrying the nucleic acid according to claim 12 or 13, the expression vector according to claim 14, or the bispecific recombinant antibody according to any one of claims 1 to 11.

## Patentansprüche

1. Bispezifischer rekombinanter Antikörper, umfassend:
CDR-Sequenzen der CD3-Antikörpervariable Region gemäß SEQ ID NR: 1 bis SEQ ID NR: 6; und
CDR-Sequenzen der B7H6-Antikörpervariable Region gemäß SEQ ID NR: 7 bis SEQ ID NR: 12.

2. Bispezifischer rekombinanter Antikörper nach Anspruch 1, umfassend CD3-Antikörpervariable Regionen, wobei die CD3-Antikörpervariable Regionen umfassen:
eine leichte Kettenvariable Region gemäß SEQ ID NR: 13, und eine schwere Kettenvariable Region gemäß SEQ ID NR: 14.

3. Bispezifischer rekombinanter Antikörper nach Anspruch 1, umfassend B7H6-Antikörpervariable Regionen, wobei:
die B7H6-Antikörpervariablen Regionen umfassen: eine leichte Kettenvariable Region gemäß SEQ ID NR: 20, und eine schwere Kettenvariable Region der Aminosäuresequenz gemäß SEQ ID NR: 23; oder
die B7H6-Antikörpervariablen Regionen umfassen: eine leichte Kettenvariable Region gemäß SEQ ID NR: 16, und eine schwere Kettenvariable Region der Aminosäuresequenz gemäß SEQ ID NR: 22; oder
die B7H6-Antikörpervariablen Regionen umfassen: eine leichte Kettenvariable Region gemäß SEQ ID NR: 16, und eine schwere Kettenvariable Region der Aminosäuresequenz gemäß SEQ ID NR: 23; oder
die B7H6-Antikörpervariablen Regionen umfassen: eine leichte Kettenvariable Region gemäß SEQ ID NR: 16, und eine schwere Kettenvariable Region der Aminosäuresequenz gemäß SEQ ID NR: 24; oder
die B7H6-Antikörpervariablen Regionen umfassen: eine leichte Kettenvariable Region gemäß SEQ ID NR: 16, und eine schwere Kettenvariable Region der Aminosäuresequenz gemäß SEQ ID NR: 25; oder
die B7H6-Antikörpervariablen Regionen umfassen: eine leichte Kettenvariable Region gemäß SEQ ID NR: 17, und eine schwere Kettenvariable Region der Aminosäuresequenz gemäß SEQ ID NR: 22; oder
die B7H6-Antikörpervariablen Regionen umfassen: eine leichte Kettenvariable Region gemäß SEQ ID NR: 17, und eine schwere Kettenvariable Region der Aminosäuresequenz gemäß SEQ ID NR: 23; oder
die B7H6-Antikörpervariablen Regionen umfassen: eine leichte Kettenvariable Region gemäß SEQ ID NR: 17, und eine schwere Kettenvariable Region der Aminosäuresequenz gemäß SEQ ID NR: 24; oder
die B7H6-Antikörpervariablen Regionen umfassen: eine leichte Kettenvariable Region gemäß SEQ ID NR: 17, und eine schwere Kettenvariable Region der Aminosäuresequenz gemäß SEQ ID NR: 25; oder
die B7H6-Antikörpervariablen Regionen umfassen: eine leichte Kettenvariable Region gemäß SEQ ID NR: 19, und eine schwere Kettenvariable Region der Aminosäuresequenz gemäß SEQ ID NR: 22; oder
die B7H6-Antikörpervariablen Regionen umfassen: eine leichte Kettenvariable Region gemäß SEQ ID NR: 19, und eine schwere Kettenvariable Region der Aminosäuresequenz gemäß SEQ ID NR: 23; oder
die B7H6-Antikörpervariablen Regionen umfassen: eine leichte Kettenvariable Region gemäß SEQ ID NR: 19, und eine schwere Kettenvariable Region der Aminosäuresequenz gemäß SEQ ID NR: 24; oder
die B7H6-Antikörpervariablen Regionen umfassen: eine leichte Kettenvariable Region gemäß SEQ ID NR: 19, und eine schwere Kettenvariable Region der Aminosäuresequenz gemäß SEQ ID NR: 25; oder
die B7H6-Antikörpervariablen Regionen umfassen: eine leichte Kettenvariable Region gemäß SEQ ID NR: 20, und eine schwere Kettenvariable Region der Aminosäuresequenz gemäß SEQ ID NR: 22; oder
die B7H6-Antikörpervariablen Regionen umfassen: eine leichte Kettenvariable Region gemäß SEQ ID NR: 20, und eine schwere Kettenvariable Region der Aminosäuresequenz gemäß SEQ ID NR: 24; oder
die B7H6-Antikörpervariablen Regionen umfassen: eine leichte Kettenvariable Region gemäß SEQ ID NR: 20, und eine schwere Kettenvariable Region der Aminosäuresequenz gemäß SEQ ID NR: 25; oder
die B7H6-Antikörpervariablen Regionen umfassen: eine leichte Kettenvariable Region gemäß SEQ ID NR: 15, und eine schwere Kettenvariable Region der Aminosäuresequenz gemäß SEQ ID NR: 26; oder
die B7H6-Antikörpervariablen Regionen umfassen: eine leichte Kettenvariable Region gemäß SEQ ID NR: 18, und eine schwere Kettenvariable Region der Aminosäuresequenz gemäß SEQ ID NR: 21.

4. Bispezifischer rekombinanter Antikörper nach Anspruch 2, weiterhin umfassend ein Linker-Peptid, wobei optional das Linker-Peptid eine Aminosäuresequenz gemäß SEQ ID Nr. 46, aufweist, wobei:
ein N-Ende des Linker-Peptids mit einem C-Ende einer leichten Kettenvariablen Region eines CD3-Antikörpers verknüpft ist; und
ein C-Ende des Linker-Peptids mit einem N-Ende einer schweren Kettenvariablen Region eines CD3-Antikörpers verknüpft ist.

5. Bispezifischer rekombinanter Antikörper nach Anspruch 3, ferner umfassend ein Linker-Peptid, wobei optional das Linker-Peptid eine Aminosäuresequenz gemäß SEQ ID Nr. 46, aufweist, wobei:
ein N-Ende des Linker-Peptids mit einem C-Ende einer leichten Kettenvariablen Region von B7H6 verknüpft ist; und
ein C-Ende des Linker-Peptids mit einem N-Ende einer schweren Kettenvariablen Region von B7H6 verknüpft ist.

6. Bispezifischer rekombinanter Antikörper nach Anspruch 1, der weiterhin umfassend eine erste Fc-Region und eine zweite Fc-Region, wobei zumindest ein Teil der ersten Fc-Region und der zweiten Fc-Region von mindestens einem murinen Antikörper, einem humanen Antikörper, einem Primatenantikörper oder einer Mutante davon abgeleitet ist.

7. Bispezifischer rekombinanter Antikörper nach Anspruch 6, wobei mindestens ein Teil der ersten Fc-Region und der zweiten Fc-Region von humanem IgG oder einer Mutante davon abgeleitet ist;
wobei optional mindestens ein Teil der ersten Fc-Region und der zweiten Fc-Region von humanem IgG1 oder einer Mutante davon abgeleitet ist.

8. Bispezifischer rekombinanter Antikörper nach Anspruch 6, wobei die erste Fc-Region im Vergleich zu einer Fc-Region von Wildtyp-IgG1 mindestens eine der folgenden Mutationen aufweist: S384C-Mutation und T396W-Mutation.

9. Bispezifischer rekombinanter Antikörper nach Anspruch 8, wobei die zweite Fc-Region im Vergleich zur Fc-Region von Wildtyp-IgG1 mindestens eine der folgenden Mutationen aufweist: Y380C-Mutation, T397S-Mutation, L399A-Mutation und Y408V-Mutation.

10. Bispezifischer rekombinanter Antikörper nach einem der Ansprüche 6 bis 9, wobei:
die erste Fc-Region eine Aminosäuresequenz aufweist gemäß SEQ ID NR: 47; und
die zweite Fc-Region eine Aminosäuresequenz aufweist gemäß SEQ ID NR: 48.

11. Bispezifischer rekombinanter Antikörper, umfassend die Aminosäuresequenzen gemäß SEQ ID NR: 49 und SEQ ID NR: 63.

12. Nukleinsäure, die den bispezifischen rekombinanten Antikörper nach einem der Ansprüche 1 bis 11 codiert.

13. Nukleinsäure nach Anspruch 12, mit einer Nukleotidsequenz gemäß SEQ ID NR: 69, und mindestens einer der Nukleotidsequenzen gemäß SEQ ID NR: 70 bis SEQ ID NR: 87.

14. Expressionsvektor, der die Nukleinsäure nach Anspruch 12 oder 13 trägt.

15. Rekombinante Zelle, die die Nukleinsäure nach Anspruch 12 oder 13, den Expressionsvektor nach Anspruch 14 oder den bispezifischen rekombinanten Antikörper nach einem der Ansprüche 1 bis 11 trägt.

## Revendications

1. Un anticorps recombinant bispécifique, comprenant :
des séquences CDR de région variable d'anticorps anti-CD3 telles que définies dans SEQ ID NO : 1 à SEQ ID NO : 6 ; et
des séquences CDR de région variable d'anticorps anti-B7H6 telles que définies dans SEQ ID NO : 7 à SEQ ID NO : 12.

2. Anticorps recombinant bispécifique selon la revendication 1, **caractérisé en ce qu'**il comprend des régions variables d'anticorps anti-CD3, lesquelles régions variables d'anticorps anti-CD3 comprennent :
une région variable de chaîne légère telle que définie dans SEQ ID NO : 13 et une région variable de chaîne lourde telle que définie dans SEQ ID NO : 14.

3. Anticorps recombinant bispécifique selon la revendication 1, **caractérisé en ce qu'**il comprend des régions variables d'anticorps anti-B7H6, comprennent :
les régions variables d'anticorps anti-B7H6 comprennent : une région variable de chaîne légère telle que définie dans SEQ ID NO : 20 et une région variable de chaîne lourde de la séquence d'acides aminés telle que définie dans SEQ ID NO : 23 ; ou
les régions variables d'anticorps anti-B7H6 comprennent : une région variable de chaîne légère telle que définie dans SEQ ID NO : 16 et une région variable de chaîne lourde de la séquence d'acides aminés telle que définie dans SEQ ID NO : 22 ; ou
les régions variables d'anticorps anti-B7H6 comprennent : une région variable de chaîne légère telle que définie dans SEQ ID NO : 16 et une région variable de chaîne lourde de la séquence d'acides aminés telle que définie dans SEQ ID NO : 23 ; ou
les régions variables d'anticorps anti-B7H6 comprennent : une région variable de chaîne légère telle que définie dans SEQ ID NO : 16 et une région variable de chaîne lourde de la séquence d'acides aminés telle que définie dans SEQ ID NO : 24 ; ou
les régions variables d'anticorps anti-B7H6 comprennent : une région variable de chaîne légère telle que définie dans SEQ ID NO : 16 et une région variable de chaîne lourde de la séquence d'acides aminés telle que définie dans SEQ ID NO : 25 ; ou
les régions variables d'anticorps anti-B7H6 comprennent : une région variable de chaîne légère telle que définie dans SEQ ID NO : 17 et une région variable de chaîne lourde de la séquence d'acides aminés telle que définie dans SEQ ID NO : 22 ; ou
les régions variables d'anticorps anti-B7H6 comprennent : une région variable de chaîne légère telle que définie dans SEQ ID NO : 17 et une région variable de chaîne lourde de la séquence d'acides aminés telle que définie dans SEQ ID NO : 23 ; ou
les régions variables d'anticorps anti-B7H6 comprennent : une région variable de chaîne légère telle que définie dans SEQ ID NO : 17 et une région variable de chaîne lourde de la séquence d'acides aminés telle que définie dans SEQ ID NO : 24 ; ou
les régions variables d'anticorps anti-B7H6 comprennent : une région variable de chaîne légère telle que définie dans SEQ ID NO : 17 et une région variable de chaîne lourde de la séquence d'acides aminés telle que définie dans SEQ ID NO : 25 ; ou
les régions variables d'anticorps anti-B7H6 comprennent : une région variable de chaîne légère telle que définie dans SEQ ID NO : 19 et une région variable de chaîne lourde de la séquence d'acides aminés telle que définie dans SEQ ID NO : 22 ; ou
les régions variables d'anticorps anti-B7H6 comprennent : une région variable de chaîne légère telle que définie dans SEQ ID NO : 19 et une région variable de chaîne lourde de la séquence d'acides aminés telle que définie dans SEQ ID NO : 23 ; ou
les régions variables d'anticorps anti-B7H6 comprennent : une région variable de chaîne légère telle que définie dans SEQ ID NO : 19 et une région variable de chaîne lourde de la séquence d'acides aminés telle que définie dans SEQ ID NO : 24 ; ou
les régions variables d'anticorps anti-B7H6 comprennent : une région variable de chaîne légère telle que définie dans SEQ ID NO : 19 et une région variable de chaîne lourde de la séquence d'acides aminés telle que définie dans SEQ ID NO : 25 ; ou
les régions variables d'anticorps anti-B7H6 comprennent : une région variable de chaîne légère telle que définie dans SEQ ID NO : 20 et une région variable de chaîne lourde de la séquence d'acides aminés telle que définie dans SEQ ID NO : 22 ; ou
les régions variables d'anticorps anti-B7H6 comprennent : une région variable de chaîne légère telle que définie dans SEQ ID NO : 20 et une région variable de chaîne lourde de la séquence d'acides aminés telle que définie dans SEQ ID NO : 24 ; ou
les régions variables d'anticorps anti-B7H6 comprennent : une région variable de chaîne légère telle que définie dans SEQ ID NO : 20 et une région variable de chaîne lourde de la séquence d'acides aminés telle que définie dans SEQ ID NO : 25 ; ou
les régions variables d'anticorps anti-B7H6 comprennent : une région variable de chaîne légère telle que définie dans SEQ ID NO : 15 et une région variable de chaîne lourde de la séquence d'acides aminés telle que définie dans SEQ ID NO : 26 ; ou
les régions variables d'anticorps anti-B7H6 comprennent : une région variable de chaîne légère telle que définie dans SEQ ID NO : 18 et une région variable de chaîne lourde de la séquence d'acides aminés telle que définie dans SEQ ID NO : 21.

4. Anticorps recombinant bispécifique selon la revendication 2, **caractérisé en ce qu'**il comprend en outre un peptide lieur ; facultativement, le peptide lieur présente une séquence d'acides aminés telle que définie dans SEQ ID NO : 46, dans lequel :
une extrémité N-terminale du peptide lieur est liée à une extrémité C-terminale d'une région variable de chaîne légère d'anticorps anti-CD3 ; et
une extrémité C-terminale du peptide lieur est liée à une extrémité N-terminale d'une région variable de chaîne lourde d'anticorps anti-CD3.

5. Anticorps recombinant bispécifique selon la revendication 3, **caractérisé en ce qu'**il comprend en outre un peptide lieur ; facultativement, le peptide lieur présente une séquence d'acides aminés telle que définie dans SEQ ID NO : 46, dans lequel :
une extrémité N-terminale du peptide lieur est liée à une extrémité C-terminale d'une région variable de chaîne légère anti-B7H6 ; et
une extrémité C-terminale du peptide lieur est liée à une extrémité N-terminale de la région variable de chaîne lourde anti-B7H6.

6. Anticorps recombinant bispécifique selon la revendication 1, **caractérisé en ce qu'**il comprend en outre une première région Fc et une seconde région Fc, dans lequel au moins une partie de la première région Fc et de la seconde région Fc est dérivée d'au moins l'un d'un anticorps murin, d'un anticorps humain, d'un anticorps de primate ou d'un mutant de ceux-ci.

7. Anticorps recombinant bispécifique selon la revendication 6, **caractérisé en ce qu'**au moins une partie de la première région Fc et de la seconde région Fc est dérivée d'une IgG humaine ou d'un mutant de celle-ci ;
facultativement, au moins une partie de la première région Fc et de la seconde région Fc est dérivée d'une IgG1 humaine ou d'un mutant de celle-ci.

8. Anticorps recombinant bispécifique selon la revendication 6, **caractérisé en ce que** la première région Fc présente au moins l'une des mutations S384C et T396W par rapport à une région Fc d'IgG1 de type sauvage.

9. Anticorps recombinant bispécifique selon la revendication 8, **caractérisé en ce que** la seconde région Fc présente au moins l'une des mutations Y380C, T397S, L399A et Y408V par rapport à la région Fc d'IgG1 de type sauvage.

10. Anticorps recombinant bispécifique selon l'une quelconque des revendications 6 à 9, **caractérisé en ce que** :
la première région Fc présente une séquence d'acides aminés telle que définie dans SEQ ID NO : 47 ; et
la seconde région Fc présente une séquence d'acides aminés telle que définie dans SEQ ID NO : 48.

11. Anticorps recombinant bispécifique, **caractérisé en ce qu'**il comprend des séquences d'acides aminés telles que définies dans SEQ ID NO : 49 et SEQ ID NO : 63.

12. Acide nucléique, **caractérisé en ce qu'**il code pour l'anticorps recombinant bispécifique selon l'une quelconque des revendications 1 à 11.

13. Acide nucléique selon la revendication 12, **caractérisé en ce qu'**il présente une séquence nucléotidique telle que définie dans SEQ ID NO : 69 et au moins l'une des séquences nucléotidiques telles que définies dans SEQ ID NO : 70 à SEQ ID NO : 87.

14. Vecteur d'expression, **caractérisé en ce qu'**il comprend l'acide nucléique selon la revendication 12 ou 13.

15. Cellule recombinante, **caractérisée en ce qu'**elle porte l'acide nucléique selon la revendication 12 ou 13, le vecteur d'expression selon la revendication 14, ou exprime l'anticorps recombinant bispécifique selon l'une quelconque des revendications 1 à 11.
